# EUROPEAN PATENT SPECIFICATION

(11) **EP 2 793 872 B1**
(45) Date of publication and mention of the grant of the patent: **10.03.2021**
(21) Application number: 12815644.5
(22) Date of filing: 21.12.2012
(51) Int. Cl.: A61K 31/135, A61K 45/06, A61K 31/137, A61K 31/45, A61P 25/28, A61P 3/00

(54) **COMBINATION OF A COMPOUND HAVING THE ABILITY TO REARRANGE A LYSOSOMAL ENZYME AND AMBROXOL AND/OR A DERIVATIVE OF AMBROXOL**
KOMBINATION EINER VERBINDUNG MIT DER FÄHIGKEIT ZUR REORGANISATION EINES LYSOSOMALEN ENZYMS UND AMBROXOL UND/ODER EINES DERIVATS VON AMBROXOL
COMBINAISON D'UN COMPOSÉ AYANT L'APTITUDE À RÉARRANGER UNE ENZYME LYSOSOMALE ET D'AMBROXOL ET/OU D'UN DÉRIVÉ D'AMBROXOL

(30) Priority: 22.12.2011 EP 11010078
(43) Date of publication of application: 29.10.2014
(73) Proprietor: Centogene IP GmbH, 18055 Rostock (DE)
(72) Inventor: LUKAS, Jan, 18055 Rostock (DE); PEWS-DAVTYAN, Anahit, 18057 Rostock (DE); BELLER, Matthias, 18211 Nienhagen (DE); ROLFS, Arndt, 10629 Berlin (DE)
(74) Representative: Bohmann, Armin K.
(86) International application number: PCT/EP2012/005363
(87) International publication number: WO 2013/091897

(56) References cited:
- WO-A2-2007/137072
- US-A1- 2011 189 710
- GIANCARLO PARENTI ET AL: "Pharmacological enhancement of mutated alpha-glucosidase activity in fibroblasts from patients with Pompe disease", MOLECULAR THERAPY, NATURE PUBLISHING GROUP, GB, vol. 15, no. 3, March 2007 (2007-03), pages 508-514, XP002620248, ISSN: 1525-0024, DOI: 10.1038/SJ.MT.6300074 [retrieved on 2007-01-09]
- BENDIKOV-BAR I ET AL: "Characterization of the ERAD process of the L444P mutant glucocerebrosidase variant", BLOOD CELLS, MOLECULES AND DISEASES, LAJOLLA, US, vol. 46, no. 1, 15 January 2011 (2011-01-15), pages 4-10, XP027586259, ISSN: 1079-9796 [retrieved on 2011-01-05]
- SMID B E ET AL: "Pharmacological small molecules for the treatment of lysosomal storage disorders", EXPERT OPINION ON INVESTIGATIONAL DRUGS, ASHLEY PUBLICATIONS LTD., LONDON, GB, vol. 19, no. 11, November 2010 (2010-11), pages 1367-1379, XP008160487, ISSN: 1354-3784, DOI: 10.1517/13543784.2010.524205
- ASANO N ET AL: "In vitro inhibition and intracellular enhancement of lysosomal alpha-galactosidase A activity in Fabry lymphoblasts by 1-deoxygalactonojirimycin and its derivatives", EUROPEAN JOURNAL OF BIOCHEMISTRY, BLACKWELL PUBLISHING, BERLIN, DE, vol. 267, no. 13, July 2000 (2000-07), pages 4179-4186, XP002300871, ISSN: 0014-2956, DOI: 10.1046/J.1432-1327.2000.01457.X
- MEERA SHANMUGANATHAN ET AL: "Inhibitor screening of pharmacological chaperones for lysosomal Î-glucocerebrosidase by capillary electrophoresis", ANALYTICAL AND BIOANALYTICAL CHEMISTRY, SPRINGER, BERLIN, DE, vol. 399, no. 8, 1 February 2011 (2011-02-01), pages 2843-2853, XP019884705, ISSN: 1618-2650, DOI: 10.1007/S00216-011-4671-6
- GIUSEPPINA ANDREOTTI ET AL: "Therapy of Fabry disease with pharmacological chaperones: from in silico predictions to in vitro tests", ORPHANET JOURNAL OF RARE DISEASES, BIOMED CENTRAL LTD, LO, vol. 6, no. 1, 17 October 2011 (2011-10-17), page 66, XP021114231, ISSN: 1750-1172, DOI: 10.1186/1750-1172-6-66
- WUSTMAN B A ET AL: "G.O. 7 Pharmacological chaperones as an alternate treatment for Pompe disease", NEUROMUSCULAR DISORDERS, PERGAMON PRESS, GB, vol. 16, no. 9-10, October 2006 (2006-10), pages 724-725, XP027967108, ISSN: 0960-8966 [retrieved on 2006-10-01]
- PARENTI G ET AL: "Pompe disease: From new views on pathophysiology to innovative therapeutic strategies", CURRENT PHARMACEUTICAL BIOTECHNOLOGY, BENTHAM SCIENCE PUBLISHERS, NL, vol. 12, no. 6, June 2011 (2011-06), pages 902-915, XP008161415, ISSN: 1389-2010, DOI: 10.2174/138920111795542606
- Giancarlo Parenti ET AL: "Pharmacological Enhancement of Mutated [alpha]-Glucosidase Activity in Fibroblasts from Patients with Pompe Disease", Molecular therapy : the journal of the American Society of Gene Therapy, vol. 15, no. 3, 1 March 2007 (2007-03-01), pages 508-514, XP055560072, US ISSN: 1525-0016, DOI: 10.1038/sj.mt.6300074
- Guanghua Wu ET AL: "Genetic analysis of lysosomal alpha-galactosidase A gene in sporadic Parkinson's disease", Neuroscience Letters, vol. 500, no. 1, 1 August 2011 (2011-08-01), pages 31-35, XP055562796, AMSTERDAM, NL ISSN: 0304-3940, DOI: 10.1016/j.neulet.2011.05.238

## Description

The present invention is related to a combination comprising a first constituent and a second constituent, wherein the first constituent is a chaperon having the ability to rearrange a lysosomal protein, the combination for use in a method for the treatment of a subject, use of the combination for the manufactuere of a medicmant, a pharmaceutical preparation comprising a first constituent, a second constituent, optionally a further constituent, wherein the first constituent is a chaperon having the ability to rearrange a lysosomal protein, a method for preparing the pharmaceutical preparation, a chaperon having the abiliy to rearrange a lysosomal protein for use in a method for the treatment of a diseases, and ambroxol or derivatives thereof for use in a method for the treatment of a disease.

Lysosomal storage diseases, also referred to herein as lysosomal storage disorders or LSDs, are a group of rare inherited metabolic disorders that result from defects in lysosomal function (Winchester B, et al. (2000), Biochem. Soc. Trans. 28 (2): 150-4). AN LSD results when a specific organelle in the body's cells - the lysosome - malfunctions. The lysosome processes unwanted material via the activity of proteins, such as enzymes, into substances that the cell can utilize. Some of the more prominent LSDs are Pompe's disease and Fabry disease.

LSDs are caused by lysosomal dysfunction usually as a consequence of reduced or absent activity of a single protein, such as a lysosomal enzyme, which is required for the metabolism of lipids, glycoproteins or mucopolysaccharides.

Like other genetic diseases, individuals typically inherit an LSD from their parents. Individually, an LSD occurs with frequencies of less than about 1:100,000 to 1:250,000, however, as a group the incidence is about 1:5,000 - 1:10,000. The overall majority of these disorders are autosomal recessively inherited; only a few are X-linked inherited, such as Fabry disease and Hunter syndrome.

Although each disorder results from different gene mutations that translate into a deficiency or reduction of a lysosomal protein's activity they all share a common biochemical characteristic - nearly all lysosomal disorders originate from an abnormal accumulation of substances inside the lysosome which is due to a deficiency or reduction of the particular lysosomal protein's activity.

LSDs affect mostly children and they often die at a young and unpredictable age, many within a few months or years of birth. Many children die of this disease following years of suffering from various symptoms of their particular disorder. The symptoms of LSDs vary, depending on the particular disorder and other variables like the age of onset, and can be mild to severe. They can include developmental delay, movement disorders, seizures, dementia, deafness and/or blindness. Some people with an LSD have enlarged livers (hepatomegaly) and enlarged spleens (splenomegaly), pulmonary and cardiac problems, and bones that develop abnormally.

There are up to now no causative cures for LSDs available. Therapies which have been tried with different success at least in animal models comprise enzyme replacement therapy, bone marrow transplantation, substrate reduction therapy, gene therapy and pharmacological chaperone therapy. Although the experimental technique of gene therapy may offer such cures in the future, therapies available at present are mainly symptomatic.

In enzyme replacement therapy, also referred to herein as ERT, recombinantly expressed or synthetized enzyme is administered intravenously which compensates for the deficiency of the affected enzyme. Cell receptors mediate the uptake of the recombinant enzyme. The constant need for treatment, the costs associated therewith and particularly the dependence on cell receptor mediated uptake are disadvantageous in ERT.

Bone marrow transplantation also referred to herein as BMT, has been supposed as a therapy for the treatment of LSDs. However, the efficacy and effectiveness of this therapy is in doubt or may be insufficient, as it is still not known whether the number of cells transferred by BMT will provide enough of the missing enzyme to successfully treat patients suffering from an LSD in general.

In substrate reduction therapy, also referred to herein as SRT, upstream inhibition of biological pathways results in reduction of substrate produced for the enzyme to process. In SRT adverse effects may result from an influence of the applied substances on other cellular processes.

Pharmacological chaperone therapy, also referred to herein as PCT, is a technique used to stabilize the lysosomal protein having reduced activity produced by patients and was examined for certain LSDs. PCT is based on the use of chaperone molecules that assist the folding of mutated enzymes and improve their stability and lysosomal trafficking.

In Pompe disease it was shown for deoxynojirimycin, an iminosugar also referred to herein as DNJ, to increase acid α-glucosidase activity, the mutated enzyme in Pompe disease (Parenti G. et al., Mol Ther. 2007 Mar;15(3):508-14. Epub 2007 Jan 9).

In Fabry disease, which is caused by mutations of lysosomal alpha-galactosidase A, also referred to herein as GLA or α-Gal A, 1-Deoxygalactonojirimycin, also referred to herein as DGJ, AT1001 or Migalastat, an inhibitor of GLA serves as a pharmacological chaperone and enhances the activity of the mutated enzyme (Asano N. et al., Eur J Biochem. 2000 Jul; 267(13):4179-86).

Iminosugars such as DGJ or DNJ may thus be selectively used as pharmacological chaperones to assist rearrangement of lysosomal proteins having reduced activity, such as mutant lysosomal proteins, and allow the treatment of patients with an LSD such as Fabry disease or Pompe disease. Pharmacological Chaperone administered in an effective dose can cause an increase of mutation reduced enzyme activity. Nevertheless, a person skilled in the art will acknowledge that the administration of pharmacological chaperones, such as iminosugars, such as for example Isofagomine applied in Gaucher's Disease, have to be exactly dosed, for example in a scheme where dosing is followed by a withdrawal period that enables the cell and enzyme to temporarily get rid of the inhibitor to display beneficial effect (Khanna et al; FEBS Journal 277 (2010) 1618-1638). It will be also acknowledged that to distinguish between inhibitory and subinhibitory concentrations is difficult and needs to be evaluated in in vivo studies. While treating Fabry's disease, i.e. mutant GLA in a cell-free environment, dosages far below 1µM are already inhibiting (Asano et al., supra). Asano and colleagues demonstrated a decrease of GLA activity in patient lymphoblasts while exceeding concentration of 100µM DGJ. In a cell culture based GLA overexpression system concentrations up to ImM (Wu et al., Hum Mutat, 2011 Aug; 32(8):965-77) are tolerated without any detectable inhibition of the tested mutant enzyme. However, it is important to note that a washout period is also included in the therein described experimental setup, i.e. two hours prior to the conduction of the assay the medium containing the Pharmacological Chaperone is removed, which is indicative for a disturbance of the assay by high concentrations of iminosugars, i.e. inhibition. In accordance therewith, it will be also acknowledged that a therapy using higher doses is hard to establish in patients and therefore needs to be considered carefully in clinical trials where 10µM are acknowledged as to be a clinically achievable concentration.

Pharmacological chaperone therapy aims to restore the activity of a lysosomal protein having reduced activity. Nevertheless, the development of pharmacological chaperon treatment of LSDs is still at an early stage. Its strong dependence on the particular patient which is to be treated, more specifically the particular LSD and its particular cause, for example, a particular mutation of the specific lysosomal protein, affect efficacy and effectiveness of said therapy.

Furthermore, the successful administration of pharmacological chaperones against LSDs is hampered by the inhibitory effect and potential toxicity when high doses of pharmacological chaperones are administered (Wu et al., supra; Asano et al., supra and Khanna et al., supra).

WO 2007/137072 A2 is related to *in vitro* and *in vivo* methods for determining whether a patient with Fabry disease will respond to treatment with a specific pharmacological chaperone.

Bendikov-Bar I et al. (Blood Cells, Molecules, and Diseases 46 (2011), pp. 4-10) report on the characterization of the ERAD process of the L444P mutant glucocerebrosidase variant.

Asano N et al. (Eur. J. Biochem. 267, 4179-4186) report on *in vitro* inhibition and intracellular enhancement of lysosomal α-galactosidase A activity in Fabry lymphoblasts by 1-deoxygalactonojirimycin and its derivatives.

The problem underlying the present invention is to provide a means for the treatment of LSDs, and methods for the treatment of LSDs.

It is a further problem underlying the present invention to enhance protein activity of a lysosomal protein having reduced activity.

It is a still further problem of the present invention to enhance the efficacy and effectiveness of a pharmacological chaperone therapy in the treatment of LSDs.

These and other problems underlying the instant invention are solved by the subject matter of the attached independent claims. Preferred embodiments may be taken from the attached dependent claims.

More specifically, the problem underlying the present invention is solved in a first aspect by a ombination comprising a first constituent and a second constituent,
wherein the first constituent is a chaperon having the ability to rearrange a lysosomal protein, wherein the lysosomal protein has a reduced activity and wherein the chaperon is an imino sugar selected from the group consisting of 1-deoxygalactonojirimycin (DGJ), alpha-galacto-homonojirimycin, alpha-allo-homonojirimycin, beta-1-C-butyl-deoxygalactonojirimycin, beta-1-C-butyl-deoxynojirimycin, N-nonyl-deoxynojirimycin (NN-DNJ), N-octyl-2,5-anhydro-2,5-imino-D-glucitol, N-octyl-isofagomine, Isofagomine (IFG), calystegine A3, calystegine B1, calystegine B2, calystegine C1, 1,5-dideoxy-1,5-iminoxylitol (DIX), alpha-1-C-nonyl-DIX, alpha-1-C-octyl-1-DNJ, N-acetyl-glucosamine-thiazoline (NGT), 6-acetamido-6-deoxycastanospermine (ACAS), N-butyl-DNJ, Deoxynojirimycin (DNJ), 2-Acetamido-1,2-dideoxynojirimycin (AdDNJ), N-dodecyl-DNJ, 6-nonyl-isofagomine, N-methyl calystegine A₃, 4-epi-isofagomine, 1-deoxynojirimycin, alpha-homonojirimycin, castanospermine, 1-deoxymannojirimycin, Swainsonine, 2-hydroxy-isofagomine, 1-deoxyfuconojirimycin, beta-homofuconojirimycin, 2,5-imino-1,2,5-trideoxy-L-glucitol, 2,5-dideoxy-2,5-imino-D-fucitol, 2,5-imino-1,2,5-trideoxy-D-altritol, 1,2-dideoxy-2-N-acetamido-nojirimycin, 1,2-dideoxy-2-N-acetamido-galaconojirimycin, 2-N-acetylamino-isofagomine, 1,2-dideoxy-2-acetamidonojirimycin, 2-N-acetamido-isofagomine, 1,2-dideoxy-2-acetamido-nojirimycin, 1-deoxyiduronojirimycin, 2-carboxy-3,4,5-trideoxypiperidine, 6-carboxy-isofagomine, 2,6-dideoxy-2,6-imino-sialic acid, and Castanospermine (CAS); and pharmaceutically acceptable salts thereof; and
wherein the second constituent is Ambroxol or a pharmaceutically acceptable salt thereof,
   bromhexine or a pharmaceutically acceptable salt thereof,
a compound of the formula (IV) 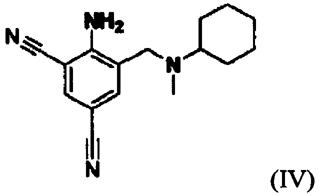 or a pharmaceutically acceptable salt thereof,
a compound of the formula (V) 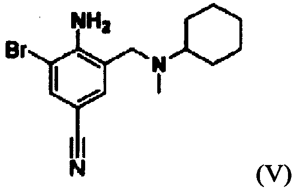 or a pharmaceutically acceptable salt thereof,
a compound of formula (VI) 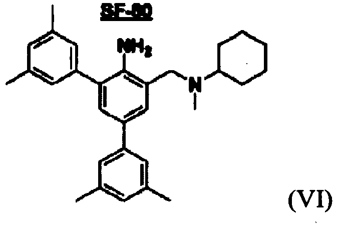 or a pharmaceutically acceptable salt thereof,
a compound of formula (VII) 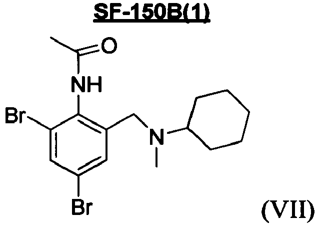 or a pharmaceutically acceptable salt thereof,
a compound of formula (VIII) 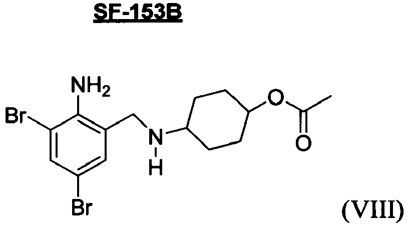 or a pharmaceutically acceptable salt thereof, and/or
a compound of formula (IX) 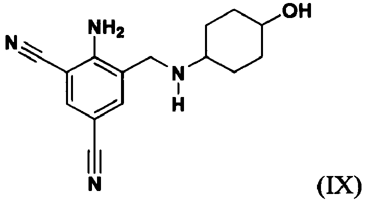 or a pharmaceutically acceptable salt thereof.

In an embodiment of the first aspect, the lysosomal protein is affected in a disease, and wherein the lysosomal protein is selected from the group consisting of alpha-galactosidase A and alpha-glucosidase.

In an embodiment of the first aspect, the combination is a pharmaceutical combination.

In an embodiment of the first aspect, the combination is suitable for or is for use in a method for the treatment of a subject comprising the administration of the combination to the subject. In an embodiment of the first aspect, the subject is suffering from or at risk of suffering from a disease, wherein the disease is characterized by an enzyme having reduced activity, wherein the enzyme having reduced activity is selected from the group consisting of alpha-galactosidase A and alpha-glucosidase.

In an embodiment of the first aspect, the disease is selected from the group comprising Fabry disease, Schindler-Kanzaki disease, Pompe's disease and Parkinson disease.

In an embodiment of the first aspect, the enzyme is alpha-galactosidase A and the disease is selected from the group comprising Fabry disease, Schindler-Kanzaki disease and Parkinson disease.

In an embodiment of the first aspect, the enzyme is alpha-glucosidase and the disease is Pompe's disease.

More specifically, the problem underlying the present invention is solved in a second aspect by the use of the combination according to the first aspect, including any embodiment thereof, for the manufacture of a medicament for the treatment or prevention of a disease.

More specifically, the problem underlying the present invention is sovled in a third aspect by a pharmaceutical preparation comprising a first constituent, a second constituent optionally a further constituent,
wherein the first constituent is a chaperon having the ability to rearrange a lysosomal protein, wherein the lysosomal protein has a reduced activity and wherein the chaperon is selected from the group consisting of 1-deoxygalactonojirimycin (DGJ), alpha-galacto-homonojirimycin, alpha-allo-homonojirimycin, beta-1-C-butyl-deoxygalactonojirimycin, beta-1-C-butyl-deoxynojirimycin, N-nonyl-deoxynojirimycin (NN-DNJ), N-octyl-2,5-anhydro-2,5-imino-D-glucitol, N-octyl-isofagomine, Isofagomine (IFG), calystegine A3, calystegine B1, calystegine B2, calystegine C1, 1,5-dideoxy-1,5-iminoxylitol (DIX), alpha-1-C-nonyl-DIX, alpha-1-C-octyl-1-DNJ, N-acetyl-glucosamine-thiazoline (NGT), 6-acetamido-6-deoxycastanospermine (ACAS), N-butyl-DNJ, Deoxynojirimycin (DNJ), 2-Acetamido-1,2-dideoxynojirimycin (AdDNJ), N-dodecyl-DNJ, 6-nonyl-isofagomine, N-methyl calystegine A₃, 4-epi-isofagomine, 1-deoxynojirimycin, alpha-homonojirimycin, castanospermine, 1-deoxymannojirimycin, Swainsonine, 2-hydroxy-isofagomine, 1-deoxyfuconojirimycin, beta-homofuconojirimycin, 2,5-imino-1,2,5-trideoxy-L-glucitol, 2,5-dideoxy-2,5-imino-D-fucitol, 2,5-imino-1,2,5-trideoxy-D-altritol, 1,2-dideoxy-2-N-acetamido-nojirimycin, 1,2-dideoxy-2-N-acetamidogalaconojirimycin, 2-N-acetylamino-isofagomine, 1,2-dideoxy-2-acetamido-nojirimycin, 2-N-acetamido-isofagomine, 1,2-dideoxy-2-acetamido-nojirimycin, 1-deoxyiduronojirimycin, 2-carboxy-3,4,5-trideoxypiperidine, 6-carboxy-isofagomine, 2,6-dideoxy-2,6-imino-sialic acid, and Castanospermine (CAS); and pharmaceutically acceptable salts thereof,
wherein the second constituent is Ambroxol or a pharmaceutically acceptable salt thereof, bromhexine or a pharmaceutically acceptable salt thereof, a compound of the formula (IV) 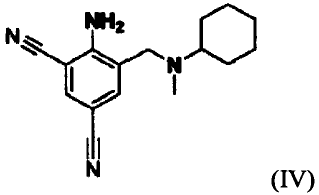 or a pharmaceutically acceptable salt thereof,
a compound of the formula (V) 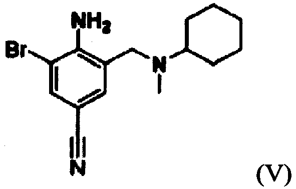 or a pharmaceutically acceptable salt thereof,
a compound of formula (VI) 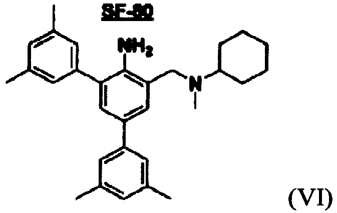 or a pharmaceutically acceptable salt thereof,
a compound of formula (VII) 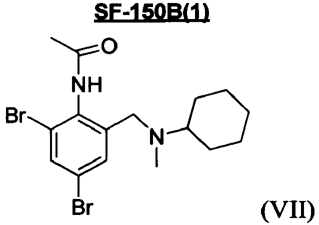 or a pharmaceutically acceptable salt thereof,
a compound of formula (VIII) 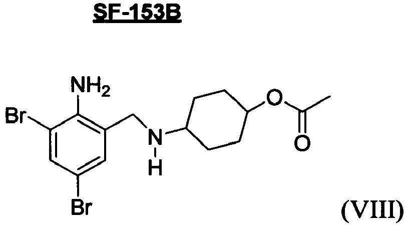 or a pharmaceutically acceptable salt thereof, and/or
a compound of formula (IX) 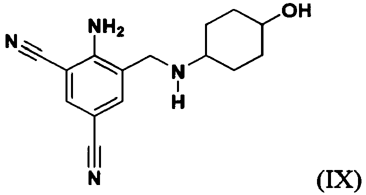 or a pharmaceutically acceptable salt thereof, and
wherein the further constituent is selected from the group comprising a pharmaceutically acceptable excipient and a pharmaceutically active agent, and
wherein preferably the pharmaceutical preparation increases the reduced activity of the lysosomal protein, and wherein the lysosomal protein has a reduced activity.

More specifically, the problem underlying the present invention is solved in a fourth aspect by a method for preparing a pharmaceutical preparation according to the first aspect, including any embodiment thereof, comprising the steps of formulating the first constituent and the second constituent into a single dosage form or into two separate dosage forms, wherein in case of two separate dosage forms a first of the two separate dosage forms contains the first constituent and a second of the two separate dosage forms contains the second constituent.

More specifically, the problem underlying the present invention is solved in a fifth aspect by a chaperon having the ability to rearrange a lysosomal protein,
wherein the lysosomal protein has a reduced activity and wherein the chaperone is selected from the group consisting of 1-deoxygalactonojirimycin (DGJ), alpha-galacto-homonojirimycin, alpha-allo-homonojirimycin, beta-1-C-butyl-deoxygalactonojirimycin, beta-1-C-butyl-deoxynojirimycin, N-nonyl-deoxynojirimycin (NN-DNJ), N-octyl-2,5-anhydro-2,5-imino-D-glucitol, N-octyl-isofagomine, Isofagomine (IFG), calystegine A3, calystegine B1, calystegine B2, calystegine C1, 1,5-dideoxy-1,5-iminoxylitol (DIX), alpha-1-C-nonyl-DIX, alpha-1-C-octyl-1-DNJ, N-acetyl-glucosamine-thiazoline (NGT), 6-acetamido-6-deoxycastanospermine (ACAS), N-butyl-DNJ, Deoxynojirimycin (DNJ), 2-Acetamido-1,2-dideoxynojirimycin (AdDNJ), N-dodecyl-DNJ, 6-nonyl-isofagomine, N-methyl calystegine A₃, 4-epi-isofagomine, 1-deoxynojirimycin, alpha-homonojirimycin, castanospermine, 1-deoxymannojirimycin, Swainsonine, 2-hydroxy-isofagomine, 1-deoxyfuconojirimycin, beta-homofuconojirimycin, 2,5-imino-1,2,5-trideoxy-L-glucitol, 2,5-dideoxy-2,5-imino-D-fucitol, 2,5-imino-1,2,5-trideoxy-D-altritol, 1,2-dideoxy-2-N-acetamido-nojirimycin, 1,2-dideoxy-2-N-acetamidogalaconojirimycin, 2-N-acetylamino-isofagomine, 1,2-dideoxy-2-acetamido-nojirimycin, 2-N-acetamido-isofagomine, 1,2-dideoxy-2-acetamido-nojirimycin, 1-deoxyiduronojirimycin, 2-carboxy-3,4,5-trideoxypiperidine, 6-carboxy-isofagomine, 2,6-dideoxy-2,6-imino-sialic acid, and Castanospermine (CAS); and pharmaceutically acceptable salts thereof, for use in a method for the treatment of a disease,
wherein the method comprises administering to a subject the chaperon and, prior to, concomitantly with or after Ambroxol or a pharmaceutically acceptable salt thereof, bromhexine or a pharmaceutically acceptable salt thereof, a compound of the formula (IV) 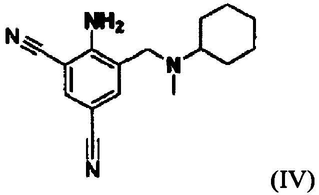 or a pharmaceutically acceptable salt thereof,
a compound of the formula (V) 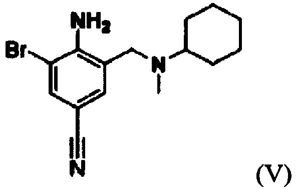 or a pharmaceutically acceptable salt thereof,
a compound of formula (VI) 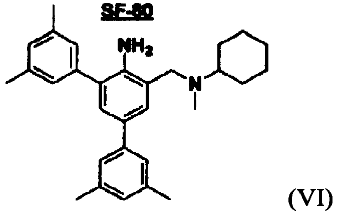 or a pharmaceutically acceptable salt thereof,
a compound of formula (VII) 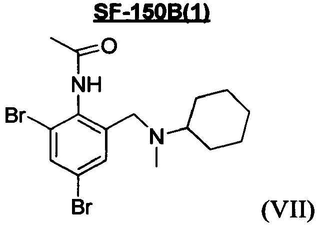 or a pharmaceutically acceptable salt thereof,
a compound of formula (VIII) 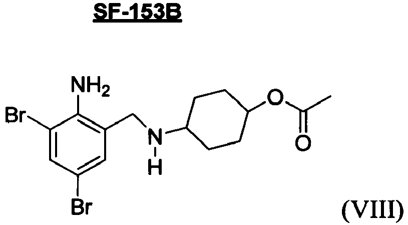 or a pharmaceutically acceptable salt thereof, and/or
a compound of formula (IX) 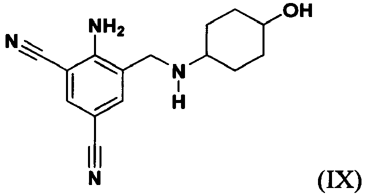 or a pharmaceutically acceptable salt thereof.

More specifically, the problem underlying the present invention is solved in a sixth aspect by ambroxol or a pharmaceutically acceptable salt thereof, bromhexine or a pharmaceutically acceptable salt thereof, a compound of the formula (IV) 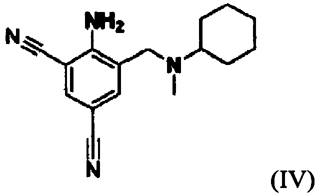 or a pharmaceutically acceptable salt thereof,
a compound of the formula (V) 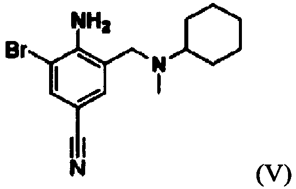 or a pharmaceutically acceptable salt thereof,
a compound of formula (VI) 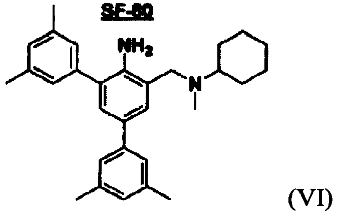 or a pharmaceutically acceptable salt thereof,
a compound of formula (VII) 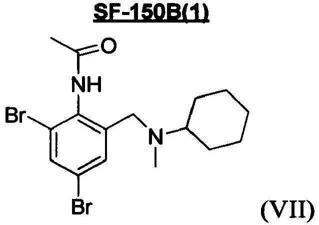 or a pharmaceutically acceptable salt thereof,
a compound of formula (VIII) 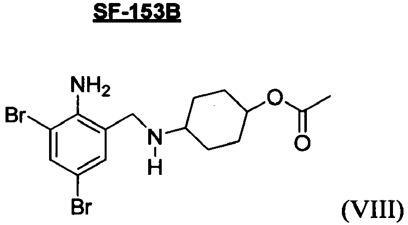 or a pharmaceutically acceptable salt thereof, and/or
a compound of formula (IX) 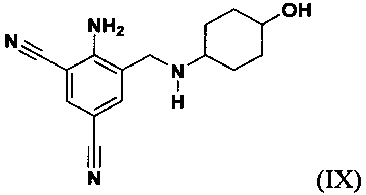 or a pharmaceutically acceptable salt thereof for use in a method for the treatment of a disease,
wherein the method comprises administering to a subject Ambroxol or a pharmaceutically acceptable salt thereof, bromhexine or a pharmaceutically acceptable salt thereof, a compound of the formula (IV) 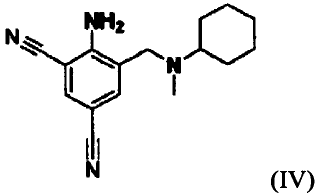 or a pharmaceutically acceptable salt thereof,
a compound of the formula (V) 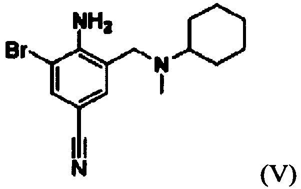 or a pharmaceutically acceptable salt thereof,
a compound of formula (VI) 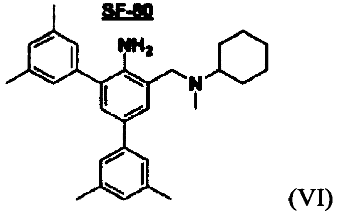 or a pharmaceutically acceptable salt thereof,
a compound of formula (VII) 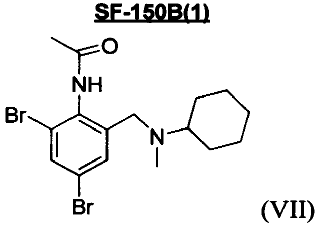 or a pharmaceutically acceptable salt thereof,
a compound of formula (VIII) 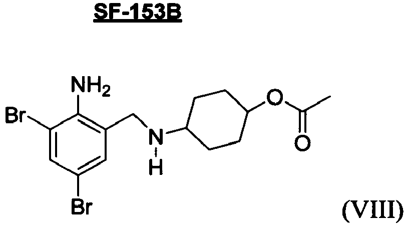 or a pharmaceutically acceptable salt thereof, and/or
a compound of formula (IX) 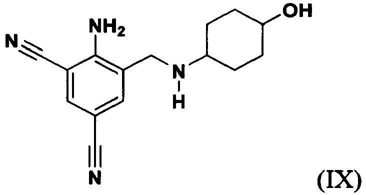 or a pharmaceutically acceptable salt thereof and, prior to, concomitantly with or after a chaperon having the ability to rearrange a lysosomal protein,
wherein the lysosomal protein has a reduced activity and wherein the chaperon is selected from the group consisting of 1-deoxygalactonojirimycin (DGJ), alpha-galacto-homonojirimycin, alpha-allo-homonojirimycin, beta-1-C-butyl-deoxygalactonojirimycin, beta-1-C-butyl-deoxynojirimycin, N-nonyl-deoxynojirimycin (NN-DNJ), N-octyl-2,5-anhydro-2,5-imino-D-glucitol, N-octyl-isofagomine, Isofagomine (IFG), calystegine A3, calystegine B1, calystegine B2, calystegine C1, 1,5-dideoxy-1,5-iminoxylitol (DIX), alpha-1-C-nonyl-DIX, alpha-1-C-octyl-1-DNJ, N-acetyl-glucosamine-thiazoline (NGT), 6-acetamido-6-deoxycastanospermine (ACAS), N-butyl-DNJ, Deoxynojirimycin (DNJ), 2-Acetamido-1,2-dideoxynojirimycin (AdDNJ), N-dodecyl-DNJ, 6-nonyl-isofagomine, N-methyl calystegine A₃, 4-epi-isofagomine, 1-deoxynojirimycin, alpha-homonojirimycin, castanospermine, 1-deoxymannojirimycin, Swainsonine, 2-hydroxy-isofagomine, 1-deoxyfuconojirimycin, beta-homofuconojirimycin, 2,5-imino-1,2,5-trideoxy-L-glucitol, 2,5-dideoxy-2,5-imino-D-fucitol, 2,5-imino-1,2,5-trideoxy-D-altritol, 1,2-dideoxy-2-N-acetamido-nojirimycin, 1,2-dideoxy-2-N-acetamidogalaconojirimycin, 2-N-acetylamino-isofagomine, 1,2-dideoxy-2-acetamido-nojirimycin, 2-N-acetamido-isofagomine, 1,2-dideoxy-2-acetamido-nojirimycin, 1-deoxyiduronojirimycin, 2-carboxy-3,4,5-trideoxypiperidine, 6-carboxy-isofagomine, 2,6-dideoxy-2,6-imino-sialic acid, and Castanospermine (CAS); and pharmaceutically acceptable salts thereof.

More specifically, the problem underlying the present invention is solved in a seventh aspect by a combination according to the first aspect, including any embodiment thereof, for use in a method of personalized therapeutic treatment of a subject, wherein the method comprises the following steps:
step a): determining whether in a sample of the subject the lysosomal protein has a reduced activity, preferably such reduced activity results from one or more mutation of the lysosomal protein compared to the wild type lysosomal protein;
step b): identifying a compound having the ability to rearrange the lysosomal protein having reduced activity, and wherein the compound is suitable for or is increasing the reduced activity of the lysosomal protein; and
step c):administering to the subject the first constituent prior to, concomitantly with or after the second constituent.

In an embodiment of the first, second, third and fourth aspect, the first constituent is alpha-galacto-homonojirimycin or a pharmaceutically acceptable salt thereof; and the second constituent is Ambroxol and/or a derivative thereof.

In an embodiment of the first, second, third and fourth aspect, the first constituent is alpha-allo-homonojirimycin or a pharmaceutically acceptable salt thereof; and the second constituent is Ambroxol and/or a derivative thereof.

In an embodiment of the first, second, third and fourth aspect, the first constituent is beta-1-C-butyl-deoxygalactonojirimycin or a pharmaceutically acceptable salt thereof; and the second constituent is Ambroxol and/or a derivative thereof.

In an embodiment of the first, second, third and fourth aspect, the first constituent is N-acetyl-glucosamine-thiazoline (NGT) or a pharmaceutically acceptable salt thereof; and the second constituent is Ambroxol and/or a derivative thereof.

In an embodiment of the first, second, third and fourth aspect, the first constituent is 6-acetamido-6-deoxycastanospermine (ACAS) or a pharmaceutically acceptable salt thereof; and the second constituent is Ambroxol and/or a derivative thereof.

In an embodiment of the first, second, third and fourth aspect, the first constituent is N-butyl-DNJ or a pharmaceutically acceptable salt thereof; and the second constituent is Ambroxol and/or a derivative thereof.

In an embodiment of the first, second, third and fourth aspect, the first constituent is deoxynojirimycin or a pharmaceutically acceptable salt thereof; and the second constituent is Ambroxol and/or a derivative thereof.

Disclosed is a method for the treatment of a disease, wherein the method comprises administering to a subject a first constituent as defined in any one of the first, second and third aspect of the invention of the invention as defined in the claims, prior to, concomitantly with or after a second constituent as defined in any one of the first, second and third aspect of the invention as defined in the claims, and/or a combination according to any one of the first, second and third aspect of the invention as defined in the claims.

Also disclosed is a method for increasing activity of a lysosomal protein, wherein the lysosomal protein has a reduced activity, wherein the method comprises administering to a cell a compound having the ability to rearrange the lysosomal protein, and Ambroxol and/or a derivative thereof.

Equally disclosed in a method for increasing activity of a lysosomal protein, wherein the lysosomal protein has a reduced activity, wherein the method comprises administering to a subject a compound having the ability to rearrange the lysosomal protein, and Ambroxol and/or a derivative thereof, wherein the compound having the ability to rearrange the lysosomal protein increases the activity of the lysosomal protein.

Disclosed is a method for increasing activity of a lysosomal protein, wherein the lysosomal protein has a reduced activity, wherein the method comprises, administering to a subject a first constituent as defined in any one first, second and third aspect of the invention as defind in the claims and, prior to, concomitantly with or after a second constituent as defined in any one the first, second and third aspect of the invention as defined in the claims, and/or a combination according to any one of the first, second and third aspect of the invention as defined in the claims and/or a pharmaceutical preparation according to the third asecpt of the invention as defined in the claims, wherein the combination and/or the pharmaceutical preparation increases the activity of the lysosomal protein.

The instant disclosure also relates to a combination comprising a first constituent and a second constituent, wherein the first constituent is a compound having the ability to rearrange a lysosomal protein, wherein the lysosomal protein has a reduced activity wherein the reduced activity is reduced due to a mutation of the lysosomal protein, and wherein the second constituent is Ambroxol and/or a derivative of Ambroxol, wherein the combination is preferably a combination according to any one of the first, second and third aspect of the invention as defined in the claims, for use in a method of personalized therapeutic treatment of a subject, wherein the method comprises the following steps:
step a): determining whether in a sample of the subject the lysosomal protein has a reduced activity, preferably such reduced activity results from one or more mutation of the lysosomal protein compared to the wild type lysosomal protein;
step b): identifying a compound having the ability to rearrange the lysosomal protein having reduced activity, and wherein the compound is suitable for or is increasing the reduced activity of the lysosomal protein; and
step c):administering to the subject the first constituent prior to, concomitantly with or after the second constituent.

The instant disclosure also related to a pharmaceutical preparation comprising a first constituent, a second constituent and optionally a further constituent,
wherein the first constituent is a compound having the ability to rearrange a lysosomal protein, wherein the lysosomal protein has a reduced activity, wherein the reduced activity is reduced due to a mutation of the lysosomal protein, and wherein the second constituent is Ambroxol and/or a derivative of Ambroxol, wherein the further constituent is selected from the group comprising pharmaceutically acceptable excipients and pharmaceutically active agents, wherein the pharmaceutical preparation is preferably a pharmaceutical preparation according to the third aspect of the invention as defined in the claims, for use in a method of personalized therapeutic treatment of a subject, wherein the method comprises the following steps:
step a): determining whether in a sample of the subject the lysosomal protein has a reduced activity, preferably such reduced activity results from one or more mutation of the lysosomal protein compared to the wild type lysosomal protein;
step b): identifying a compound having the ability to rearrange the lysosomal protein having reduced activity, and wherein the compound is suitable for or is increasing the reduced activity of the lysosomal protein; and
step c): administering to the subject the first constituent prior to, concomitantly with or after the second constituent.

In an embodiment of the first, second, third, fourth, sixth and seventh aspect, the compound having the ability to rearrange a lysosomal protein is a chaperon.

In an embodiment of the first, second, third, fourth, sixth and seventh aspect, the chaperon is a pharmacological chaperon or a pharmaceutically acceptable salt, solvate or derivative thereof.

The present inventors have surprisingly found that the combined use of a compound having the ability to rearrange a lysosomal protein having reduced activity and Ambroxol or a derivative of Ambroxol is suitable for the treatment of LSDs such as Fabry disease and Pompe disease. In such combination, preferably the compound having the ability to rearrange a lysosomal protein having reduced activity is a chaperone, preferably a pharmacological chaperone.

More specifically, the present inventors when conducting experiments in a cell culture system which is an acknowledged model for LSDs and Fabry disease in particular (Ishii et al.; Biochem. J. (2007) 406, 285-295; Shin et al.; Pharmacogenet Genomics. Sep 2008; 18(9): 773-780; Parenti G et al., Mol Ther. 2007; 15:508-14; Okumiya T et al., Mol Genet Metab 2007 Jan;90(1):49-57) have found that when DGJ of Galactose is administered together with Ambroxol the activity of mutant alpha-galactosidase A is surprisingly and unexpectedly increased compared to the effect arising from administering DGJ or Galactose only.

When conducting further studies the present inventors also found that when NB-DNJ is administered together with Ambroxol the activity of mutant acid α-Glucosidase is surprisingly and unexpectedly increased compared to the effect arising from administering NB-DNJ only.

In connection therewith it is particularly noteworthy that, as also found by the instant inventors, administering Ambroxol alone, i.e. without DGJ, does not exhibit any enhancing effect on alpha-galactosidase A activity in alpha-galactosidase A mutants frequently resulting in Fabry disease, and that administering Ambroxol alone, i.e. without NB-DNJ does not exhibit any enhancing effect on acid α-Glucosidase activity in acid α-Glucosidase mutants frequently resulting in Pompe disease.

It is the merit of the present inventors having found that administering a combination according to the present invention is advantageous over the enhancement of the activity of the lysosomal protein having a reduced activity with a compound having the ability to rearrange a lysosomal protein alone. More particularly, administering Ambroxol and/or a derivative of Ambroxol in combination with said compound having the ability to rearrange a lysosomal protein further enhances the activity of the lysosomal protein having reduced protein activity compared to administering said compound having the ability to rearrange a lysosomal protein alone. Thus administration of a combination of the present invention is advantageous over the administration of the compound having the ability to rearrange a lysosomal protein alone in that further enhancement of the activity of the protein having reduced activity is achieved and/or a significantly lower amount of the compound having the ability to rearrange a lysosomal protein may be administered to a patient still resulting in the same or similar beneficial effect of treatment as if the compound having the ability to rearrange a lysosomal proteins is administered alone at a higher concentration or in a higher amount.

More particularly, it is the merit of the present inventors having found that the combined use of a pharmacological chaperone and Ambroxol and/or a derivative of Ambroxol is suitable for increasing the activity of a mutant protein in the treatment of an LSD such as Fabry disease compared to the application of said pharmacological chaperone alone. The amount of pharmacological chaperone administered to a subject can be reduced if the pharmacological chaperone is administered as the first constituent of the combination according to the present invention and will result in the same or similar level of activity or a higher level of activity of the lysosomal protein as if the pharmacological chaperone is administered alone in a higher amount.

Furthermore, it will be acknowledged by a person skilled in the art that any feature, advantage, embodiment of and any statement made herein in relation to a combination of the present invention equally applies to a pharmaceutical preparation according to the present invention, a method according to the present invention, a use according to the present invention, and vice versa.

Furthermore, it will be acknowledged by a person skilled in the art that any feature, advantage, embodiment of and any statement made herein in relation to any aspect of the present invention equally applies to each and any of the other aspects of the present invention. The term "reduced activity" of a lysosomal protein as used herein preferably means that the activity of a lysosomal protein, e.g. a lysosomal protein being a lysosomal protein mutant resulting from a mutation, is reduced compared to a control, such as a control protein, for example said protein without such mutation. In an embodiment such reduced activity is the activity of an enzyme which is reduced due to a mutation of said enzyme compared to an activity of said enzyme without such mutation and wherein, preferably, said reduced enzyme activity results in a disease, e.g. an LSD, such as Fabry's disease.

It is a further advantage of the combination according to the present invention that the activity of a lysosomal protein having a reduced activity, wherein treatment with a pharmacological chaperone alone does not result in an increase of activity of the lysosomal protein mutant sufficient to treat a subject, can be elevated to a level sufficient for the treatment, preferably therapeutic treatment of the subject. In other words, in particular lysosomal protein mutants wherein the treatment with a particular pharmacological chaperone results in an increase of activity of the lysosomal protein mutants the increase is too low to treat the subject. If the pharmacological chaperone is administered as a first constituent of the combination according to the present invention the increase of activity of the lysosomal protein mutant can be sufficient to treat the subject.

A sufficient treatment as used herein preferably is a treatment which results in elevating and/or increasing the activity of a lysosomal protein having a reduced activity due to a mutation, i.e. a lysosomal protein mutant, to a level of 20%, preferably of 25%, of the activity of the lysosomal protein not having the mutation and preferably determined in the absence of treatment. In an embodiment, such sufficient treatment results in a therapeutic effect indlcuding, but not limited to, treatment of the LSD from which the subject suffers or at least amelioration of the symptoms of the subject suffering from the LSD.

It will be immediately acknowledged by a skilled person that various methods for determining the activity of a protein, such as the activity of an enzyme, and thus determining whether said activity is reduced compared to a control such as a protein without mutation(s) which result(s) in such reduced activity, are known to a person skilled in the art and particularly depend on the protein the activity of which is determined and, preferably, compared to a control. For example, determining the activity of α-galactosidase A and/or α-glucosidase is described as enzymatic measurement of α-galactosidase A and α-glucosidase in Example 1 herein, respectively. In order to determine the activity of β-Hexosaminidase A/B and/or whether said activity is reduced preferably the assay as described in Wood et al. is used (Wood, S and Macdougall, BG Am J Hum Genet 28:489-495, 1976). In order to determine the activity of beta-galactosidase and/or whether said activity is reduced preferably the assay as described in Yang et al. is used (Yang et al., Journal of Biomedical Science 2010, 17:79).

It will be acknowledged by a person skilled in the art that in certain diseases resulting from reduced activity of a lysosomal protein typically resulting from a mutation of said lysosomal protein, the administration of Ambroxol alone results in an enhancement of the activity of a/the mutant lysosomal protein. It is thus important to understand that the present invention is based on the finding that the effect of a compound having the ability to rearrange a protein having reduced activity, such as a pharmacological chaperone, is enhanced when administered together with Ambroxol and/or a derivative of Ambroxol, wherein the administration of Ambroxol and/or a derivative of Ambroxol is not enhancing the activity of the protein having reduced activity when administered alone. The administration of said combination is thus advantageous over the administration of said compound having the ability to rearrange a protein having reduced activity alone in that Ambroxol enhances the activity of said compound.

In connection therewith it will be acknowledged by a skilled person that any derivative of Ambroxol and/or bromhexine enhancing the activity of a/the protein having reduced activity when administered in combination with said compound having the ability to rearrange a/the protein having reduced activity is suitable for the practicing of the instant invention.

A compound having the ability to rearrange a lysosomal protein having reduced activity as used herein preferably means a small molecule which reversibly binds to the lysosomal protein having reduced activity, said binding resulting in an increase in the reduced activity, i.e. said binding resulting in an activity which is higher compared to the activity of the lysosomal protein without the compound having the ability to rearrange a/the lysosomal protein having reduced activity. Preferably, said binding corrects a folding defect of the protein having reduced activity through a stabilization effect. More preferably, said binding prevents degradation of said protein. A small molecule as used herein preferably is a small molecule according to Lipinski's rule of five. In an embodiment of the combination according to the present invention as defined in the claims, the compound having the ability to rearrange a lysosomal protein having reduced activity preferably is a chaperone and more preferably is a pharmacological chaperone. In a preferred embodiment of the combination according to the present invention a compound having the ability to rearrange a lysosomal protein, wherein the lysosomal protein has a reduced activity, is capable of increasing and/or elevating the reduced activity. In a further embodiment a compound having the ability to rearrange a lysosomal protein is a compound which is capable of increasing and/or elevating the reduced activity irrespective of whether or not said compound has the ability to rearrange a lysosomal protein, i.e. the activity of the lysosomal protein is higher when the compound is present compared to the activity of the lysosomal protein when the compound is not present.

A person skilled in the art will acknowledge that a pharmacological chaperon, such as an iminosugar, e.g. DGJ or DNJ, interacts with a specific enzyme and/or a specific enzyme having a mutation. The use of pharmacological chaperones in the treatment of LSDs is summarized in, e.g., Parenti et al., (Parenti et al., 2009 EMBO Mol Med 1, 268-279).

For example, NB-DNJ but not NB-DGJ effects folding of beta-Glucocerebrosidase (Butters TD et al., Glycobiology 2005, 15:43R-52R). However, sphingolipid synthesis can be inhibited by both drugs. Furthermore, DNJ is often used as negative control for testing putative drugs and it has no effect on enhancing protein activity in Fabry (Fan et al. 2003, Methods in Enzymology, Vol. 363, p. 412-420) or Gaucher. Interestingly, Parenti et al. showed an effect of DNJ in enhancing protein activity in Pompe's disease (Parenti et al. 2007 Mol Ther. 2007 Mar;15(3):508-14. Epub 2007 Jan 9).

Further examples for the specific interaction of pharmacological chaperons are described in Butters et al. (2005, supra) and Parenti et al. (2009, supra).

The present inventors assume, without wishing to be bound by any theory, that the specific interaction and non-interaction, respectively, of pharmacological chaperons with lysosomal protein mutants is due to a stereochemical hindrance, for example with L-sugars or with DNJ, which prevents access to the active site of the lysososmal protein. Accordingly, rearrangement of the lysosomal protein mutant is or is not possible and thus enhancement of transport and/or activity is or is not prevented. The instant inventors further assume that due to interaction of the pharmacological chaperone with the active site of the lysosomal protein a rearrangement of the lysosomal protein is induced leading to a stabilization of the lysosomal protein mutant, similar to wild type. Thus, the mutant lysosomal protein being no longer misfolded or less misfolded is no longer recognized by the cellular degradation machinery and accordingly and preferably escapes early degradation. As a result, transport into the lysosome is enhanced. Applying different assays and methods Yam et al. (Yam et al., supra) and Ishii et al. (Ishii et al., supra) both showed accumulation of the enzyme after treatment in the lysosome in Fabry.

In addition, it is important to understand that a disease which results from a lysosomal protein having a reduced activity and wherein the reduced activity is due to a mutation of the lysosomal protein may actually be the result of different mutations, i.e. different subjects may suffer from the same disease having different mutations of the lysosomal proteins. By way of example, more than 400 mutations which result in Fabry's disease are known to date. In connection therewith, a person skilled in the art will immediately acknowledge that for successful treatment of such disease the reduced activity of the lysosomal protein having reduced activity has to be increased. More particularly, the particular lysosomal protein mutant has to be a mutant responsive for the treatment with a compound having the ability to rearrange a lysosomal protein having a reduced activity, such as a pharmacological chaperone. A "responsive mutant" as used herein preferably means a lysosomal protein having a particular mutation, wherein the lysosomal protein has a reduced activity due to the mutation and wherein the compound having the ability to rearrange a lysosomal protein having a reduced activity is suitable for or is increasing and/or enhancing the reduced activity so that the activity of the lysosomal protein is increased.

In an embodiment of the combination and/or pharmaceutical preparation according to the present invention as defined in the claims, the activity of the lysosomal protein is reduced due to a mutation. In a further embodiment of the present invention the mutation is a mutation responsive to a/the compound having the ability to rearrange the lysosomal protein, wherein the lysosomal protein has a reduced activity. In an embodiment of the combination and/or the pharmaceutical preparation according to the present invention the compound having the ability to rearrange a lysosomal protein, wherein the lysososmal protein has a reduced activity, is suitable for and/or is capable of increasing the activity of the lysosomal protein.

A person skilled in the art will thus also acknowledge that a compound having the ability to rearrange a protein having reduced activity may be specifically effective in enhancing the activity of a specific protein in a specific disease. In other words, a certain compound having the ability to rearrange a protein having reduced activity, such as a certain pharmacological chaperone, may be administered in a certain disease as said pharmacological chaperone enhances the activity of a certain protein having reduced activity in said certain disease.

In an embodiment of the invention as defined in the claims, a compound having the ability to rearrange a protein having reduced activity is an active site-specific chaperone.

The following table 1 is taken from Fan et al. (Fan JQ et al., Biol Chem. 2008 Jan;389(1):1-11.) and comprises a list of exemplary active site-specific chaperones, also referred to herein as ASSC, and the respective LSD, wherein the activity of the respective affected protein having reduced activity may be enhanced upon treatment with the indicated ASSC.

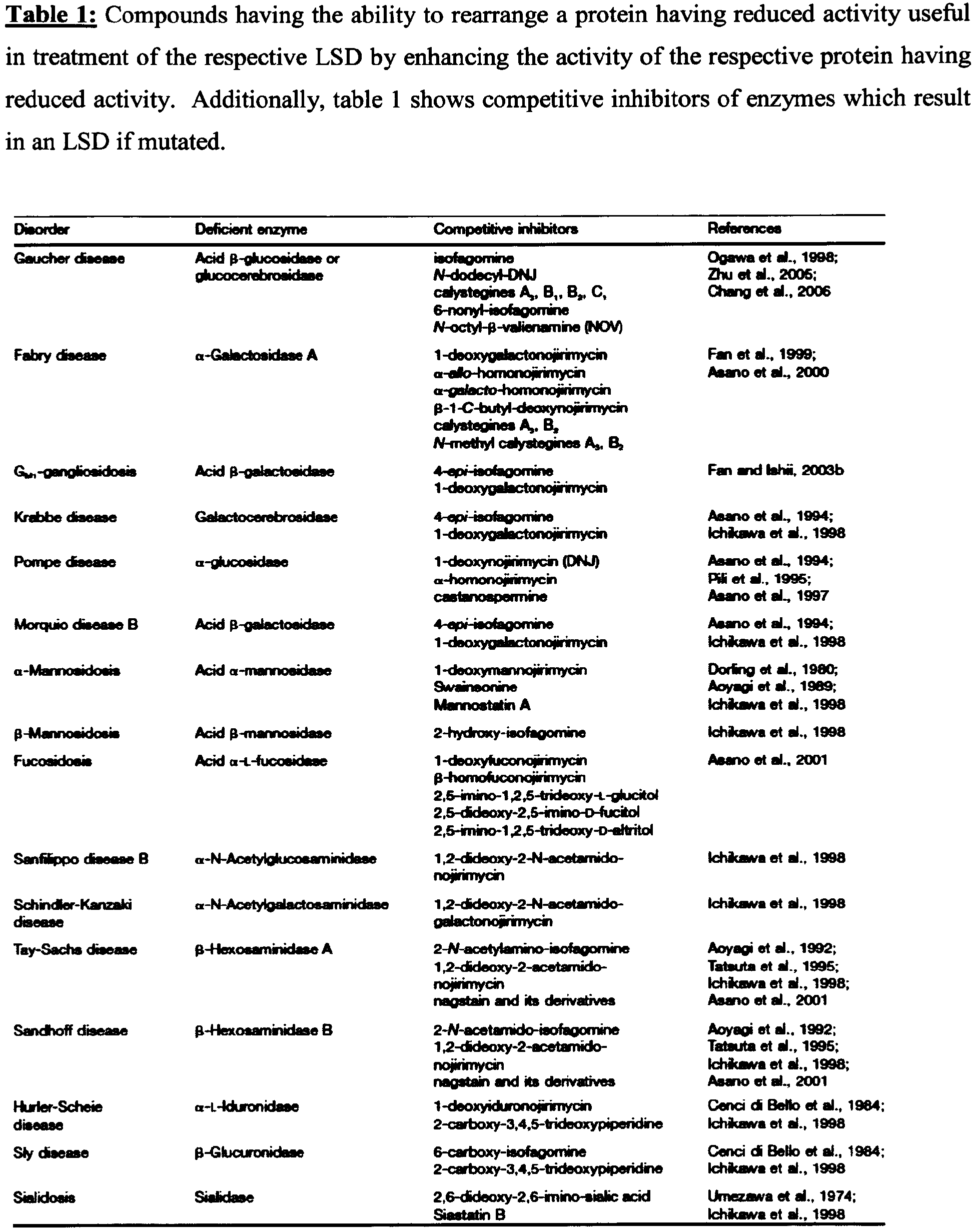

Additionally, Sawkar et al. (Sawkar, A. R. et a., Proc. Natl. Acad. Sci. U. S. A. 2002, 99, 15428-15433) showed that NB-DNJ and NN-DNJ can function as pharmacological chaperones in Gaucher's disease.

A person skilled in the art will acknowledge that in an embodiment an LSD is characterized by a lysosomal dysfunction which results from genetic mutation(s) usually leading to a reduced activity, including deficiency of protein activity, of a protein such as an enzyme, a membrane transport protein, a trans-membrane protein or a soluble non-enzymatic protein preferably required for or involved in the metabolism of lipids, glycoproteins or mucopolysaccharides. Said protein having reduced activity preferably is a lysosomal protein and the reduced activity thereof more preferably results from misfolding of the protein. Correcting said misfolding of said protein by rearrangement which comprises stabilization of said lysosomal protein having reduced activity, results in an enhancement of said reduced protein's activity and is, in an embodiment of the invention, thus suitable for the treatment of a disease, preferably an LSD.

In molecular biology, chaperones are proteins that assist folding or unfolding and assembly or disassembly of other macromolecular structures, such as proteins, but do not occur in these structures when the latter are performing their normal biological functions. Nevertheless, chaperones are not strictly concerned with protein folding. Moreover, a chaperon may assist in the assembly of, e.g., nucleosomes from folded histones and DNA, and such assembly chaperones, especially in the nucleus, are concerned with the assembly of folded subunits into oligomeric structures.

Chaperones as preferably used herein do not necessarily convey steric information required for proteins to fold. One major function of chaperones is to prevent both newly synthesized polypeptide chains and assembled subunits from aggregating into nonfunctional structures. It is for this reason that many chaperones, but by no means all, are also heat shock proteins because the tendency to aggregate increases as proteins are denatured by stress. Many chaperones are heat shock proteins, that is, proteins expressed in response to elevated temperatures or other cellular stresses. The reason for this behavior is that protein folding is severely affected by heat and, therefore, some chaperones act to repair the potential damage caused by misfolding.

The term "affected" as used herein preferably means "to change" or "being changed" and more preferably refers to a changed behavior, structure, activity or condition. For example, an affected protein may be affected in such that its activity is changed, i.e. increased or decreased, or as a further example an affected protein may be affected in such that its structure or sequence is changed compared to the protein's structure or sequence in an unaffected stage. The term "to affect" refers to herein preferably to an influence which a change has on something else. Thus said protein being affected in a disease means that said changed activity and/or structure or sequence is changed compared to the activity and/or structure or sequence of said protein in a healthy patient. In a preferred embodiment thereof, the change goes along with a change in the condition of a subject the metabolism of lysosomal protein of which is affected; more preferably such change goes along with a disease or a predispotion of a disease.

Other chaperones are involved in folding newly made proteins as they are extruded from the ribosome. Although most newly synthesized proteins can fold in the absence of chaperones, a minority strictly requires them.

Other types of chaperones are involved in transport across membranes, for example membranes of the mitochondria and endoplasmic reticulum (ER) in eukaryotes. Bacterial translocation-specific chaperones maintain newly synthesized precursor polypeptide chains in a translocation-competent (generally unfolded) state and guide them to the translocon.

Molecular chaperones as used herein are preferably involved in protein folding and provide other functions in addition to stabilization. Molecular chaperones recognize unfolded protein elements such as exposed hydrophobic patches and bind to unfolded proteins and prevent aggregation or provide stabilization during folding, after which the folded protein dissociates. Molecular chaperones also direct proteins that do not fold correctly to degradation pathways.

Chemical chaperones, as used herein, preferably induce stabilizing effects by non-specific binding. Chemical chaperones include but are nto limited to glycerol.

Chaperon functions also comprise among others assistance in protein degradation, bacterial adhesion activity and responding to diseases linked to protein aggregation.

Protein misfolding is recognized as an important pathophysiological cause of protein deficiency or reduced activity in many genetic disorders, especially in LSDs. Inherited mutations can disrupt native protein folding, thereby producing proteins with misfolded conformations. These misfolded proteins are consequently retained and degraded by endoplasmic reticulum-associated degradation, although they would otherwise be catalytically fully or partially active. Lack of protein function or reduced activity of lysosomal proteins results in substrate accumulation, impaired transport of ions or molecules, and disruption of biosynthetic pathways.

Recently, it was found that potent competitive inhibitors for enzymes associated with LSDs enhance the activity of such enzymes in cells when administered at concentrations lower than that normally required to inhibit the intracellular enzyme activity. The effect is particularly significant on certain defective or mutant enzymes, but also occurs in cells containing the normal enzyme type, preferably the non-defective enzyme and/or the non-mutant enzyme. The use of such pharmacological chaperones, such as DGJ, in PCT of an LSD preferably results from stabilizing the lysosomal mutant protein having reduced activity by the selective binding of the pharmacological chaperone to said mutant protein in its misfolded state. More preferably, said binding results in a more stable state of the protein and increased stability leads to decrease in degradation by endoplasmic reticulum-associated degradation. A pharmacological chaperone is preferably an active site-directed competitive inhibitor or a specific-site chaperone. Such active site-specific chaperones preferably act as reversible inhibitors that efficiently bind the mutant protein in the endoplasmic reticulum and facilitate folding and/or stabilization of the mutant protein resulting in more protein being processed and transported to the correct location, for example the lysosome. The pharmacological chaperone thereby preferably acts as a reversible binder and dissociates in the correct location where a higher level of residual protein activity is achieved. Nevertheless, a person skilled in the art will acknowledge that high concentrations of pharmacological chaperones will result in an inhibitory effect on the activity of the mutant protein (Asano et al., supra; Khanna et al., supra).

Accordingly, pharmacological chaperones are preferably applied in a sub-inhibitory concentration, wherein the stabilization of the mutant protein after rescue from the endoplasmatic reticulum preferably also results in a longer half-life of the mutant protein. Specific-site chaperones in contrast to active site chaperones do not bind the active site of the mutant protein. Therefore specific-site chaperones may remain bound to the mutant protein as long as the function of said protein is not inhibited.

Pharmacological chaperones used in the combination of the present invention preferably have at least one pharmaceutical characteristics selected from the group comprising low toxicity, good solubility and brain-barrier penetration.

Pharmacological chaperones as used herein are preferably selected from the group comprising sugars and iminosugar and derivatives of the sugars and iminosugar as well as pharmaceutical acceptable salts thereof.

The following table 2 is taken from Toprak et al. (Toprak et al., 2004, The Journal of biological chemistry, Vol. 279, No. 14, Issue of April 2, pp. 13478-13487, 2004) and shows illustrative but non-limiting examples of imino sugars useful as pharmacological chaperones in the combination according to the present invention, preferably wherein the lysosomal protein is hexosaminidase A.

**Table 2: Imino sugars - pharmacological chaperones**

| **Structure** | **Name** | Ki/IC₅₀ |
|---|---|---|
| 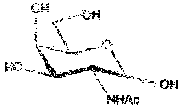 | *N*-Acetyl-galactosamine (GalNAc) | Ki = 1.9 mM¹ |
| 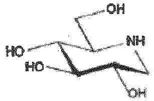 | Deoxynojirimycin (DNJ) | **N/A** |
| 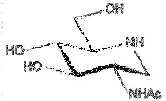 | 2-Acetamido-1,2-dideoxynojirimycin (AdDNJ) | Ki = 700 nM² |
| 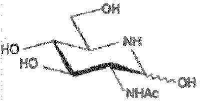 | 2-Acetamido-2-deoxynojirimycin (ADNJ) | Ki = 5 nM² |
| 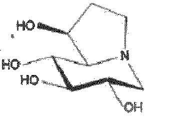 | Castanospemine (CAS) | N/A |
| 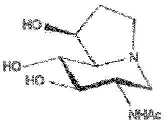 | 6-Acetamido-6-deoxycastanospemine (ACAS) | IC₅₀ = 500 nM¹ |
| 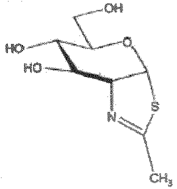 | *N*-Acetyl-glucosamine-thiazoline (NGT) | Ki = 280 nM² |
| | | Ki = 300 nM¹ |

Pharmacological chaperones as used herein are selected from the group comprising 1-deoxygalactonojirimycin (DGJ), alpha-galacto-homonojirimycin, alpha-allo-homonojirimycin, beta-1-C-butyl-deoxygalactonojirimycin, beta-1-C-butyl-deoxynojirimycin, N-nonyl-deoxynojirimycin (NN-DNJ), N-octyl-2,5-anhydro-2,5-imino-D-glucitol, N-octyl-isofagomine, Isofagomine (IFG), calystegine A3, calystegine B1, calystegine B2, calystegine C1, 1,5-dideoxy-1,5-iminoxylitol (DIX), alpha-1-C-nonyl-DIX, alpha-1-C-octyl-1-DNJ, N-acetyl-glucosamine-thiazoline (NGT), 6-acetamido-6-deoxycastanospermine (ACAS), N-bultyl-DNJ, Deoxynojirimycin (DNJ), 2-Acetamido-1,2-dideoxynojirimycin (AdDNJ), N-dodecyl-DNJ, 6-nonyl-isofagomine, N-methyl calystegine A3, 4-epi-isofagomine, 1-deoxynojirimycin, alpha-homonojirimycin, castanospermine, 1-deoxymannojirimycin, Swainsonine, 2-hydroxy-isofagomine, 1-deoxyfuconojirimycin, beta-homofuconojirimycin, 2,5-imino-1,2,5-trideoxy-L-glucitol, 2,5-dideoxy-2,5-imino-D-fucitol, 2,5-imino-1,2,5-trideoxy-D-altritol, 1,2-dideoxy-2-N-acetamido-nojirimycin, 1,2-dideoxy-2-N-acetamido-galaconojirimycin, 2-N-acetylamino-isofagomine, 1,2-dideoxy-2-acetamido-nojirimycin, 2-N-acetamido-isofagomine, 1,2-dideoxy-2-acetamido-nojirimycin, 1-deoxyiduronojirimycin, 2-carboxy-3,4,5-trideoxypiperidine, 6-carboxy-isofagomine, 2,6-dideoxy-2,6-imino-sialic acid, and Castanospermine (CAS); and pharmaceutically acceptable salts thereof.

A person skilled in the art will know other pharmacological chaperones which may be applied for pharmacological chaperone therapy of LSDs.

Preferably a pharmacological chaperone has the ability to rearrange a lysosomal protein having reduced activity, said reduced activity preferably resulting from a mutation and being the or a cause of an LSD.

In an embodiment of the invention as defined in the claims, the combination comprises a pharmaceutical acceptable salt of a pharmacological chaperon. In an embodiment of the invention, the pharmaceutically acceptable salt is selected from the group comprising an organic acid and an inorganic salt. In an embodiment of the present invention, an organic acid is selected from the group comprising citrate, acetate, benzoate and tartrate, preferably isofagomine tartrate. In an embodiment of the combination according to the present invention an inorganic salt is selected from the group comprising hydrochloride and hydrobromide, i.e. HCl and HBr, as for example DGJ-HCl.

A person skilled in the art will acknowledge that the symptoms of LSDs vary, depending on the particular disorder or disease, and other variables like the age of onset, and can be mild to severe. They can include developmental delay, movement disorders, seizures, dementia, deafness and/or blindness. Some people with an LSD have enlarged livers (hepatomegaly) and enlarged spleens (splenomegaly), pulmonary and cardiac problems, and bones that grow abnormally.

For the diagnosis of an LSD the majority of patients are initially screened by an enzyme assay, which is the most efficient method to arrive at a definitive diagnosis. In other cases, for example, in some families where the disease-causing mutation(s) is/are known and in certain genetic isolates, mutation analysis may be performed. In addition, after a diagnosis is made by biochemical means, mutation analysis may be performed for certain disorders.

Representative, not-limiting examples of LSDs are described in the following.

Fabry disease, also referred to herein as Fabry's disease, Anderson-Fabry disease, angiokeratoma corporis diffusum and alpha-galactosidase A deficiency, is a rare X-linked inherited LSD, which can cause a wide range of systemic symptoms (James, William D.; Berger, Timothy G.; et al. (2006). Andrews' Diseases of the Skin: clinical Dermatology. Saunders Elsevier. p. 538, ISBN0-7216-2921-0). A deficiency of the enzyme alpha galactosidase A, also referred to herein as a-GAL A, GAL, GLA or alpha-Gal A, due to mutation causes a glycolipid known as globotriaosylceramide, also referred to herein as Gb3, GL-3, or ceramide trihexoside to accumulate within the blood vessels, other tissues, and organs (Karen JK et al., 2005 Dermatol. Online J. 11 (4): 8). This accumulation leads to an impairment of their proper functioning. The DNA mutations which cause the disease are X-linked. The condition affects hemizygous males, as well as homozygous, and potentially heterozygous females, so called carriers. Whilst males typically experience severe symptoms, women can range from being asymptomatic to having severe symptoms. This variability is thought to be due to X-inactivation patterns during embryonic development of the female (James, William D. supra).

Symptoms are typically first experienced in early childhood and can be very difficult to understand; the rarity of Fabry disease to many clinicians sometimes leads to misdiagnoses. Manifestations of the disease usually increase in number and severity while at individual ages. Kidney complications are a common and serious effect of the disease; renal insufficiency and renal failure may worsen throughout life. Proteinuria which causes foamy urine, is often the first sign of kidney involvement. End stage renal failure in males can typically occur in the third decade of life, and is a common cause of death due to the disease.

Fabry disease is indicated when associated symptoms are present, and can be diagnosed by a blood test to measure the level of alpha-galactosidase activity. Chromosomal analysis of the alpha-galactosidase A gene is the most accurate method of diagnosis, and many mutations which cause the disease, have been noted.

Naturally, a-Gal A is likely to be present only at very low levels in the blood, particularly in males. In females, owing to X-inactivation patterns, levels are commonly normal even if the patient is not asymptomatic.

Glycogen storage disease type II, also referred to herein as Pompe disease, Pompe's disease or acid maltase deficiency is an autosomal recessive metabolic disorder which damages muscle and nerve cells throughout the body. It is the only glycogen storage disease with a defect in lysosomal metabolism.

Pompe disease is caused by an accumulation of glycogen in the lysosome due to deficiency of the lysosomal acid alpha-glucosidase enzyme, which is a lysosomal hydrolase. The enzyme degrades alpha -1,4 and alpha -1,6 linkages in glycogen, maltose and isomaltose and is required for the degradation of 1-3% of cellular glycogen. The deficiency of this enzyme results in the accumulation of structurally normal glycogen in lysosomes and cytoplasm in affected individuals. The build-up of glycogen causes progressive muscle weakness (myopathy) throughout the body and affects various body tissues, particularly in the heart, skeletal muscles, liver and nervous system. Excessive glycogen storage within lysosomes may interrupt normal functioning of other organelles and lead to cellular injury.

The enzyme deficiency is caused by a mutation in a gene, namely acid alpha-glucosidase, also referred to herein as acid maltase, on long arm of chromosome 17 at 17q25.2-q25.3 (base pairs 75,689,876 to 75,708,272). The number of mutations described in 2010 was 289 with 67 being non-pathogenic mutations and 197 being pathogenic mutations. The remainder is still being evaluated for their association with disease. Most cases appear to be due to three mutations. A transversion (T → G) mutation is the most common among adults with this disorder. This mutation interrupts a splicing site.

Pompe disease associated symptoms include severe lack of muscle tone, weakness, an enlarged liver (hepatomegaly), and an enlarged heart (cardiomegaly). Mental function is not affected. Development appears normal for the first weeks or months but slowly declines as the disease progresses. Swallowing may become difficult and the tongue may protrude and become enlarged. Most children die from respiratory or cardiac complications before 2 years of age.

Juvenile onset symptoms appear in early to late childhood and include progressive weakness of respiratory muscles in the trunk, diaphragm and lower limbs, as well as exercise intolerance. Intelligence is normal.

Adult onset symptoms also involve generalized muscle weakness and wasting of respiratory muscles in the trunk, lower limbs, and diaphragm. Many patients report respiratory distress, headache at night or upon waking, diminished deep tendon reflexes, and proximal muscle weakness, such as difficulty in climbing stairs. Intellect is not affected. A small number of adult patients live without major symptoms or limitations.

Pompe's disease is one of the infiltrative causes of restrictive cardiomyopathy. The infantile form usually comes to medical attention within the first few months of life. The usual presenting features are cardiomegaly (92%), hypotonia (88%), cardiomyopathy (88%), respiratory distress (78%), muscle weakness (63%), feeding difficulties (57%) and failure to thrive (53%). The main clinical findings include floppy baby appearance, delayed motor milestones and feeding difficulties. Moderate hepatomegaly may be present. Facial features include macroglossia, open mouth, wide open eyes, nasal flaring (due to respiratory distress), and poor facial muscle tone. Cardiopulmonary involvement is manifest by increased respiratory rate, use of accessory muscles for respiration, recurrent chest infections, decreased air entry in the left lower zone (due to cardiomegaly), arrhythmias and evidence of heart failure. Median age at death in untreated cases is 8.7 months and is usually due to cardiorespiratory failure.

The usual initial investigations of this form of the disease include chest X ray, electrocardiogram and echocardiography. Typical findings are those of an enlarged heart with nonspecific conduction defects. Biochemical investigations include serum creatine kinase (typically increased 10-fold) with lesser elevations of the serum aldolase, aspartate transaminase, alanine transaminase and lactic dehydrogenase. Diagnosis is made by estimating the acid alpha glucoside activity in either skin biopsy (fibroblasts), muscle biopsy (muscle cells) or in white blood cells. The choice of sample depends on the facilities available at the diagnostic laboratory.

The disease may be different from a lysosomal storage disease. Such disease may be a disease associated with an LSD. Such disease may be a neurodegenerative disorder. In an embodiment of the combination according to the present invention as defined in the claims. the neurodegenerative disorder is selected from the group comprising Parkinson's disease. In connection therewith, it has to be understood that it has been recently discovered that there is a link between mutations in lysosomal enzymes and neurological disorders different from a lysosomal storage disease.

As one example there is a well-established link between mutations in the glucocerebrosidase gene and Parkinson's disease. More specifically, although Parkinson's disease results from accumulation of synuclein, mutations of the glucocerebrosidase gene occur in Parkinson patients with a frequency that is 6-fold increased (Sidransky et al., NEJM, 2009). Accordingly, Parkinson's disease is a disease associated with an LSD, more particularly with Gaucher's disease. More particularly, it has been shown that beta glucocerebrosidase co-localizes with alpha synuclein, which is the plaque forming material in Parkinson's disease. (Sidransky et al., NEJM, 2009). The present inventors thus currently assume that the misfolding of mutant glucocerebrosidase in Gaucher may enhance accumulation of synuclein. Accordingly, the combination according to the present invention wherein the first constituent is a compound having the ability to rearrange mutant glucocerebrosidase, wherein the mutant glucocerebrosidase has a reduced activity, and wherein the second constituent is Ambroxol and/or a derivative of Ambroxol is suitable for or is for the treatment of Parkinson's disease.

In an embodiment of the invention as defined in the lysosomal protein is glucocerebrosidase and the disease preferably is Parkinson disease.

Parkinson's disease also referred to herein as Parkinson disease or PD, is a degenerative disorder of the central nervous system that often impairs the sufferer's motor skills, speech, and other functions (Jankovic J et al., April 2008, J. Neurol. Neurosurg. Psychiatr. 79 (4): 368-76).

Parkinson's disease also referred to herein preferably as PD, belongs to a group of conditions called movement disorders.

Parkinson's disease is characterized by muscle rigidity, tremor, a slowing of physical movement (bradykinesia) and a loss of physical movement (akinesia) in extreme cases. The primary symptoms are the results of decreased stimulation of the motor cortex by the basal ganglia, normally caused by the insufficient formation and action of dopamine, which is produced in the dopaminergic neurons of the brain, specifically the substantia nigra. Secondary symptoms may include high level cognitive dysfunction and subtle language problems. PD is both chronic and progressive. PD is the most common cause of chronic progressive Parkinsonism, a term which refers to the syndrome of tremor, rigidity, bradykinesia and postural instability. While many forms of Parkinsonism are idiopathic, "secondary" cases may result from toxicity most notably of drugs, head trauma, or other medical disorders.

The term Parkinsonism is used for symptoms of tremor, stiffness, and slowing of movement caused by loss of dopamine. "Parkinson's disease" is the synonym of "primary Parkinsonism", i.e., isolated Parkinsonism due to a neurodegenerative process without any secondary systemic cause. In some cases, it would be inaccurate to say that the cause is "unknown", because a small proportion is caused by genetic mutations. It is possible for a patient to be initially diagnosed with Parkinson's disease but then to develop additional features, requiring revision of the diagnosis (National Institute for Health and Clinical Excellence. Clinical guideline 35: Parkinson's disease. London, June 2006).

The usual anti-Parkinson's medications are typically either less effective or completely ineffective in controlling symptoms; patients may be exquisitely sensitive to neuroleptic medications like haloperidol, so correct differential diagnosis is important. Essential tremor may be mistaken for Parkinson's disease, but lacks all other features besides tremor, and has particular characteristics distinguishing it from Parkinson's disease, such as improvement with beta blockers and alcoholic beverages. PD is not considered to be a fatal disease by itself, but it progresses with time. The average life expectancy of a PD patient is generally lower than for people who do not have the disease. In the late stages of the disease, PD may cause complications such as choking, pneumonia, and falls that can lead to death. The progression of symptoms in PD may take 20 years or more. In some people, however, the disease progresses more quickly. There is no way to predict what course the disease will take for an individual person. With appropriate treatment, most people with PD can live productive lives for many years after diagnosis. There are some indications that PD acquires resistance to drug treatment by evolving into a Parkinson-plus disorder, usually Lewy body dementia, although transitions to progressive supranuclear palsy or multiple system atrophy are not unknown (Apaydin H. et al., January 2002, Archives of Neurology 59 (1): 102-12; Spanaki C. et al., October 2006, Neurology 67 (8): 1518-9.).

Niemann-Pick diseases are genetic diseases which are classified in a subgroup of LSDs called sphingolipidoses or lipid storage disorders in which harmful quantities of fatty compounds, or lipids, accumulate in the spleen, liver, lungs, bone marrow, and brain. In the classic infantile type A variant, a missense mutation causes deficiency of sphingomyelinase. Sphingomyelin is a component of cell membrane including the organellar membrane and so the enzyme deficiency blocks degradation of lipid, resulting in the accumulation of sphingomyelin within lysosomes in the macrophage-monocyte phagocyte lineage. Affected cells become enlarged, sometimes up to 90 microns in diameter, secondary to the distention of lysosomes with sphingomyelin and cholesterol.

Symptoms are related to the organs in which the fatty compounds or lipids accumulate. Enlargement of the liver and spleen (hepatosplenomegaly) may cause reduced appetite, abdominal distension and pain as well as thrombocytopenia secondary to splenomegaly. Sphingomyelin accumulation in the central nervous system, including the cerebellum, results in unsteady gait (ataxia), slurring of speech (dysarthria) and discoordinated swallowing (dysphagia). Basal ganglia dysfunction causes abnormal posturing of the limbs, trunk and face (dystonia) and upper brainstem disease results in impaired voluntary rapid eye movements (supranuclear gaze palsy). More widespread disease involving the cerebral cortex and subcortical structures is responsible for gradual loss of intellectual abilities causing dementia and seizures. Sleep related disorders are also seen, including gelastic cataplexy, which means a sudden loss of muscle tone associated with laughter, and sleep inversion, which means sleepiness during the day and wakefulness at night.

Treatments for Niemann-Pick disease are limited with care being mostly supportive. Anecdotally, organ transplant has been attempted with limited success. Future prospects include enzyme replacement and gene therapy. Bone marrow transplant has been attempted for Type B. Supportive care through nutrition, medication, physical therapy and being followed by specialists can help with quality of life.

The drug Zavesca comprising Miglustat as an active ingredient, provided by Actelion, has been approved at least in the European Union for the treatment of progressive neurological manifestations in adult patients and pediatric patients with Niemann-Pick type C disease, also referred to herein as NPC.

In an embodiment of the invention as defined in the claims, the lysosomal protein having reduced activity is affected in an LSD.

A person skilled in the art will know other LSDs which can be treated with PCT and to which the combination according to the present invention may be applied.

An LSD, as preferably used herein, is characterized in at least one of the following:
a) a defective metabolism of glycosaminoglycans such as in, e.g., mucopolysaccharidosis, also referred to herein preferably as MPS, comprising among others MPS I, such as Hurler, Hurler-Scheie, Scheie; MPS II, such as Hunter; MPS III such as Sanfilippo syndrome type A, Sanfilippo syndrome type B, Sanfilippo syndrome type C and Sanfilippo syndrome type D; MPS IV such as Morquio type A and B; MPS VI such as Maroteaux-Lamy; MPS VII such as Sly; MPS IX such as hyaluronidase deficiency, multiple sulfatase deficiency;
b) a defective degradation of glycan portion of glycoprotein such as in, among others, aspartylglucosaminuria; fucosidosis, type I and II; mannosidosis; sialidosis, type I and II;
c) a defective degradation of glycogen such as in, e.g., Pompe's disease;
d) a defective degradation of sphingolipid components such as in, among others, acid sphingomyelinase deficiency such as Niemann-Pick A & B; Fabry disease; Farber disease; Gaucher disease type I, II and III, GM1 gangliosidosis, type I, II and III; GM2 gangliosidosis such as Tay-Sachs type I, II, III and Sandhoff; Krabbe disease; metachromatic leukodystrophy, type I, II and III;
e) a defective degradation of polypeptides such as in, e.g., pycnodysostosis;
f) a defective degradation or transport of cholesterol, cholesterol esters, or other complex lipids such as in, e.g. neuronal ceroid lipofuscinosis, type I, II, III and IV;
g) multiple deficiencies of lysosomal enzymes such as in, e.g., galactosialidosis, Danon Disease, GM2 gangliosidosis, mucolipidosis type II and mucolipidosis III; and
h) transport and trafficking defects such as in, e.g., cystinosis; Danon disease; mucolipidosis type IV; Niemann-Pick type C; infantile sialic acid storage disease; Salla disease.

In case an LSD is an LSD having a defective degradation of a sphingolipid component, it will be understood by a person skilled in the art that wherein a degradation of a sphingolipid component is defective, preferably also the degeneration of several sphingolipid components may be defective.

The lysosomal protein may be selected from the group comprising an enzyme, a membrane transport protein, a trans-membrane protein or a soluble non-enzymatic protein. Preferably, the reduced activity is reduced as a result of at least one mutation within the nucleotide sequence of a gene coding for the lysosomal protein. The gene product of said mutant gene is more preferably the protein having reduced activity. Most preferably said reduced activity results in an LSD and/or the reduced activity is reduced due to a mutation of the amino acid sequence of the lysosomal protein.

The lysosomal protein being an enzyme said lysosomal enzyme may be selected from the group comprising a lysosomal hydrolase and a phosphotransferase.

The phosphotransferase may bee selected from the group comprising N-acetylglucosamine-1-phosphate transferase.

The lysosomal storage disease is a lysosomal storage disease having a defective degradation of a glycan portion of a glycoprotein. In connection therewith, it will be acknowledged that N-glycosylated proteins, in principle, can be affected by a lack of or a reduction of degradation resulting in lysosomal storage. In case of aspartylglucosaminuria, for example, Asn-GlcNAc units accumulate in the lysosome, whereas in case of manosidase the oligosaccharides Man(α1 → 3)Man(β1 → 4)GlcNac, Man(α1 → 2)Man(α1 → 3)Man(β1 → 4)GlcNac and Man(α1 → 2)Man(α1 → 2)Man(al → 3)Man(β1 → 4)GlcNac are formed (DeGasperi R et al., J Biol Chem 1991, 266:16556-16563.). It will be understood that in the latter case high mannosylated proteins are affected with preference. As to fucosidosis, fucose containing sugars are degraded. It will be understood that fucose is a sugar which is capable of forming the last moiety of a chain or is capable of linking two different sugars.

A person skilled in the art will acknowledge that, for example, lysosomal proteins, secretory proteins and membrane proteins, such as adhesion proteins and/or transporter proteins, can be glycosylated.

The lysosomal storage disease may be a lysosomal storage disease having a defective degradation of polypeptides. In connection therewith, it will be understood that Cathepsin K is a cysteine protease. Accordingly, all proteins comprising a cysteine residue and a nearby amino acid having a basic side chain are candidates for degradation by Cathepsin K. It will also be acknowledged that Cysteine proteases carry out various tasks in different physiological processes of the cell.

The lysosomal storage disease may be a lysosomal storage disease having a defective degradation or transport of cholesterol, cholesterol esters and/or other complex lipids. A complex lipid as used herein preferably refers to lipids comprising lipofuscine. In connection therewith, it will be understood that lipofuscine accumulates in NCLs and comprise a protein moiety, a lipid moiety, preferably a sugar and preferably metal ion, wherein the lipid moiety results from oxidation of unsaturated fatty acids.

The lysosomal storage disease may be a lysosomal storage disease having multiple deficiencies of lysosomal enzymes. In an embodiment the lysosomal storage disease having multiple deficiencies of lysosomal enzymes is selected from the group comprising galactosialidosis, Danon Disease (LAMP2), pycnodysostosis (Cathepsin K), multiple sulfatase deficiency (Fgly-Generating Enzyme), GM2 gangliosidosis (GM2-Activator), mucolipidosis type II and mucolipidosis type III (N-acetylglucosaminyl-1-phosphotransferase). In connection therewith "multiple deficiencies" as used herein preferably means at least two deficiencies of at least two different enzyme activities in the lysosome, preferably of at least two different enzymes. As an example, the gene CTSA is mutated in galactosialidosis. CTSA encodes Cathepsin A (CatA), which is a factor forming complex(es) with other lysosomal hydrolases (beta-galactosidase). In other words, CatA is important for the functioning of different hydrolases. If not present various degradation processes will not function properly. Accordingly, multiple deficiencies are caused. In case of mucolipidosis the same principle applies: In mucolipidosis II the enzyme N-acetylglucosaminyl-1-phosphotransferase is mutated, which is responsible for marking lysosomale enzymes for their transport into the lysosome. The mutation in said gene leading to a reduced activity of the protein/gene product results in multiple deficiencies of enzyme activities which occur in the lysosome. In other words, the mutated genes the mutation of which results in multiple deficiencies assist other enzymes in correct processing and assist said enzymes in their catalytic function, respectively.

The lysosomal protein may be an enzyme, more preferably, the enzyme is selected from the group comprising mutant α-galactosidase A preferably resulting in Fabry disease, mutant acid α-glucosidase preferably resulting in Pompe disease, mutant acid sphingomyelinase preferably resulting in Niemann-Pick diseases type A or B, mutant N-acetylglucosamine-1-phosphotransferase preferably resulting in mucolipidosis type II and IIIA, mutant beta-hexosaminidase A/B preferably resulting in Tay-Sachs' or Sandhoff disease, mutant beta galactosidase preferably resulting in GM1 gangliosidosis, mutant acid Lipase preferably resulting in Wolman Disease and/or CESD, mutant galactosylceramidase preferably resulting in Krabbe Disease, mutant arylsulfatase B preferably resulting in Maroteaux-Lamy Syndrome, i.e. mucopolysaccharidosis VI, mutant alpha-L iduronidase preferably resulting in Hurler Syndrome, i.e. mucopolysaccharidosis I, mutant alpha-mannosidase preferably resulting in alpha-mannosidosis, mutant beta-glucuronidase preferably resulting in Sly Syndrome, mutant arylsulfatase A preferably resulting in metachromatic leukodystrophy, mutant NPC1 protein preferably resulting in Niemann-Pick disease type C, mutant α-L-iduronidase preferably resulting in Hurler-Scheie syndrome, mutant α-L-iduronidase preferably resulting in Scheie syndrome, mutant iduronatsulfatsilfatase preferably resulting in Hunter syndrome, mutant α-N-acetylglukoseamidase preferably resulting in Sanfilippo syndrome type B, mutant acetyl-CoA: α-glukosaminid-N-acetyltransferase preferably resulting in Sanfilippo syndrome type C, mutant N-acetyl-glukosamin-6-sulfatsulfatase preferably resulting in Sanfilippo syndrome type D, mutant N-acetyl-glukosamin-6-sulfatsulfatase preferably resulting in Morquio type A, mutant β-galactosidasepreferably resulting in Morquio type B, mutant N-acetylgalactosamin-4-sulfat-sulfatase preferably resulting in Maroteaux-Lamy, mutant β-glucuronidase preferably resulting in Sly, mutant hyaluronidase preferably resulting in hyaluronidase deficiency; mutant N-acetylglucosamine-1- phosphatetransferase preferably resulting in N-acetylglucosamine-1- phosphate transferase mucolipidose III gamma and mutation of multiple sulfatase enzymes preferably resulting in multiple sulfatase deficiency.

The combination may comprise a first constituent and a second constituent, wherein the first constituent is a compound having the ability to rearrange a lysosomal protein, wherein the lysosomal protein has a reduced activity, wherein the first constituent is castegine A3 or a pharmaceutically acceptable salt thereof; and wherein the second constituent is Ambroxol and/or a derivative thereof, preferably the lysosomal protein is α-galactosidase A, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Fabry's disease.

The combination may comprise a first constituent and a second constituent, wherein the first constituent is a compound having the ability to rearrange a lysosomal protein, wherein the lysosomal protein has a reduced activity, wherein the first constituent is castegine B2 or a pharmaceutically acceptable salt thereof; and wherein the second constituent is Ambroxol and/or a derivative thereof, preferably the lysosomal protein is α-galactosidase A, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Fabry's disease.

The combination may comprise a first constituent and a second constituent, wherein the first constituent is a compound having the ability to rearrange a lysosomal protein, wherein the lysosomal protein has a reduced activity, wherein the first constituent is N-methyl calystegine A3 or a pharmaceutically acceptable salt thereof; and wherein the second constituent is Ambroxol and/or a derivative thereof, preferably the lysosomal protein is α-galactosidase A, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Fabry's disease.

The combination may comprise a first constituent and a second constituent, wherein the first constituent is a compound having the ability to rearrange a lysosomal protein, wherein the lysosomal protein has a reduced activity, wherein the first constituent is N-methyl calystegine B2 or a pharmaceutically acceptable salt thereof; and wherein the second constituent is Ambroxol and/or a derivative thereof, preferably the lysosomal protein is α-galactosidase A, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Fabry's disease.

The combination may comprise a first constituent and a second constituent, wherein the first constituent is a compound having the ability to rearrange a lysosomal protein, wherein the lysosomal protein has a reduced activity, wherein the first constituent is 2-N-acetylamino-isofagomine or a pharmaceutically acceptable salt thereof; and wherein the second constituent is Ambroxol and/or a derivative thereof, preferably the lysosomal protein is beta-hexosaminidase A preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, Fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Tay-Sachs disease.

The combination may comprise a first constituent and a second constituent, wherein the first constituent is a compound having the ability to rearrange a lysosomal protein, wherein the lysosomal protein has a reduced activity, wherein the first constituent is 1,2-dideoxy-2-acetamido-nojirimycin or a pharmaceutically acceptable salt thereof; and wherein the second constituent is Ambroxol and/or a derivative thereof, preferably the lysosomal protein is beta-hexosaminidase A preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, Fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Tay-Sachs disease.

The combination may comprise a first constituent and a second constituent, wherein the first constituent is a compound having the ability to rearrange a lysosomal protein, wherein the lysosomal protein has a reduced activity, wherein the first constituent is nagastain or a pharmaceutically acceptable salt thereof; and wherein the second constituent is Ambroxol and/or a derivative thereof, preferably the lysosomal protein is beta-hexosaminidase A preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Tay-Sachs disease.

The combination may comprise a first constituent and a second constituent, wherein the first constituent is a compound having the ability to rearrange a lysosomal protein, wherein the lysosomal protein has a reduced activity, wherein the first constituent is nagastain or a pharmaceutically acceptable salt thereof; and wherein the second constituent is Ambroxol and/or a derivative thereof, preferably the lysosomal protein is beta-hexosaminidase B, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Sandhoff disease.

The combination may comprise a first constituent and a second constituent, wherein the first constituent is a compound having the ability to rearrange a lysosomal protein, wherein the lysosomal protein has a reduced activity, wherein the first constituent is 2-N-acetylamino-isofagomine or a pharmaceutically acceptable salt thereof; and wherein the second constituent is Ambroxol and/or a derivative thereof, preferably the lysosomal protein is beta-hexosaminidase B, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, Fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Sandhoff disease.

The combination may comprise a first constituent and a second constituent, wherein the first constituent is a compound having the ability to rearrange a lysosomal protein, wherein the lysosomal protein has a reduced activity, wherein the first constituent is 1,2-dideoxy-2-acetamido-nojirimycin or a pharmaceutically acceptable salt thereof; and wherein the second constituent is Ambroxol and/or a derivative thereof, preferably the lysosomal protein is beta-hexosaminidase B, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Sandhoff disease.

The combination may comprise a first constituent and a second constituent, wherein the first constituent is a compound having the ability to rearrange a lysosomal protein, wherein the lysosomal protein has a reduced activity, wherein the first constituent is 4-epi-isofagomine or a pharmaceutically acceptable salt thereof; and wherein the second constituent is Ambroxol and/or a derivative thereof, preferably the lysosomal protein is acid beta-galactosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is GM1-gangliosidosis.

The combination may comprise a first constituent and a second constituent, wherein the first constituent is a compound having the ability to rearrange a lysosomal protein, wherein the lysosomal protein has a reduced activity, wherein the first constituent is 4-epi-isofagomine or a pharmaceutically acceptable salt thereof; and wherein the second constituent is Ambroxol and/or a derivative thereof, preferably the lysosomal protein is acid beta-galactosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Morquio disease B.

The combination may comprise a first constituent and a second constituent, wherein the first constituent is a compound having the ability to rearrange a lysosomal protein, wherein the lysosomal protein has a reduced activity, wherein the first constituent is 4-epi-isofagomine or a pharmaceutically acceptable salt thereof; and wherein the second constituent is Ambroxol and/or a derivative thereof, preferably the lysosomal protein is galactocerebrosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Krabbe disease.

The combination may comprise a first constituent and a second constituent, wherein the first constituent is a compound having the ability to rearrange a lysosomal protein, wherein the lysosomal protein has a reduced activity, wherein the first constituent is 1-deoxygalactonojirimycin or a pharmaceutically acceptable salt thereof; and wherein the second constituent is Ambroxol and/or a derivative thereof, preferably the lysosomal protein is acid beta-galactosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is GM1-gangliosidosis.

The combination may comprise a first constituent and a second constituent, wherein the first constituent is a compound having the ability to rearrange a lysosomal protein, wherein the lysosomal protein has a reduced activity, wherein the first constituent is 1-deoxygalactonojirimycin or a pharmaceutically acceptable salt thereof; and wherein the second constituent is Ambroxol and/or a derivative thereof, preferably the lysosomal protein is acid beta-galactosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Morquio disease B.

The combination may comprise a first constituent and a second constituent, wherein the first constituent is a compound having the ability to rearrange a lysosomal protein, wherein the lysosomal protein has a reduced activity, wherein the first constituent is 1-deoxygalactonojirimycin or a pharmaceutically acceptable salt thereof; and wherein the second constituent is Ambroxol and/or a derivative thereof, preferably the lysosomal protein is galactocerebrosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Krabbe disease.

The combination may comprise a first constituent and a second constituent, wherein the first constituent is a compound having the ability to rearrange a lysosomal protein, wherein the lysosomal protein has a reduced activity, wherein the first constituent is 1-deoxymannojirimycin or a pharmaceutically acceptable salt thereof; and wherein the second constituent is Ambroxol and/or a derivative thereof, preferably the lysosomal protein is acid alpha-manosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is alpha-manosidosis.

The combination may comprise a first constituent and a second constituent, wherein the first constituent is a compound having the ability to rearrange a lysosomal protein, wherein the lysosomal protein has a reduced activity, wherein the first constituent is swainsonine or a pharmaceutically acceptable salt thereof; and wherein the second constituent is Ambroxol and/or a derivative thereof, preferably the lysosomal protein is acid alpha-manosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is alpha-manosidosis.

The combination may comprise a first constituent and a second constituent, wherein the first constituent is a compound having the ability to rearrange a lysosomal protein, wherein the lysosomal protein has a reduced activity, wherein the first constituent is mannostatin A or a pharmaceutically acceptable salt thereof; and wherein the second constituent is Ambroxol and/or a derivative thereof, preferably the lysosomal protein is acid alpha-manosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is alpha-manosidosis.

The combination may comprise a first constituent and a second constituent, wherein the first constituent is a compound having the ability to rearrange a lysosomal protein, wherein the lysosomal protein has a reduced activity, wherein the first constituent is 2-hydroxy-isofagomine or a pharmaceutically acceptable salt thereof; and wherein the second constituent is Ambroxol and/or a derivative thereof, preferably the lysosomal protein is acid beta-manosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is beta-manosidosis.

The combination may comprise a first constituent and a second constituent, wherein the first constituent is a compound having the ability to rearrange a lysosomal protein, wherein the lysosomal protein has a reduced activity, wherein the first constituent is 1-deoxyfuconojirimycin or a pharmaceutically acceptable salt thereof; and wherein the second constituent is Ambroxol and/or a derivative thereof, preferably the lysosomal protein is acid alpha-fucosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is fucosidosis.

The combination may comprise a first constituent and a second constituent, wherein the first constituent is a compound having the ability to rearrange a lysosomal protein, wherein the lysosomal protein has a reduced activity, wherein the first constituent is beta-homofuconojirimycin or a pharmaceutically acceptable salt thereof; and wherein the second constituent is Ambroxol and/or a derivative thereof, preferably the lysosomal protein is acid alpha-fucosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is fucosidosis.

The combination may comprise a first constituent and a second constituent, wherein the first constituent is a compound having the ability to rearrange a lysosomal protein, wherein the lysosomal protein has a reduced activity, wherein the first constituent is 2,5-imino-1,2,5-trideoxy-L-glucitol or a pharmaceutically acceptable salt thereof; and wherein the second constituent is Ambroxol and/or a derivative thereof, preferably the lysosomal protein is acid alpha-fucosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is fucosidosis.

The combination may comprise a first constituent and a second constituent, wherein the first constituent is a compound having the ability to rearrange a lysosomal protein, wherein the lysosomal protein has a reduced activity, wherein the first constituent is 2,5-dideoxy-2,5-imino-D-fucitol or a pharmaceutically acceptable salt thereof; and wherein the second constituent is Ambroxol and/or a derivative thereof, preferably the lysosomal protein is acid alpha-fucosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is fucosidosis.

The combination may comprise a first constituent and a second constituent, wherein the first constituent is a compound having the ability to rearrange a lysosomal protein, wherein the lysosomal protein has a reduced activity, wherein the first constituent is 2,5-imino-1,2,5-trideoxy-D-altritol or a pharmaceutically acceptable salt thereof; and wherein the second constituent is Ambroxol and/or a derivative thereof, preferably the lysosomal protein is acid alpha-fucosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is fucosidosis.

The combination may comprise a first constituent and a second constituent, wherein the first constituent is a compound having the ability to rearrange a lysosomal protein, wherein the lysosomal protein has a reduced activity, wherein the first constituent is 1,2-dideoxy-2-N-acetamido-nojirimycin or a pharmaceutically acceptable salt thereof; and wherein the second constituent is Ambroxol and/or a derivative thereof, preferably the lysosomal protein is alpha-N-Acetylglucosaminidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Sanfilippo disease B.

The combination may comprise a first constituent and a second constituent, wherein the first constituent is a compound having the ability to rearrange a lysosomal protein, wherein the lysosomal protein has a reduced activity, wherein the first constituent is 1,2-dideoxy-2-N-acetamido-galactonojirimycin or a pharmaceutically acceptable salt thereof; and wherein the second constituent is Ambroxol and/or a derivative thereof, preferably the lysosomal protein is alpha-N-Acetylgalactosaminidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Schindler-Kanzaki disease.

The combination may comprise a first constituent and a second constituent, wherein the first constituent is a compound having the ability to rearrange a lysosomal protein, wherein the lysosomal protein has a reduced activity, wherein the first constituent is 1-deoxyduronojirimycin or a pharmaceutically acceptable salt thereof; and wherein the second constituent is Ambroxol and/or a derivative thereof, preferably the lysosomal protein is alpha-L-iduronidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Hurler-Scheie disease.

The combination may comprise a first constituent and a second constituent, wherein the first constituent is a compound having the ability to rearrange a lysosomal protein, wherein the lysosomal protein has a reduced activity, wherein the first constituent is 2-carboxy-3,4,5-trideoxypiperidine or a pharmaceutically acceptable salt thereof; and wherein the second constituent is Ambroxol and/or a derivative thereof, preferably the lysosomal protein is alpha-L-Iduronidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Hurler-Scheie disease.

The combination may comprise a first constituent and a second constituent, wherein the first constituent is a compound having the ability to rearrange a lysosomal protein, wherein the lysosomal protein has a reduced activity, wherein the first constituent is 6-carboxy-isofagomine or a pharmaceutically acceptable salt thereof; and wherein the second constituent is Ambroxol and/or a derivative thereof, preferably the lysosomal protein is beta-glucuronidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Sly disease.

The combination may comprise a first constituent and a second constituent, wherein the first constituent is a compound having the ability to rearrange a lysosomal protein, wherein the lysosomal protein has a reduced activity, wherein the first constituent is 2-carboxy-3,4,5-trideoxypiperidine or a pharmaceutically acceptable salt thereof; and wherein the second constituent is Ambroxol and/or a derivative thereof, preferably the lysosomal protein is beta-glucuronidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Sly disease.

The combination may comprise a first constituent and a second constituent, wherein the first constituent is a compound having the ability to rearrange a lysosomal protein, wherein the lysosomal protein has a reduced activity, wherein the first constituent is 2,6-dideoxy-2,6-imino-sialic acid or a pharmaceutically acceptable salt thereof; and wherein the second constituent is Ambroxol and/or a derivative thereof, preferably the lysosomal protein is sialidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is sialidosis.

The combination may comprise a first constituent and a second constituent, wherein the first constituent is a compound having the ability to rearrange a lysosomal protein, wherein the lysosomal protein has a reduced activity, wherein the first constituent is siastin B or a pharmaceutically acceptable salt thereof; and wherein the second constituent is Ambroxol and/or a derivative thereof, preferably the lysosomal protein is sialidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is sialidosis.

The combination may comprise a first constituent and a second constituent, wherein the first constituent is a compound having the ability to rearrange a lysosomal protein, wherein the lysosomal protein has a reduced activity, wherein the first constituent is N-nonyl-deoxynojirimycin (NN-DNJ) or a pharmaceutically acceptable salt thereof; and wherein the second constituent is Ambroxol and/or a derivative thereof, preferably the lysosomal protein is glucocerebrosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Parkinson disease.

The combination may comprise a first constituent and a second constituent, wherein the first constituent is a compound having the ability to rearrange a lysosomal protein, wherein the lysosomal protein has a reduced activity, wherein the first constituent is N-octyl-2,5-anhydro-2,5-imino-D-glucitol or a pharmaceutically acceptable salt thereof; and wherein the second constituent is Ambroxol and/or a derivative thereof, preferably the lysosomal protein is glucocerebrosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Parkinson disease.

The combination may comprise a first constituent and a second constituent, wherein the first constituent is a compound having the ability to rearrange a lysosomal protein, wherein the lysosomal protein has a reduced activity, wherein the first constituent is N-octyl-isofagomine or a pharmaceutically acceptable salt thereof; and wherein the second constituent is Ambroxol and/or a derivative thereof, preferably the lysosomal protein is glucocerebrosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Parkinson disease.

The combination may comprise a first constituent and a second constituent, wherein the first constituent is a compound having the ability to rearrange a lysosomal protein, wherein the lysosomal protein has a reduced activity, wherein the first constituent is N-octyl-beta-valienamine (NOV) or a pharmaceutically acceptable salt thereof; and wherein the second constituent is Ambroxol and/or a derivative thereof, preferably the lysosomal protein is glucocerebrosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Parkinson disease.

The combination may comprise a first constituent and a second constituent, wherein the first constituent is a compound having the ability to rearrange a lysosomal protein, wherein the lysosomal protein has a reduced activity, wherein the first constituent is isofagomine (IFG) or a pharmaceutically acceptable salt thereof; and wherein the second constituent is Ambroxol and/or a derivative thereof, preferably the lysosomal protein is glucocerebrosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Parkinson disease.

The combination may comprise a first constituent and a second constituent, wherein the first constituent is a compound having the ability to rearrange a lysosomal protein, wherein the lysosomal protein has a reduced activity, wherein the first constituent is calystegine A3 or a pharmaceutically acceptable salt thereof; and wherein the second constituent is Ambroxol and/or a derivative thereof, preferably the lysosomal protein is glucocerebrosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Parkinson disease.

The combination may comprise a first constituent and a second constituent, wherein the first constituent is a compound having the ability to rearrange a lysosomal protein, wherein the lysosomal protein has a reduced activity, wherein the first constituent is calystegine B1 or a pharmaceutically acceptable salt thereof; and wherein the second constituent is Ambroxol and/or a derivative thereof, preferably the lysosomal protein is glucocerebrosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Parkinson disease.

The combination may comprise a first constituent and a second constituent, wherein the first constituent is a compound having the ability to rearrange a lysosomal protein, wherein the lysosomal protein has a reduced activity, wherein the first constituent is calystegine B2 or a pharmaceutically acceptable salt thereof; and wherein the second constituent is Ambroxol and/or a derivative thereof, preferably the lysosomal protein is glucocerebrosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Parkinson disease.

The combination may comprise a first constituent and a second constituent, wherein the first constituent is a compound having the ability to rearrange a lysosomal protein, wherein the lysosomal protein has a reduced activity, wherein the first constituent is calystegine C1 or a pharmaceutically acceptable salt thereof; and wherein the second constituent is Ambroxol and/or a derivative thereof, preferably the lysosomal protein is glucocerebrosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Parkinson disease.

The combination may comprise a first constituent and a second constituent, wherein the first constituent is a compound having the ability to rearrange a lysosomal protein, wherein the lysosomal protein has a reduced activity, wherein the first constituent is 1,5-dideoxy-1,5-iminoxylitol (DIX) or a pharmaceutically acceptable salt thereof; and wherein the second constituent is Ambroxol and/or a derivative thereof, preferably the lysosomal protein is glucocerebrosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Parkinson disease.

The combination may comprise a first constituent and a second constituent, wherein the first constituent is a compound having the ability to rearrange a lysosomal protein, wherein the lysosomal protein has a reduced activity, wherein the first constituent is alpha-1-C-nonyl-DIX or a pharmaceutically acceptable salt thereof; and wherein the second constituent is Ambroxol and/or a derivative thereof, preferably the lysosomal protein is glucocerebrosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Parkinson disease.

The combination may comprise a first constituent and a second constituent, wherein the first constituent is a compound having the ability to rearrange a lysosomal protein, wherein the lysosomal protein has a reduced activity, wherein the first constituent is alpha-1-C-octyl-1-DNJ or a pharmaceutically acceptable salt thereof; and wherein the second constituent is Ambroxol and/or a derivative thereof, preferably the lysosomal protein is glucocerebrosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Parkinson disease.

The combination may comprise a first constituent and a second constituent, wherein the first constituent is a compound having the ability to rearrange a lysosomal protein, wherein the lysosomal protein has a reduced activity, wherein the first constituent is N-butyl-DNJ or a pharmaceutically acceptable salt thereof; and wherein the second constituent is Ambroxol and/or a derivative thereof, preferably the lysosomal protein is glucocerebrosidase, preferably the combination is for use in the treatment or prevention of a disease, whereby the disease is selected from the group comprising Fabry disease, GM1-gangliosidosis, Pompe disease, Krabbe disease, Morquio disease B, alpha-mannosidosis, beta-mannosidosis, fucosidosis, San filippo disease B, Schindler-Kanzaki-disease, Tay-Sachs disease, Sandhoff disease, Hurler-Scheie disease, Sly disease, sialidosis and Parkinson disease, more preferably the disease is Parkinson disease.

It will be understood that a person skilled in the art will be aware of other proteins having reduced activity in respective LSDs, which may be treated with the combination according to the present invention as defined in the claims or as disclosed herein.

The lysosomal protein may be a trans-membrane protein selected from the group comprising mutant NPC1 preferably resulting in Nieman-Pick disease type C1.

The lysosomal protein may be a soluble non-enzymatic protein selected from the group comprising NPC2, preferably resulting in Niemann-Pick disease type C2.

It will be understood that a person skilled in the art will be aware of other proteins having reduced activity in respective LSDs, which may be treated with the combination according to the present invention as defined in the claims or as disclosed herein.

Ambroxol, also referred to herein as 2-amino-3,5-dibromo-N-[trans-4-hydroxy cyclohexyl]benzylamine, is a drug from the expectorant class widely used for increasing surfactant secretion in lung and for decreasing mucus viscosity (Disse, B.G. et al., 1987, Eur. J. Respir. Dis. 153, 255-262). Ambroxol has been reported to protect alpha-1-proteinase inhibitor from oxidative inactivation and to inhibit the generation of reactive oxygen species from activated phagocytes in vitro (Rozniecki, J. and Nowak, D., 1987, Lu Resp. 4, 14-15; Winsel, K. and Becher, G., 1922, Eur. Resp. J. 5, 289). Furthermore, pulse radiolysis experiments showed that Ambroxol is a good scavenger of the primary water radical species, particularly e⁻_{aq} an ·OH radicals (Tamba, M. and Torreffiani, A. 2001, Rad. Phys. And Chem. 60, 43-52).

Ambroxol as used herein, preferably has the formula according to formula (I):

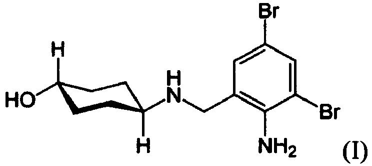

Ambroxol is the active ingredient of Mucosolvan, Ambrosan or Mucoangin. The compound is a mucoactive drug with several properties including secretolytic and secretomotoric actions that restore physiological clearance mechanisms of the respiratory tract which play an important role in the body's natural defense mechanisms. It stimulates synthesis and release of surfactant by type II pneumocytes. Surfactants act as anti-glue factors by reducing the adhesion of mucus to the bronchial wall, in improving its transport and in providing protection against infection and irritating agents (Sanderson RJ et al. Respir Phys 1976; 27: 379-392.; Kido H et al. Biol Chem 2004; 385: 1029-1034). Furthermore, Ambroxol is indicated as secretolytic therapy in bronchopulmonary diseases associated with abnormal mucus secretion and impaired mucus transport. It promotes mucus clearance, facilitates expectoration and eases productive cough, allowing patients to breathe freely and deeply (Malerba and Ragnoli, Expert Opin Drug Metab Toxicol 2008; 4(8): 1119-1129). There are many different formulations developed since the first marketing authorization in 1978. A major product is the syrup with two concentrations of the compound, 30 mg/ml and 315 mg/ml, which can be given in adults and children from the age of 1 year on and even from infant in the latter concentration. Other formulations are tablets containing 30 mg or 60 mg, and a pastille to be sucked with 15 mg Ambroxol. There is also a sustained release form with 75 mg to be given once a day. Ambroxol is also available as dry powder sachets, inhalation solution, drops and ampoules as well as effervescent tablets. Ambroxol also provides pain relief in acute sore throat. Pain in sore throat is the hallmark of acute pharyngitis (de Mey et al., Arzneimittelforschung 2008; 58(11): 557 - 568).

Ambroxol is a very potent inhibitor of the neuronal Na⁺ channels (Weiser T., Neurosci Lett. 2006; 395: 179 - 184). This property led to the development of a lozenge containing 20 mg of Ambroxol. Many state-of-the-art clinical studies (de Mey et al., supra) have demonstrated the efficacy of Ambroxol in relieving pain in acute sore throat, with a fast onset of action and a long duration of effect of at least 3 hours. Additional anti-inflammatory properties of Ambroxol are of clinical relevance since treatment lead to a marked reduction of redness of the patient's sore throat.

Lysosomal protein having reduced activity, such as mutant lysosomal hydrolases, such as alpha-galactosidase A affected in Fabry disease or acid α-glucosidase affected in Pompe disease form a heterogeneous group of enzymes and moreover pharmacologic chaperones such as DGJ or NB-DNJ, specifically act only in relation to a certain LSD, i.e. in relation to a particularly specific enzyme as has already been outlined above.

Moreover, DGJ treatment does also not address all mutations of alpha-galactosidase A (alpha-Gal A) related to Fabry disease, which inhibits the broad application of the compound in clinical use. This was shown when Fabry patient-derived T-cells were grown in the presence of DGJ. Alpha-Gal A activity increased to more than 50% of normal in several mutations but was unaffected in others. (Shin SH et al., Pharmacogenet Genomics. 2008 Sep; 18(9):773-80.).

It is therefore a common believe in the field of LSDtherapy that the response of a certain mutant enzyme affected in an LSD to a certain pharmacological chaperone is dependent on the affected enzyme, on (a)certain mutation(s) of the enzyme and thus on the certain patients (Wu et al., supra).

It is thus the merit of the present inventors having found that the combined use of a pharmacological chaperone and Ambroxol is suitable for increasing the activity of a mutant protein in the treatment of an LSD such as Fabry disease compared to the application of said pharmacological chaperone alone.

At least insofar the present invention turns away from the teaching of prior art in that administering Ambroxol is useful independent from the particular LSD and the particular pharmacological chaperone in that Ambroxol is administered in addition to a pharmacological chaperone specific for the particular treated disease causing an increase of the activity of a mutant enzyme compared to the administering of said pharmacological chaperone alone, although, preferably, Ambroxol is not causing an increase of the activity of said mutant enzyme when administered alone.

A person skilled in the art will immediately acknowledge that the combination according to the present invention as defined in the claims is able to extend the use of a compound having the ability to rearrange a lysosomal protein having reduced activity, such as a pharmacological chaperone, e.g. an iminosugar such as DGJ, to a wider range of indications as if administered alone in that the treatment with the combination according to the present invention allows the treatment of the protein having reduced activity due to (a) particular mutation(s) which may be only insufficiently treated with the compound having the ability to rearrange a lysosomal protein having reduced activity, such as DGJ, alone. More specifically, the additional application of Ambroxol, Bromhexine and/or a derivative thereof comprised in the combination of the present invention may enhance the activity of the mutant protein having reduced activity to a level sufficient to prevent the patient from having symptoms, from the onset of the disease or at least for suffering from less severe symptoms. In contrast, the responsiveness of a mutant protein having said particular mutation to the treatment with DGJ alone may not be sufficient to induce a therapeutic effect.

Valenzano et al. (Valenzano et al., Assay Drug Dev Technol. 2011 Jun;9(3):213-35), for example, describe that in most LSDs, 1% to 6% of normal activity has been estimated to be sufficient to delay or prevent disease onset or to yield a milder form of the disease. For instance, 1%-2% of normal α-iduronidase activity has been reported in mild cases of MPS I (Hopwood and Muller, Clin Sci (Lond). 1979 Sep;57(3):265-72) ; <1% of normal GCase activity has been reported in mild Gaucher disease (Desnick RJ and Fan JQ, In: Futerman AH, editor; Zimran A, editor. Gaucher Disease. CRC Press; Boca Raton, FL: 2006. p. 544); 6% of normal β-galactosidase activity has been reported in late-onset GM1 gangliosidosis (Suzuki Y et al., Perspect Med Chem. 2009;3:7-19); 2-4% of normal β-hexosaminidase activity has been reported in the adult form of GM2 gangliosidosis (Conzelmann et al. Am J Hum Genet. 1983 Sep;35(5):900-13); and 10% of normal enzyme activity seems to prevent GM1 and GM2 gangliosidoses altogether (Leinekugel P. et al., Hum Genet. 1992;88:513-523; Mahuran DJ., Biochim Biophys Acta. 1999;1455:105-138).

It will thus be acknowledged by a person skilled in the art that 10%, preferably 20% and more preferably 25% of protein activity compared to wild type protein activity is considered to be sufficient for a therapeutically effective treatment, i.e. a treatment sufficient to induce a therapeutic effect, and/or an enhancement of the activity of the mutant protein having reduced activity to a level sufficient to prevent the patient from having symptoms, from the onset of the disease or at least for suffering from less severe symptoms.

For example, enhancing the activity of a mutant protein compared to wild type protein activity to 5% by administering DGJ alone, the activity of the mutant protein being 1% without any treatment, will not be sufficient to prevent the onset of disease, i.e. the treatment will not be therapeutically effective. By contrast, the application of the combination according to the present invention, going along with additional application of Ambroxol, bromhexine and/or a derivative thereof may further enhance the mutant protein's activity to 20%, thus being therapeutically effective and prevent the patient from suffering from symptoms, or at least may cause less severe symptoms.

In other words, the present inventors found that applying DGJ and Ambroxol in combination according to the present invention as defined in the claims is able to increase the activity of mutant protein measured in accordance with the cell-culture system as described herein to a level which would prompt a skilled clinician to evaluate the patient's health status as "healthy", i.e. having an activity of mutant protein which usually does not result in symptoms otherwise caused by the mutant protein's reduced activity, whereas the treatment with DGJ alone results in protein activity of the mutant protein which would prompt the skilled clinician to evaluate the patient's health status as being "unclear" or as "diseased".

The present inventors also surprisingly observed an increase in the activity of a mutant protein upon treatment with the combination according to the present invention as defined in the claims compared to the activity of the mutant protein upon treatment with the compound having the ability to rearrange a lysosomal protein having reduced activity alone.

It is commonly known in the art that a certain protein having reduced activity due to a certain mutation will respond better to the treatment with a compound having the ability to rearrange a lysosomal protein having reduced activity than other proteins having other mutations. The effectiveness of treatment may thus allow distinguishing between responsive mutations and non-responsive mutations of the protein having reduced activity.

The present inventors observed that more responsive mutations, i.e. proteins having mutations where the enhancement of protein activity due to administration of the compound having the ability to rearrange a lysosomal protein having reduced activity was higher, also exhibited a stronger effect in further enhancing the activity of said protein having said certain mutation by applying the combination according to the present invention. Insofar, restoration of the activity of a lysosomal protein the activity of which is reduced in a disease and more specifically a lysosomal storage disease to an activity of at least 10 %, preferably at least 20 % and more preferably at least 25 % of the lysosomal protein of wild type and/or of the lysosomal protein in a healthy subject, is preferably regarded as a treatment, preferably as a treatment of the disease.

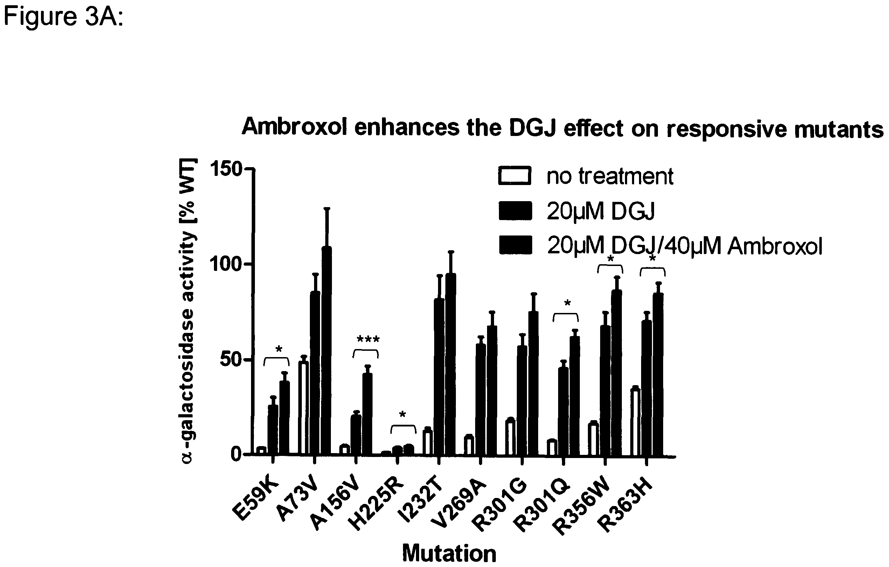

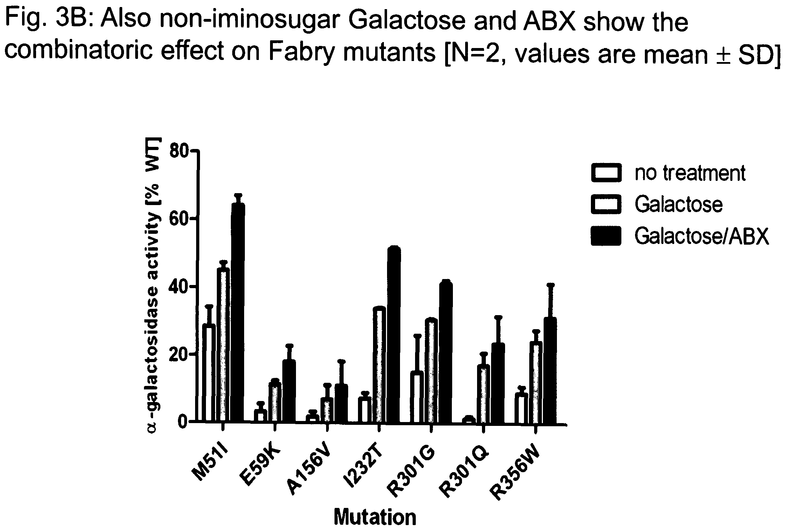

For example, and as may also be taken from Fig. 3 and Example 3 of the instant description, a stronger effect in further enhancing the activity of certain GLA mutations, which exhibit a strong responsiveness to treatment with DGJ alone (see also table 3), could be seen when applying the combination according to the present invention. More specifically, said certain GLA mutations are, for example, E59K (14.2-fold), A156V (3.9- fold) and R301Q (5.6- fold) whereas the effect in enhancing the activity of mutant proteins was less strong with regard to proteins less responsive to DGJ treatment. R356W (3.7-fold) and R363H (1.8-fold) additionally show a relatively strong residual activity, i.e. high protein activity without treatment.

In connection therewith, it is important to note that other studies, e.g. related to the treatment of Gaucher's disease with ryanodine receptors (RyRs), already considered an increase of 1.3-fold compared to the residual activity of the mutant enzyme as being clinically relevant (Wang et al., ACS Chemical Biology, 2011).

Furthermore, Wu et al. (Wu et al., supra) showed that a protein having a particular mutation and being responsive to the treatment with a pharmacological chaperone in vitro was not responsive when tested in a patient having said mutant form of the protein, whereby the patient was treated with 10µM DGJ, and whereas in all other patients tested, the responsiveness of the mutations of said patients tested in the HEK cell culture system represented the responsiveness in clinical application.

It is thus a preferred embodiment of the invention as defined in the claims that the activity of the protein having reduced activity is reduced due to a mutation of said protein, wherein the mutation is responsive to the treatment with a compound having the ability to rearrange the lysosomal protein having reduced activity when administered alone, i.e. the activity of the protein having reduced activity is enhanced due to the treatment with a compound having the ability to rearrange the lysosomal protein.

In a more preferred embodiment of the invention as defined in the claims, the protein having reduced activity is responsive to the treatment with a compound having the ability to rearrange the lysosomal protein having reduced activity in a patient, wherein the enhancement of the activity of said protein results in a protein activity of preferably 5% and most preferably 10% compared to the wild type protein activity.

As Ambroxol is widely used in connection with different diseases and symptoms including acute pain, such as toothache, postoperative pain or herpes zoster induced pain, as well as cranial infection, stroke, burns, pancreatitis or trauma, colic and headache, a person skilled in the art will acknowledge that a dosage applied in the treatment of a patient suffering from or being at risk of suffering from a lysosomal disease, such as Fabry disease will take into consideration the already approved dosages to be administered in connection with said diseases for which Ambroxol has been approved by health authorities. Accordingly, dosages of Ambroxol preferably range from 30 to 4000 mg per day as described in US patent application 11/856280 using e.g. Ambroxol tablets comprising 150-1200 mg as essentially described in US patent application 10/888362 filed July 9, 2004 by Boehringer Ingelheim International GmbH or a lozenge as described in US patent 6,663,889 filed April 23, 2002 by Boehringer Ingelheim International GmbH. A person skilled in the art will also acknowledge that up to 20g of Ambroxol per patient is applied for detoxification of patients suffering from liver failure.

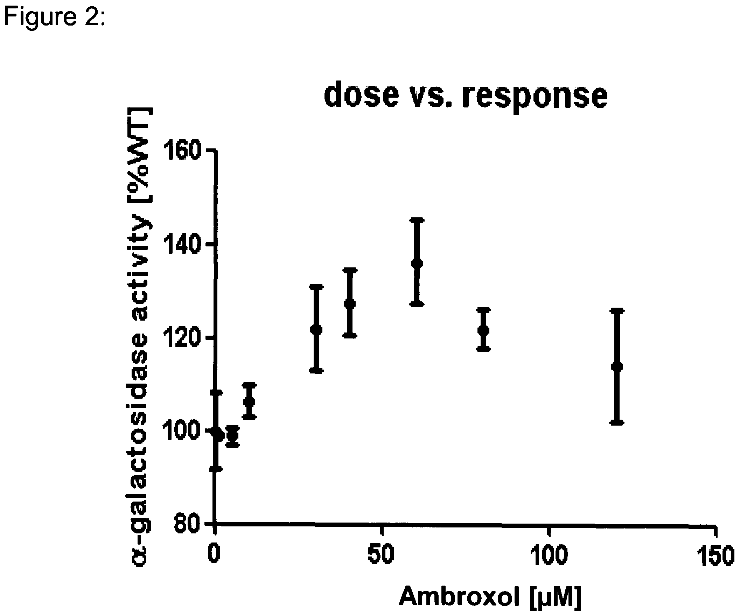

In the cell culture system preferably used in connection with the present invention an amount of Ambroxol that results in an increase of alpha-galactosidase A activity is preferably in a range of 20 to 60µM, preferably 40µM, in order to achieve a stable effect, and may also be taken from Fig. 2 and Example 3 as described herein.

A person skilled in the art will further acknowledge that a dosage to be administered to a patient in order to treat a particular LSD, whereby said patient is suffering from or is at risk of developing the same, will depend on the particular LSD to be treated as well as on the particular combination according to the present invention and more particularly on the particular compound having the ability to rearrange a lysosomal protein having reduced activity such as a particular chaperone, and on the particular derivative of Ambroxol such as bromhexine or Ambroxol contained in said composition, and on the particular condition of the patient to be treated. Accordingly, a person skilled in the art will apply a dosage of Ambroxol in the light of what has been outlined above, starting from a low dosage of Ambroxol and varying said dosage depending on the success of treatment, whereby the success of treatment may be monitored and controlled by regularly assessing the activity of the lysosomal protein having reduced activity affected in the particular LSD.

When conducting experiments in the cell culture system as outlined herein the present inventors have also found that when DGJ is administered together with bromhexine instead of Ambroxol, the activity of alpha-galactosidase A is also increased compared to the effect arising from DGJ alone.

In connection therewith, it is important to note that Leet et al. (Lee et al. Pharmacol Res. 2004 Jan;49(1):93-8) showed that a maximum concentration of Ambroxol in human plasma as high as 61.5 ng/ml could be achieved which corresponds to 0.15µM. With regard to Bromhexine Bechgaard et al. (Bechgaard et al. Biopharm Drug Dispos. 1982 Oct-Dec;3(4):337-44) disclose a concentration of bromhexine of 0.08µM. A person skilled in the art will acknowledge that when administering a 32 mg tablet (77.6µmol) an intestinal absorption of 22-27% occurs. Referring to the blood volume and after substraction of loss by first pass metabolism this should result in approximately 0.7µM, i.e. which refers to a 10-fold increase. In clinical trials 10µM DGJ have been acknowledged as to be a clinically achievable concentration (Wu et al., 2011, Hum Mutat).

A person skilled in the art will acknowledge that Ambroxol is a metabolite of bromhexine. Bromhexine, also referred to herein as 2,4-dibromo-6-{[cyclohexyl(methyl)amino]methyl} aniline is a mucolytic agent used in the treatment of respiratory disorders associated with viscid or excessive mucus. Bromhexine as referred to herein preferably has the formula:

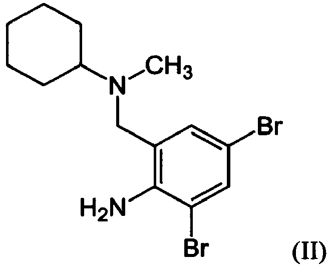

In addition, bromhexine has antioxidant properties. Bromhexine thus supports the body's own natural mechanisms for clearing mucus from the respiratory tract. It is secretolytic, i.e. it increases the production of serous mucus in the respiratory tract and makes the phlegm thinner and less sticky. This contributes to a secretomotoric effect, i.e. it helps the cilia - tiny hairs that line the respiratory tract - to transport the phlegm out of the lungs. For this reason, it is often added to some antitussive (cough) syrups.

Bromhexine is a synthetic derivative of the herbal active ingredient vasicine. It has been shown to increase the proportion of serous bronchial secretion, making it more easily expectorated. Bromhexine also enhances mucus transport by reducing mucus viscosity and by activating the ciliated epithelium. In clinical studies, Bromhexine showed secretolytic and secretomotoric effects in the bronchial tract area which facilitates expectoration and eases cough. It is indicated as "secretolytic therapy in bronchopulmonary diseases associated with abnormal mucus secretion and impaired mucus transport". Bromhexine is contained in various formulations, high and low strength syrups 8mg/5ml, 4 mg/5ml, tablets and soluble tablets (both with 8 mg bromhexine) and solution for oral use 10 mg/ 5 ml. The posology varies with the age, but there are products for all age groups from infant on. Bromhexine is a well-established and well tolerated product in its known indication. A skilled person will agree that a dosage schedule of 8-16 mg administered three times per day will serve as a good starting point for a therapy, wherein the combination of the present invention will be applied.

It is particularly noteworthy that the use of Ambroxol and/or a derivative thereof, such as bromhexine in connection with the present invention is advantageous for the clinical application of the combination of the present invention in that Ambroxol and bromhexine constitute well tolerated drugs approved for other clinical uses.

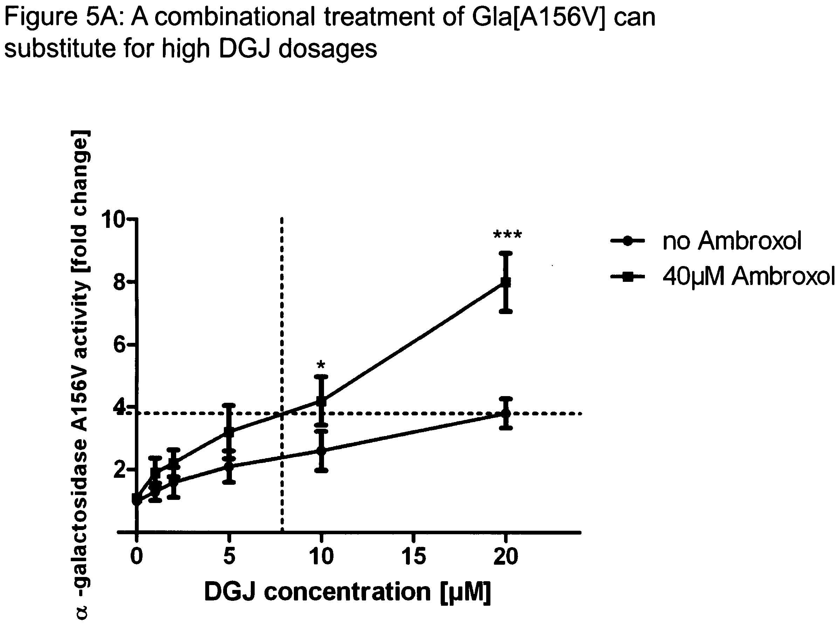

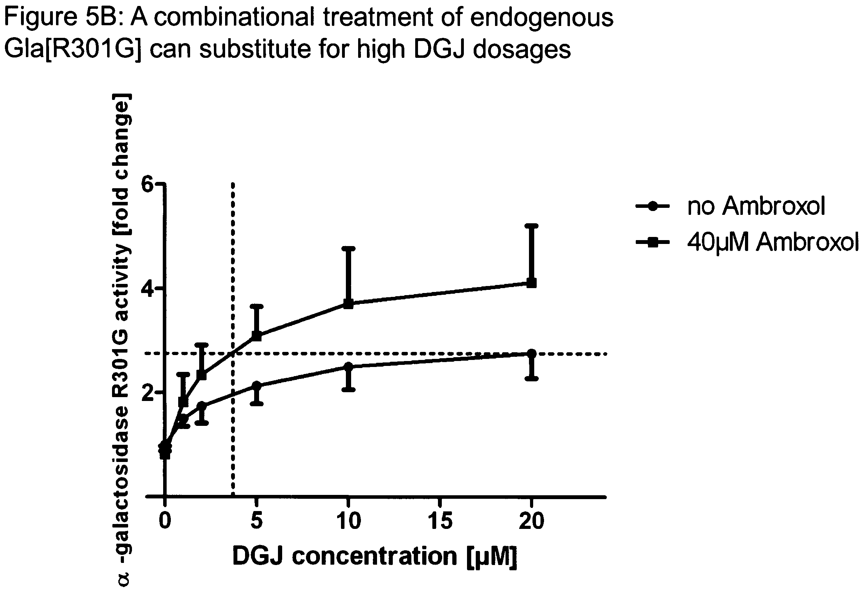

Application of high concentrations of pharmacological chaperones is known to result in an inhibitory effect on the mutant protein's activity and may also exhibit toxicity. As will also be described herein in connection with Figures 5A and 5B the combined use of a dose of a pharmacological chaperone, preferably a sub-inhibitory dose, and Ambroxol and/or a derivative such as bromhexine is of further advantage as substantially lower doses of the pharmacological chaperone may be used in order to achieve the same effect. In other words, the treatment with a combination of the present invention is able to substitute the application of a high dose of the pharmacological chaperone in order to achieve the same effect, i.e. the same increase in mutant enzyme activity.

According to the invention as defined in the claims, the combination comprises a compound having the ability to rearrange a lysosomal protein having reduced activity and Ambroxol and/or a derivative of Ambroxol. In an embodiment, said derivative of Ambroxol is bromhexine. It is thus also an embodiment of the present invention that a combination according to the invention comprises bromhexine and Ambroxol; Ambroxol; bromhexine; Ambroxol and bromhexine and optionally at least one derivative of bromhexine other than Ambroxol; Ambroxol and/or at least one derivative of bromhexine other than Ambroxol and/or bromhexine; and/or at least one derivative of bromhexine other than Ambroxol and/or bromhexine.

In an embodiment of the invention as defined in the claims, the salt of Ambroxol or bromhexine is derived from an organic or inorganic acid. In an embodiment of the combination of the present invention the salt of Ambroxol or bromhexine is derived from an organic or inorganic acid selected from the group comprising hydrohalides such HCl, HBr, H₃PO₄, as well as organic acids such as acetic acid, benzoic acid, tartaric acid and citric acid.

A derivative as used herein preferably means any pharmaceutically acceptable salt, solvate, ester or amide, or salt or solvate of such ester or amide, of a compound comprised in a combination of the present invention or any other compound which upon administration to the recipient is capable of providing, directly or indirectly, the compound or an active metabolite or residue thereof, e.g. a prodrug. Preferred pharmaceutically acceptable derivatives according to the invention are any pharmaceutically acceptable salts, solvates or prodrugs.

A person skilled in the art will immediately acknowledge that Ambroxol is a metabolite of bromhexine and, accordingly, that Ambroxol is a derivative of bromhexine, as well as vice versa, preferably bromhexine is a derivative of Ambroxol. In a preferred embodiment of the invention as defined in the claims, a derivative of bromhexine and/or Ambroxol, or the compound having the ability to rearrange a lysosomal protein having reduced activity is a pharmaceutically active and pharmaceutically acceptable derivative. In other words, the derivative is preferably less toxic, more preferably not toxic, and is enhancing the activity of the protein having reduced activity when applied in the combination according to the present invention.

A derivative of bromhexine and/or Ambroxol may be a compound of formula (III): 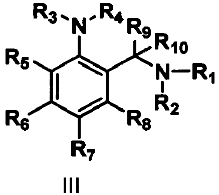 or a pharmaceutically acceptable salt thereof, wherein
each and any of R1, R2, R3, and R4 is individually and independently selected from hydrogen atom, an aliphatic carbon group with 1 to 12 carbon atoms, whereby this aliphatic carbon group can be either linear or cyclic, optionally with one or more O, N, S, P and/or halide substituents on the aliphatic carbon group, and an aromatic or heteroaromatic group, whereby this aromatic or heteroatomatic group constitutes of 2 to 18 carbon atoms, optionally with one or more additional N, O, S or halide atoms;
each and any of R5, R6, R7 and R8 is individually and independently selected from hydrogen atom, halide substituents such as I, Br, Cl, F, oxygen substituents such as OH, Oalkyl, OAryl, alkyl carboxylic acid derivatives such as CN, CO₂alkyl, CO₂aryl, CONH₂, CONHalkyl, CONHaryl, CON(alkyl)₂, CON(aryl)₂, acyl substituents such as C(O)alkyl, C(O)aryl, sulfur substituents such as SH, Salkyl, Saryl, SOalkyl, SOaryl, SO₂alkyl, SO₂aryl, SO₃alkyl, SO₃aryl, nitrogen substituents such as NH₂, NHalkyl, NHaryl, Nalkyl₂, Naryl₂, N(alkyl)aryl, NHSO₂alkyl, NHSO₂aryl, N(alkyl)SO₂alkyl, N(alkyl)SO₂aryl, N(aryl)SO₂alkyl, N(aryl)SO₂aryl, whereby alkyl stands for a linear or cyclic aliphatic carbon group from C1 to C6 with additional O, N, S, P and/or halide substituents on the aliphatic carbon group, and whereby aryl stands for an aromatic or heteroaromatic group with 2 to 18 carbon atoms, optionally with one or more additional N, O, S or halide atoms; and
each and any of R9 and R10 is individually and independently selected from hydrogen atom and an aliphatic carbon group from C1-C4, optionally with one or more additional O, N and/or halide substituents.

A derivative of bromhexine and/or Ambroxol may be a compound of formula (III) or a pharmaceutically acceptable salt thereof, wherein
each and any of R1, R2, R3 and R4 is individually and independently selected from hydrogen atom, an aliphatic carbon group with 1 to 6 carbon atoms, whereby the aliphatic carbon group can be either linear or cyclic, optionally with one or more O, N, and/or halide substituents on the aliphatic carbon group, and an aromatic or heteroaromatic group, whereby the aromatic or heteroaromatic group constitutes of 3 to 9 carbon atoms, optionally with one ore more additional N, O, or halide atoms;
each and any of R5, R6, R7 and R8 is individually and independently selected from hydrogen atom, halide substituents such as I, Br, Cl, F, oxygen substituents such as OH, Oalkyl, Oaryl, alkyl, carboxylic acid derivatives such as CN, CO₂alkyl, CONH₂, CONHalkyl, CONHaryl, acyl substituents such as C(O)aryl, nitrogen substituents such as NH₂, NHalkyl, NHaryl, NHSO₂alkyl, NHSO₂ryl, whereby alkyl stands for a linear or cyclic aliphatic carbon group from C1 to C4, optionally with one or more O, N, and/or halide substituents on the alkyl group, and whereby aryl stands for an aromatic or heteroaromatic group with 3 to 9 carbon atoms, optionally with one or more additional N, O or halide atoms; and
each and any of R9 and R10 is individually and independently selected from hydrogen atom and an aliphatic carbon group from C1-C4, optionally with one or more O, N and/or halide substituents.

In an embodiment of the invention as defined in the claims, a derivative of bromhexine and/or Ambroxol is a compound of the formula (IV) 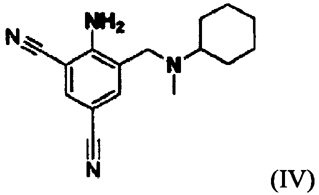 or a pharmaceutically acceptable salt thereof.

A compound of the formula (IV) is also referred to herein preferably as SF-54B.

In an embodiment of the invention as defined in the claims, a derivative of bromhexine and/or Ambroxol is a compound of the formula (V) 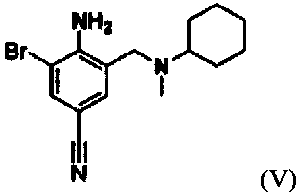 or a pharmaceutically acceptable salt thereof.

A compound of the formula (V) is also referred to herein preferably as SF-55C.

In an embodiment of the invention as defined in the claims, a derivative of bromhexine and/or Ambroxol is a compound of formula (VI) 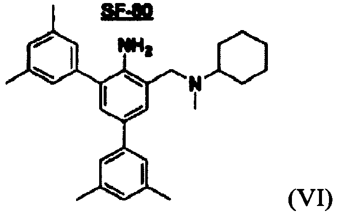 or a pharmaceutically acceptable salt thereof.

A compound of the formula (VI) is also referred to herein preferably as SF-80.

In an embodiment of the invention as defined in the claims, a derivative of bromhexine and/or Ambroxol is a compound of formula (VII) 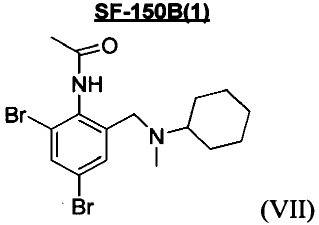 or a pharmaceutically acceptable salt thereof.

A compound of formula (VII) is also referred to herein preferably as SF-150B or SF-150B(1).

In an embodiment of the invention as defined in the claims, a derivative of bromhexine and/or Ambroxol is a compound of formula (VIII) 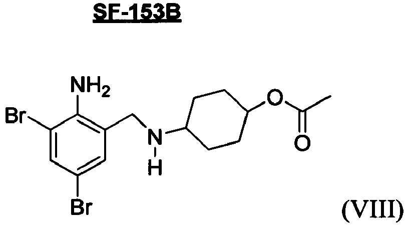 or a pharmaceutically acceptable salt thereof.

A compound of formula (VIII) is also referred to herein preferably as SF-153B.

In an embodiment of the invention as defined in the claims a derivative of bromhexine and/or Ambroxol is a compound of formula (IX) 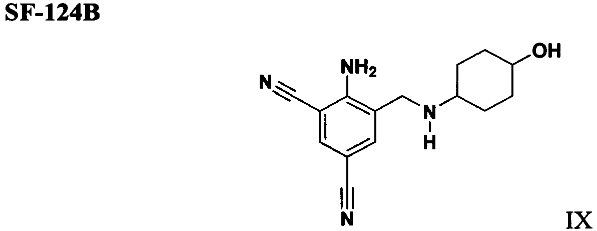 or a pharmaceutically acceptable salt thereof.

A compound of formula (IX) is also referred to herein preferably as SF-124B.

Suitable pharmaceutically acceptable salts of the compounds comprised in a combination of the present invention, more particularly, of Ambroxol, of a derivative of Ambroxol, such as bromhexine or of a compound having the ability to rearrange a lysosomal protein having reduced activity, include acid salts, for example sodium, potassium, calcium, magnesium and tetraalkylammonium and the like, or mono- or di- basic salts with the appropriate acid, for example organic carboxylic acids such as acetic, lactic, tartaric, malic, isethionic, lactobionic and succinic acids; organic sulfonic acids such as methanesulfonic, ethanesulfonic, benzenesulfonic and p-toluenesulfonic acids and inorganic acids such as hydrochloric, sulfuric, phosphoric and sulfamic acids and the like. Some of the compounds of this invention may be crystallized or recrystallized from solvents such as aqueous and organic solvents. In such cases solvates may be formed. The present invention encompasses within its scope stoichiometric solvates including hydrates as well as compounds containing variable amounts of water that may be produced by processes such as lyophilisation.

In an embodiment of the method for preparing a pharmaceutical preparation of the invention as defined in the claims, the method comprises the step of formulating as a first constituent a compound having the ability to rearrange a lysosomal protein, and as a second constituent Ambroxol and/or a derivative of Ambroxol either into a single dosage form or into two separate dosage forms. In an embodiment thereof a first of the two separate dosage forms contains the first constituent and a second of the two separate dosage forms contains the second constituent. In a further embodiment thereof the first constituent and the second constituent are formulated into one single dosage form.

In an embodiment of the invention as defined in the claims, a dosage form is selected from the group comprising tablets, capsules, powder, mixture, effervescence tablets and solutions.

It will be immediately understood by a person skilled in the art that in an embodiment of the method for preparing a pharmaceutical preparation of the invention as defined in the claims, wherein the first constituent and the second constituent are formulated into a single dosage form both the first constituent and the second constituent are comprised, for example, in a single tablet, a single capsule, a single powder, a single mixture, a single effervescence tablet or a single solution. Accordingly, in an embodiment of the method for preparing a pharmaceutical preparation, wherein a first of the two separate dosage forms contains the first constituent and a second of the two separate dosage forms contains the second constituent the first constituent is formulated into a first dosage form and the second constituent is formulated into a second dosage form, wherein preferably the first dosage form and the second dosage form each and individually are selected from the group comprising tablets, capsules, powder, mixture, effervescence tablets and solutions. Accordingly, the first constituent is, for example, formulated into a first dosage form, wherein the dosage form is a tablet and the second constituent is formulated into a second dosage form, wherein the second dosage form is a tablet, a capsule, a powder, a mixture, an effervescence tablet or a solution. It is within the present invention that the first constituent and the second constituent of the composition of the invention may be contained in the same dosage form, whereby the first constituent and the second constituent are contained in the same physical entity of such dosage form, for example in the same tablet or the same solution; in such embodiment the first and the second constituent can be admixed with each other or can be physically or chemically separated from each other within such physical entity.

A subject as referred to herein preferably means an individual, preferably a human, to which the combination according to the present invention and/or pharmaceutical preparation according to the present invention and/or compound having the ability to rearrange the lysosomal protein according to the present invention and/or Ambroxol and/or a derivative thereof according to the present invention is to be administered and/or is administered and/or has been administered independent from the health status of the individual and/or the genetic status of the individual. In an embodiment of the various aspects of the present invention a subject is a patient. Patient as used herein preferably means a subject, wherein the subject is suffering from or is at risk of suffering from a disease.

In an embodiment of the invention as defined in the claims, the combination of the invention as defined in the claims is for use in a method of personalized therapeutic treatment of a subject. In connection therewith, it has to be understood that a skilled clinician will categorize a patient's health status, such as the status of a human male, to be "ill" if, for example, a value of GLA activity is determined in a sample from said patient which is lower than a cut-off value of 5 nmol MU/mg total protein/h. It will be acknowledged by a skilled person that the decision whether or whether not the combination according to the present invention will be administered to a patient is preferably solely dependent on the genetic status of the patient, i.e. the particular mutation of the respective lysosomal protein. More particularly such method of personalized therapeutic treatment of a subject preferably comprises the following steps:
step a): determining whether in a sample of the subject the lysosomal protein has a reduced activity, preferably such reduced activity results from one or more mutation being contained in the lysosomal protein compared to the wild type lysosomal protein;
step b): identifying a compound having the ability to rearrange the lysosomal protein having reduced activity, and wherein the compound is suitable for or is increasing the reduced activity of the lysosomal protein; and
step c): administering to the subject the pharmaceutical preparation, wherein the first constituent is the compound suitable for or increasing the reduced activity of the lysosomal protein identified in step b) and wherein the second constituent is Ambroxol and/or a derivative of Ambroxol.

Accordingly, if a patient shows less than 5 nmol MU/mg/hr GLA activity said patient has to have a "responsive mutation" such as one disclosed in table 3 herein, whereas the health status of a patient having a value of GLA activity of more than 5 nmol MU/mg total protein but less than 10 nmol MU/mg total protein/h will be categorized to be "unclear".

If, according to the invention as defined in the claims, a protein, polypeptide or peptide, including but not limited to an enzyme, is said to have a mutation, this preferably means that the amino acid sequence of the protein, polypeptide or peptide is changed, more preferably changed compared to the amino acid of the wild type amino acid sequence and/or the amino acid sequence of a healthy subject.

In an embodiment of the invention as defined in the claims, the term "wherein the lysosomal protein has a reduced activity" means "wherein the lysosomal protein has reduced activity".

As preferably used herein, reference to a range defined by an upper limited and a lower limit discloses each and any integer contained within the upper limit and the lower limit including the upper limit value/figure and the lower value/figure. For example, 1 to 6 carbon atoms means in accordance therewith, 1 carbon atom, 2 carbon atoms, 3 carbon atoms, 4 carbon atoms, 5 carbon atoms and 6 carbon atoms.

The present invention is now further illustrated by the following figures and examples from which further features, embodiments and advantages may be taken.

More specifically:

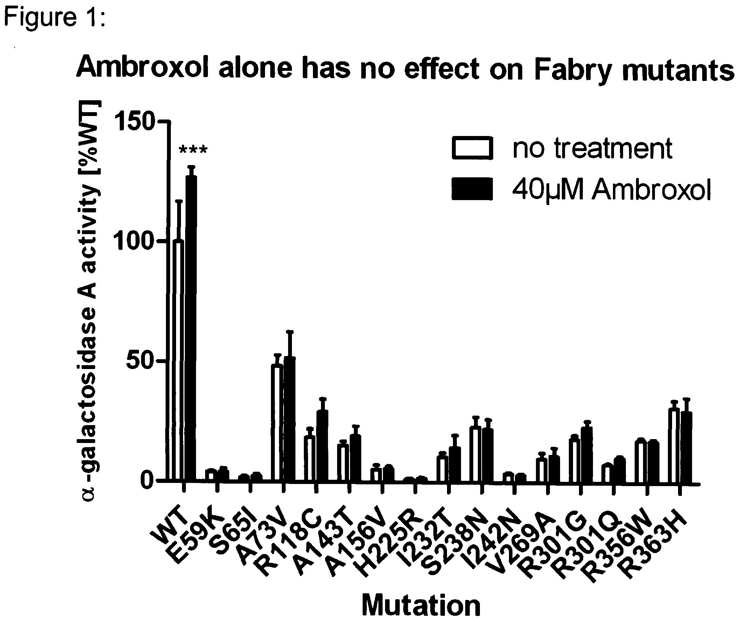

Fig. 1 is a diagram showing GLA activity of GLA mutants as present in Fabry disease and of wild type GLA with and without administration of 40 µM Ambroxol. The y-axis indicates the activity of GLA normalized to one hundred percent wild type (WT) GLA activity in the absence of treatment with Ambroxol or pharmacological chaperone. On the x-axis the mutation of the respective analyzed GLA mutants is indicated, the activity of which was determined with (grey bars) and without (white bars) the addition of 40 µM Ambroxol. The underlying experiment was carried out at least 3 times as described in Example 1; statistical analysis was carried out using two-sided student's T-test. Error bars are indicated as standard error of the mean. p-values are *= p≤0.05; **=p≤0.01; ***=p≤0.005.
Fig. 2 is a diagram showing a dose-response relationship of GLA activity as a function of Ambroxol concentration. The y-axis indicates the percentage of wild type GLA activity normalized to the activity of wild-type GLA in the absence of treatment with Ambroxol or a pharmacological chaperone. The x-axis indicates the concentration of Ambroxol which was added to the respective sample. The activity of wild type GLA was determined upon addition of the depicted concentrations of Ambroxol. The underlying experiment was carried out at least 3 times as described in Example 1. Error bars are indicated as standard error of the mean.
Fig. 3A is a diagram showing GLA activity of alpha-galactosidase (GLA) mutants upon no addition of either Ambroxol or DGJ, upon addition of 20 µM DGJ and upon addition of 20 µM DGJ and 40 µM Ambroxol (indicated from left to right for each mutant). The y-axis indicates the activity of GLA normalized to one hundred percent wild-type (WT) GLA activity in the absence or presence of treatment with a pharmacological chaperone or the combination of a pharmacological chaperone (DGJ) and Ambroxol. On the x-axis the mutation of the respective analyzed GLA mutants is indicated, the activity of which was determined upon addition of 20 µM DGJ (grey bars), addition of 20µM DGJ and 40 µM Ambroxol and without addition of either Ambroxol or DGJ (white bars). The underlying experiment was carried out at least 3 times as described in Example 1. Statistical analysis was carried out using two-sided student's T-test. Error bars are indicated as standard error of the mean. p-values are *= p≤0.05; **=p≤0.01; ***=p≤0.005.
Fig. 3B is a diagram showing GLA activity of alpha-galactosidase (GLA) mutants upon no addition of either galactose or Ambroxol, upon addition of galactose and upon addition of both galactose and Ambroxol (indicated from left to right for each mutant). The y-axis indicates the activity of GLA normalized to one hundred percent wild-type (WT) GLA activity in the absence or presence of treatment with a pharmacological chaperone or the combination of a pharmacological chaperone and Ambroxol, whereby the pharmacological chaperone is the sugar galactose. On the x-axis the mutation of the respective analyzed GLA mutants is indicated, the activity of which was determined upon addition of 100 mM galactose (light grey bars), the addition of 100 mM galactose and 40 µM Ambroxol (dark grey bars) and without the addition of either Ambroxol or galactose (white bars). The experiment has been conducted 2 times, the values are therefore given as mean ± SD.

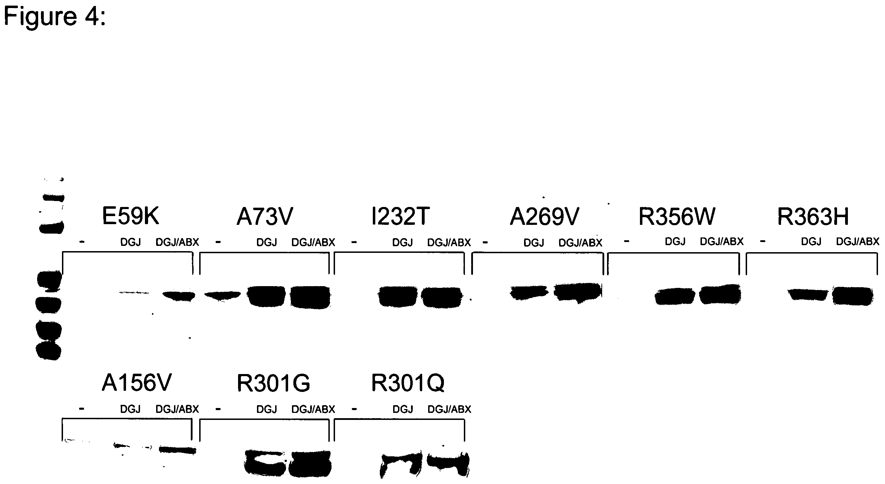

Fig. 4 is the result of a Western Blot analysis showing protein levels of GLA in cells transfected with the indicated GLA mutants, whereby the cells were not treated (indicated by "-"), treated with DGJ (indicated by "DGJ") or treated with both DGJ and Ambroxol (indicated by "DGJ/ABX"). The Experiment was carried our as described in Example 1.
Fig. 5A is a diagram showing a dose-response relationship of the activity of mutant GLA having mutation A156V, also referred to herein as GLA[A156V], as a function of DGJ concentration in the absence of Ambroxol and at 40 µM Ambroxol, respectively. The y-axis indicates the activity of GLA[A156V] as fold increase of GLA[A156V] activity without treatment with/addition of DGJ which was set as 1.0. On the x-axis the concentration of DGJ applied is indicated. The underlying experiment was carried out four times as described in Example 1, with and without application of 40µM Ambroxol, as indicated. Error bars are indicated as standard error of the mean. p-values are *= p≤0.05; **=p≤0.01; ***=p≤0.005.
Fig. 5B is a diagram showing a dose-response relationship of the activity of mutant GLA having mutation R301G, also referred to herein as GLA[R301G], as a function of DGJ concentration in the absence of Ambroxol and at 40 µM Ambroxol, respectively. The y-axis indicates the activity of GLA[R301G] as fold increase of GLA [R301G] activity withut treatment with/addition of DGJ which was set as 1.0. On the x-axis the concentration of DGJ applied is indicated. The underlying experiment was carried out four times as described in Example 1, with and without application of 40µM Ambroxol, as indicated. Error bars are indicated as standard error of the mean. p-values are *= p≤0.05; **=p≤0.01; ***=p≤0.005.

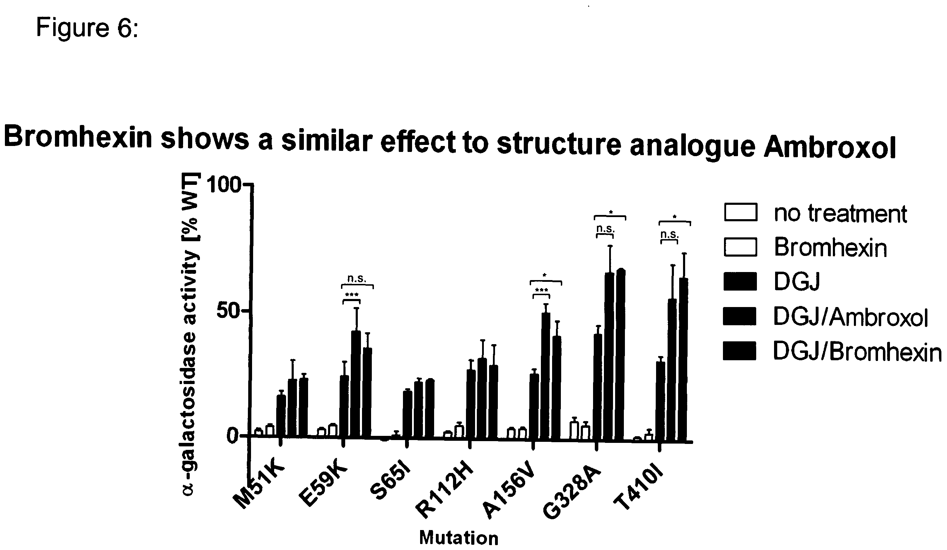

Fig. 6 is a diagram showing alpha-galactosidase (GLA) activity of various GLA mutants upon addition of (from left to right for each mutation) neither DGJ nor Ambroxol or Bromhexine, Bromhexine alone, DGJ alone, DGJ and Ambroxol, and DGJ and Bromhexine. The y-axis indicates the activity of GLA normalized to one hundred percent wild type (WT) GLA activity in the absence or presence of treatment with Bromhexine, pharmacological chaperone or the combination of pharmacological chaperone and Ambroxol or Bromhexine, respectively, with the pharmacological chaperone being DGJ. On the x-axis the mutation of the respective analyzed GLA mutants is indicated, the activity of which was determined (from left to right for each mutation) upon addition of neither Ambroxol nor DGJ (white bars), upon addition of 40µM Bromhexine (light grey bars), upon addition of 20 µM DGJ (dark grey bars), upon addition of 20µM DGJ and 40 µM Ambroxol (dark grey bars with vertical lines), and upon addition of 20µM DGJ and 40 µM Bromhexine (white bars with diagonal lines). The experiment was carried out at least 3 times as described in Example 1, statistical analysis was carried out using two-sided student's T-test. Error bars are indicated as standard error of the mean. p-values are *= p≤0.05; **=p≤0.01; ***=p≤0.005. The indicated stars show the significances of treatment with DGJ alone compared to the treatment with a combination of Bromhexine and DGJ.

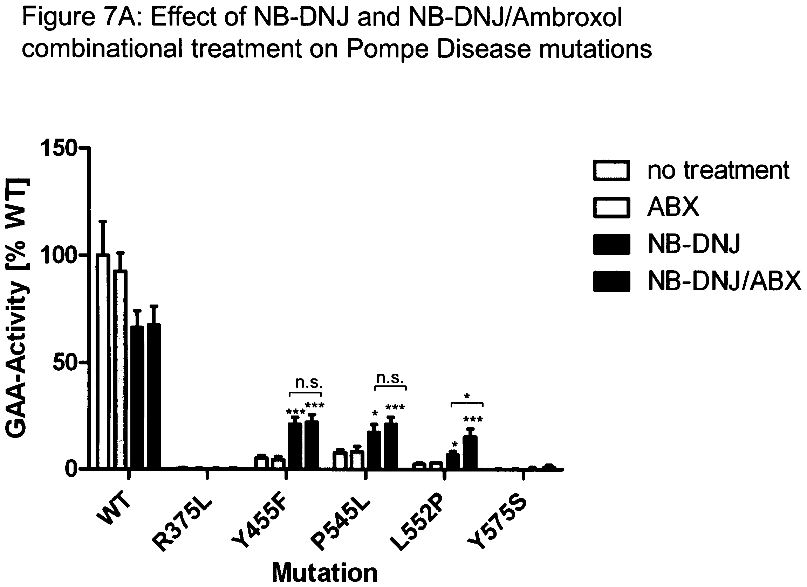

Fig. 7A is a diagram showing acid α-glucosidase (GAA) activity of variouos GAA mutants as found in Pompe disease upon addition of (from left to right for each mutation) neither Ambroxol nor NB-DNJ, Ambroxol, NB-DNJ, and NB-DNJ and Ambroxol. The y-axis indicates the activity of acid α-glucosidase normalized to one hundred percent wild type (WT) acid α-glucosidase activity in the absence or presence of treatment with Ambroxol, pharmacological chaperone or the combination of pharmacological chaperone and Ambroxol with the pharmacological chaperone being NB-DNJ. On the x-axis the mutation of the respective analyzed acid α-glucosidase mutants is indicated, the activity of which was determined (from left to right for each mutation) upon addition of neither Ambroxol nor NB-DNJ (white bars), upon addition of 40µM Ambroxol (light grey bars), upon addition of 20 µM NB-DNJ (dark grey bars), and upon addition of 20µM NB-DNJ and 40 µM Ambroxol (black bars). The underlying experiment was carried out at leat 3 times as described in Example 1. Error bars are indicated as standard error of the mean.

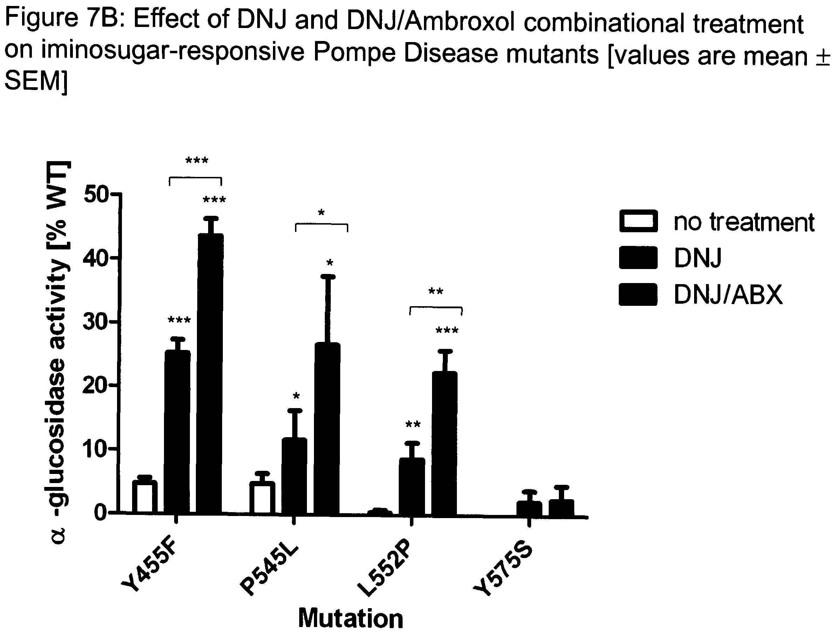

Fig. 7B is a diagram showing acid α-glucosidase activity of various acid α-glucosidase mutants upon addition of (from left to right for each mutation) neither DNJ not Ambroxol (white bars), upon addition of 20 µM DNJ (light grey bars), and upon addition of 20 µM DNJ and 40 µM Ambroxol (dark grey bars). The design is similar to Fig 7A, the iminosugar used in this experiment is DNJ instead of NB-DNJ.

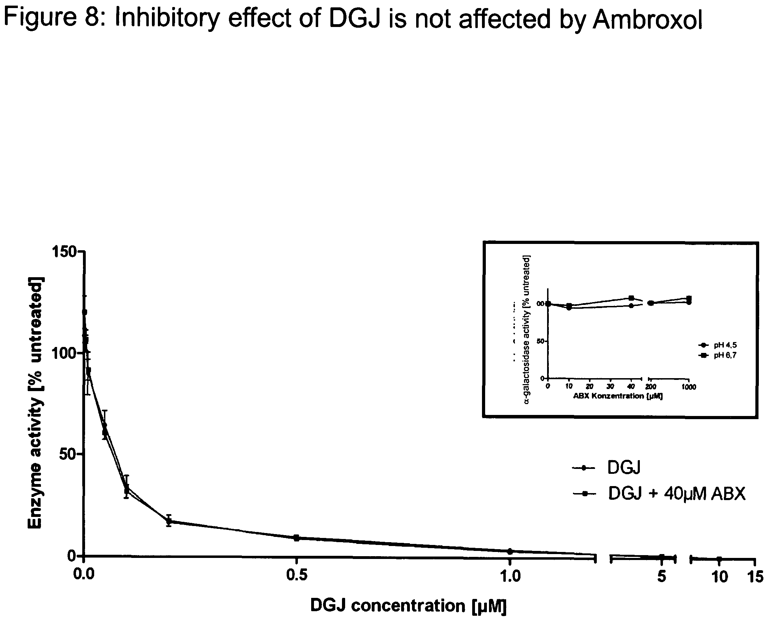

Fig. 8 is a diagram showing the activity of α-galactosidase A as a function of the concentration of DGJ and as a function of the concentration of DGJ with a constant concentration of Ambroxol of 40 µM. The y-axis indicates the activity of α-galactosidase A normalized to one hundred percent of wild-type (WT) α-galactosidase A activity in the absence of treatment with Ambroxol, pharmacological chaperone or the combination of pharmacological chaperone and Ambroxol with the pharmacological chaperone being DGJ. On the x-axis the concentration of DGJ is indicated. The Experiment was carried out at least 3 times as described in Example 1. Error bars are indicated as standard error of the mean (Microsoft, Redmont, Washington, USA). The insert in Fig. 8 shows the activity of α-galactosidase A as a function of the concentration of Ambroxol at a pH of 4.5 and at a pH of 6.7, whereby the activity is normalized activity of untreated α-galactosidase A.

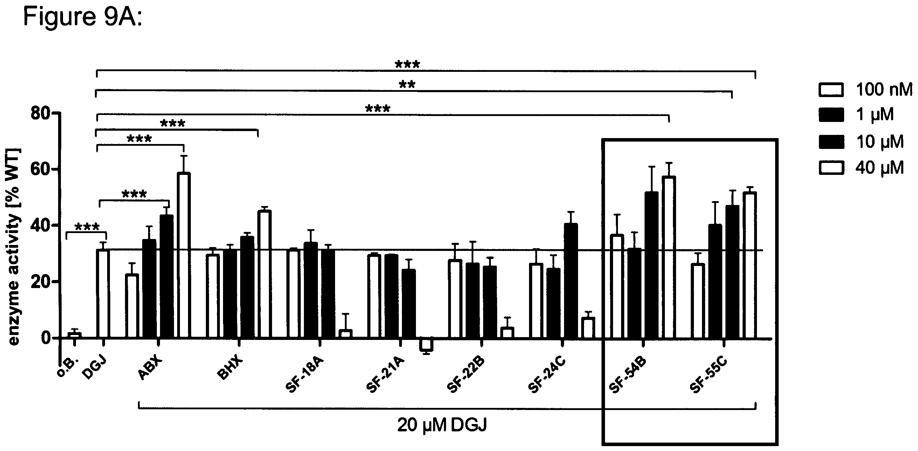

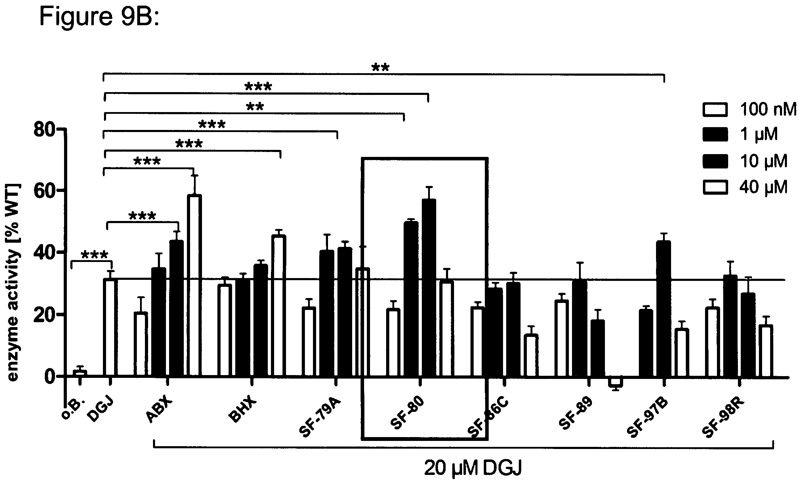

Figs. 9A and 9B are diagrams showing the activity of acid α-galactosidase A in the presence of DGJ alone, Ambroxole, Bromhexine and various compounds, whereby in case of Ambroxole, Bromhexine and the various compounds, DGJ was added such that the concentration of DGJ was 20 µM, while the concentration of Ambroxol, Bromhexine and the various compounds was (from left to right for any of Ambroxol, Bromhexine and the various compouds) 100 nM (white bars), 1 µM (grey bars), 10 µM (dark grey bars) and 40 µM (light grey bars). The y-axis indicates the activity of α-galactosidase A normalized to one hundred percent wild-type (WT) α-galactosidase A activity in the absence or presence of Ambroxol (ABX), Bromhexine (BHX) or the compounds indicated on the x-axis in combination with the pharmacological chaperone DGJ. The various compounds are derivatives of Ambroxol and Bromhexine, respectively.

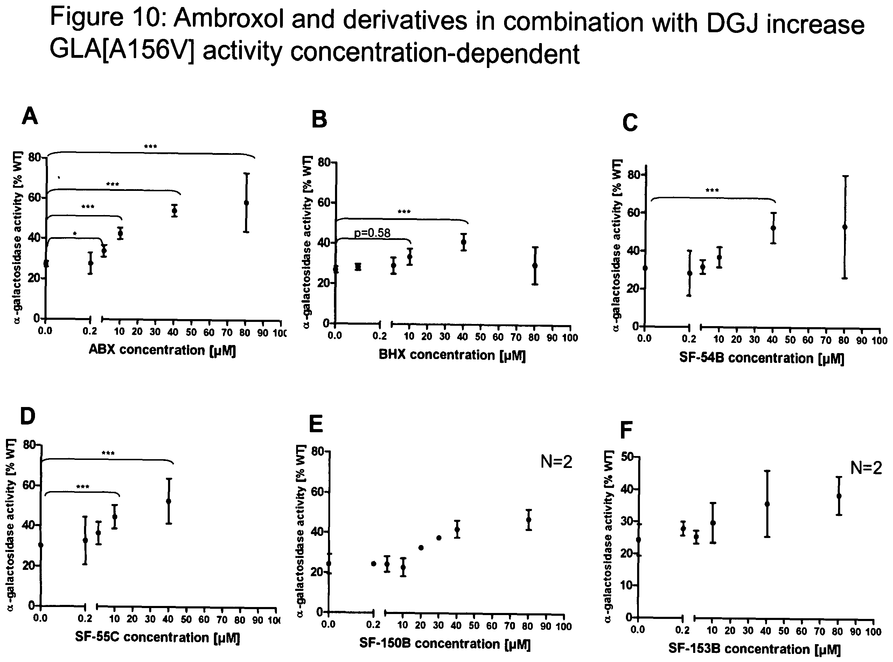

Fig. 10 is a panel of diagrams showing the activity of α-galactosidase A mutant [A156V], also referred to as GLA[A156V] as a function of the concentration of Ambroxol referred to as ABX (A), Bromhexine referred to as BHX (B), compound SF-54B (C), compound SF-55C (D), compound SF-150 B (E) and compound SF-153B (F) with DGJ being added to each reaction as pharmacological chaperone. The y-axis indicates the activity of α-galactosidase A normalized to one hundred percent of wild-type (WT) α-galactosidase A activity in the absence of treatment with the pharmacological chaperone DGJ. On the x-axis the concentration of the Ambroxol, Bromhexine and the other compounds is indicated. The experiment was carried out at least 3 times (except for E and F as indicated in the figure where n was 2) as described in Example 1. Error bars are indicated as standard error of the mean (or standard deviation when N<3). p-values are *= p≤0,05; **=p≤0,01; ***=p≤0,005. The indicated stars show the significances of treatment with DGJ alone compared to the treatment with a combination of ABX, BHX or the named compound and DGJ. BHX (B) and especially SF-55C (D) showed a toxic effect on the cells at a concentration of 80 µM.

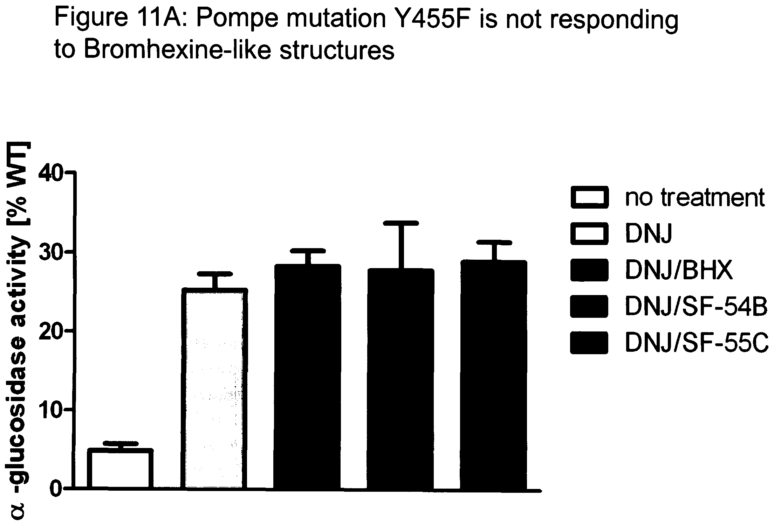

Fig. 11A is a diagram indicating acid α-glucosidase activity of acid α-glucosidase mutant Y455F as present in Pompe disease upon addition of (from left to right) neither DNJ nor any one of Bromhexine (BHX), compound SF-54C and SF-55C, upon addition of DNJ, upon addition of DNJ and Bromhexine (BHX), upon addition of DNJ and compound SF-54B, and upon addition of DNJ and compound SF-55C. The imino sugar used as a chaperone is DNJ. The design of the underlying experiment was similar to the one underlying Fig. 7A. Concentration of DNJ was 20 µM; concentration of each and any one Bromhexine, compound SF-54B and compound SF-55C was 40 µM. The underlying experiments were carried out at least 3 times (unless indicated otherwise). Error bars are indicated as standard error of the mean (or standard deviation when N<3). p-values are *= p≤0,05; **=p≤0,01; ***=p≤0,005.

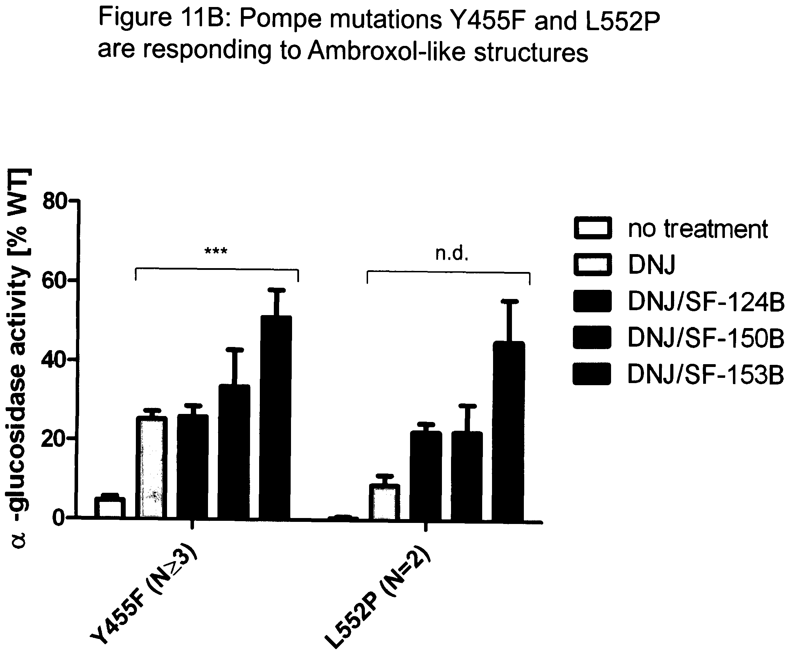

Fig. 11B is a diagram indicating acid α-glucosidase activity of acid α-glucosidase mutants Y455F and L552P as present in Pompe disease upon addition of (from left to right) neither DNJ nor any one of compound SF-124B, compound SF-150B and SF-153B, upon addition of DNJ, upon addition of DNJ and compound SF-124B, upon addition of DNJ and compound SF-150B, and upon addition of DNJ and compound SF-153B. The imino sugar used as a chaperone is DNJ. The design of the underlying experiment was similar to the one underlying Fig. 7B. Concentration of DNJ was 20 µM; concentration of each and any one Bromhexine, compound SF-54B and compound SF-55C was 40 µM. The underlying experiments were carried out at least 3 times (unless indicated otherwise). Error bars are indicated as standard error of the mean (or standard deviation when N<3). p-values are *= p≤0,05; **=p≤0,01; ***=p≤0,005.

### Example 1: Materials and Methods

### Cloning of α-galactosidase A in an overexpression vector

A bacterial clone containing the full-length cDNA of α-galactosidase A (IRAUp969H0320D, aligned to accession no. NM 000169.2 (GeneBank)) was received form ImaGenes GmbH, Berlin, Germany. Amplification was performed with the primers 5'-AGGTCGGATCCG ACAATGCAGCTGAGGAACC-3' (SEQ ID NO:1) and 5'-GGTGTTCGAATTAAAGTAAGTCTTTTAATGACATCTGCA-3' (SEQ ID NO:2) introducing unique restriction sites for BamHI and BstBI. The PCR was taken out using cloned Pfu DNA polymerase (Stratagene). The cDNA was subcloned into pGEM-T Easy vector (Promega). Prior to ligation, the cDNA insert was incubated with Taq polymerase (Qiagen) to add a poly (A) overhang at the 3'- end for TA -cloning. For expression, the α-Gal A cDNA was ligated into target vector pcDNA3.1/V5-His6 (Invitrogen).

### Cloning of acid α-glucosidase in an overexpression vector

A bacterial clone containing the full-length cDNA of α-galactosidase A (IRATp970C0971D, aligned to accession no. NM_000152.3, GeneBank) was received form ImaGenes GmbH, Berlin, Germany. Amplification was performed with the primers5'-TAG GAG CTG TCC AGG CCA TC-3' (SEQ ID NO:3) and 5'-GAG AGA CTA ACA CAC TCC GC -3' (SEQ ID NO:4). The PCR was carried out using iProof DNA Polymerase (BioRad). The cDNA was subcloned into pCRII TA TOPO vector (Invitrogen). Prior to ligation, the cDNA insert was incubated with Taq polymerase (Qiagen) to add a poly (A) overhang at the 3'- end for TA-cloning. For expression, the GAA cDNA was ligated into target vector pcDNA3.1/V5-His6 (Invitrogen) using sticky end ligation utilizing unique restriction sites (HindIII, XhoI) offered by both vectors multiple cloning sites.

### Site-directed mutagenesis of α-galactosidase A

Expression vectors containing α-Gal A and acid glucosidase mutations were generated by site-directed PCR mutagenesis (Andreotti et al., Orphanet Journal of Rare Diseases 2011, 6:66) using the QuikChange® II XL Site-Directed Mutagenesis Kit (Stratagene). Nucleotide exchanges, deletions or insertions were individually introduced by PCR amplification using PfuUltra DNA polymerase, the pcDNA3.1/GLA and pcDNA3.1/GAA plasmid vector containing the wild type sequence of the named genes were used as template and a 27-33-mer primer set, with sense and antisense primers harbouring one of the respective sequence modifications in the middle of their sequence. Each obtained mutant plasmid was subjected to sequencing analysis on a 3130 xl Genetic Analyzer (Applied Biosystems)

### Cell culture

HEK293H cells were maintained in DMEM (Dulbecco's Modified Eagle Medium, GIBCO - 31966) supplemented with 10% FBS (fetal bovine serum; PAA - A11-151) and 1% penicillin/streptomycin (GIBCO - 15140). All cells were incubated in a water-jacket incubator (Binder, Tuttlingen, GERMANY) at 37 °C under 5% CO₂. DGJ, Ambroxol and Bromhexine were received from Sigma Aldrich (Munich, GERMANY) and added to the culture medium from an aqueous stock solution (10mM). Bromhexine was obtained from Sigma Aldrich (Bromhexine Hydrochloride 17343 - ≥98.0%).

### Transient expression of mutant enzymes in HEK293H cells

1.5 x 10⁵ cells were seeded 24 hours before transfection in each well of a 24 well culture plate using 500 µl DMEM medium (GIBCO) supplemented with 10 % Fetal bovine serum (PAA). Transient expression of mutant enzymes in HEK293H cells was carried out using Lipofectamine 2000 transfection reagent (Invitrogen) according to the manufacturer's protocol. Typically, prior to transfection, a mixture of plasmid DNA (0.8 µg) and Lipofectamine 2000 transfection reagent (2 µl) in 100 µl of serum-free DMEM or Opti-MEM medium (GIBCO) was incubated at room temperature (20-25 °C) for 20 min and applied to the cells thereafter. The cells were subsequently incubated for 6 hours at 37 °C, the medium containing the transfection reagent was removed and 500 µl fresh DMEM was added. In this step, the compounds were added where intended. The cells were incubated for another 48 hours and harvested

### Enzymatic measurement of α-galactosidase A

Typically, 48 hrs after transfection with plasmid vectors containing the GLA cDNA carrying the respective mutation, cells were homogenized in 200 µl bidestilled water and subjected to 5 freeze-thaw cycles using liquid nitrogen. The supernatant collected after centrifugation of the homogenate at 10000 x g for 5 min was used in enzyme assays. Protein concentration was measured with the BCA protein assay kit (Thermo Scientific) according to the manufacturer's manual. 10 µl of the cell lysates with a concentration of 50 µg/ml were assayed with 20 µl of 4MU-α-D-galactopyranoside (4MU-α-Gal, 2 mM) in 0.06 M phosphate citrate buffer (pH 4.7) with some adaptations from the original method described by Desnick et al. (J Lab. Clin Med 81: 157, 1973). Enzyme reactions were terminated after 1 hr of incubation at 37°C by the addition of 0.2 ml of 1.0 M glycine buffer (pH 10.5), prepared by adjusting the pH using 1.0 M NaOH. The released 4MU was determined by fluorescence measurement at 360 and 465 nm as the excitation and emission wavelengths respectively in a micro plate fluorescence reader (Tecan, Männedorf, SWITZERLAND). The measured enzyme activity was first calculated as nm MU/mg protein/hr and normalized to one hundred percent wild-type activity. Experiments were conducted at least 3 times, statistical analysis was carried out using two-sided student's T-test. Error bars are indicated as standard error of the mean using the Excel Software (Microsoft, Redmont, Washington, USA). p-values are *= p≤0.05; **=p≤0.01; ***=p≤0.005

### Enzymatic measurement of α-glucosidase

Sample preparation was carried out analogously to the α-galactosidase A recordings. As substrate, a 1.3mg 4-Methylumbelliferyl-α-D-Glukopyranoside/ml solution (in sodium acetate buffer, pH 4.0) was used and 5µg sample/reaction were added. The reaction was incubated for 60 minutes at 37°C and stopped with 1M Glycine-NaOH, pH 10.5. The released 4MU was determined by fluorescence measurement at 360 and 465 nm as the excitation and emission wavelengths respectively in a micro plate fluorescence reader (Tecan, Männedorf, SWITZERLAND). The measured enzyme activity was first calculated as nm MU/mg protein/hr and normalized to one hundred percent wild-type activity. Experiments were conducted at least 3 times, statistical analysis was carried out using two-sided student's T-test. Error bars are indicated as standard error of the mean using the Excel Software (Microsoft, Redmont, Washington, USA). p-values are *= p<0.05; **=p≤0.01; ***=p≤0.005

### Western blot analysis

Western blot analysis for the detection of α-Gal A protein was performed using a commercially available rabbit anti-α-Gal A polyclonal antibody ([H-104] Santa Cruz) Furthermore, a mouse GAPDH monoclonal antibody ([6C5] abcam) was used as an internal loading control.

HEK293H cell lysates were generated by aspirating the media off the 24 well culture plates, washing the cells once with 1 x PBS (Biochrom AG) and applying 200 µl ice cold RIPA buffer supplemented with protease inhibitor cocktail tablets (Roche) to the cells prior to a 20 minute incubation on ice. The cells were then rinsed off from the wells, transferred to micro centrifuge tubes and spun at 14000 x g for 10 minutes at 4°C to pellet the cell debris. The supernatant was used for the analysis. After the determination of protein concentration, 50 µg protein were mixed with a suitable volume of 5 x Laemmli loading buffer, boiled for 5 minutes on a thermo shaker (Eppendorf), centrifuged at 14000 x g for 10 minutes at 4°C and loaded on a Criterion precast 4-15% Tris-HCl gel (Bio-Rad). Proteins were transferred electorophoretically to a Nitrocellulose (Amersham Hybond ECL) membrane (GE Healthcare). The membrane was blocked with 5% (w/v) non-fat dried skimmed milk in TBS-Tween 20 [10 mM Tris/HCl (pH 7.5) with 150 mM NaCl and 0.1% Tween 20] at room temperature for 1 hour, and then treated with a primary antibody against GAPDH diluted 1:10000 in a milk/blot solution [3% (w/v) non-fat dried skimmed milk in TBS-Tween 20] at 4°C overnight. After the blot was washed three times with excess TBS-Tween 20, the membrane was treated for 1 h at room temperature with a primary antibody against α-Gal A diluted 1:500 in the 3% milk/blot solution. After another wash procedure, a secondary antibody mix of an Alexa Fluor labeled 680 anti-rabbit IgG antibody produced in goat (Molecular Probes) and an IRDye800 conjugated anti-mouse IgG antibody produced in goat (Rockland) both diluted 1:10000 in the 3% milk/blot solution was applied to the membrane. Following extensive washing with TBS-Tween 20, protein bands were visualized with an Odyssey Infrared Imager (Li-Cor) and quantified using the Odyssey software.

### Inhibition of agalsidase alfa (Replagal®, Shire Pharmaceuticals)

Replagal was diluted to 10nM in citrate-phosphate-buffer (0.06 M) adjusted to different pH-values (4.5; 6.7). Subsequently, different concentrations of the compounds (DGJ, Ambroxol) were added ranging from 0-1mM. Finally, the substrate 4-methylumbelliferyl-α-D-galaktopyranosid (1 mM) was added to start a 15- minute incubation in a water bath at 37°C. The reaction was stopped by the addition of 200µl glycine-NaOH-buffer (1M, pH 10.5). Fluorescence measurement was carried out in a micro plate fluorescence reader (Tecan, Männedorf, SWITZERLAND) according to the method above.

### Example 2: Effect of Ambroxol on GLA enzyme activity

Various GLA mutants indicated in Fig. 1 were prepared as described in Example 1. To the enzyme preparation for the individual mutant 40 µM Ambroxol was given. The results are indicated in Fig. 1.

Fig. 1 is a diagram indicating GLA enzymatic activity for wild type GLA and indicated mutant GLA forms with and without Ambroxol. The activity was normalized with the activity observed for wild type GLA having been set as 100 %.

The experiment was conducted as described in Example 1 at least 3 times; statistical analysis was carried out using two-sided student's T-test. Error bars are indicated as standard error of the mean using the Excel Software (Microsoft, Redmont, Washington, USA). p-values are *= p≤0.05; **=p≤0.01; ***=p≤0.005

As may be taken from Fig. 1, the addition of Ambroxol resulted in an increase in enzyme activity of the GLA of the wild type. In contrast thereto, Ambroxol had no effect on the mutant forms of GLA.

The present inventors used the system established by Asano et al. (Asano et al., 2000 supra) and could reproduce the results with regard to the enhancing effect of DGJ when administered alone. More importantly, the present inventors also found that GLA is not inhibited by Ambroxol in said cell free assay.

The following table 3 lists the responsiveness of overexpressed proteins having mutations resulting in Fabry's disease with regard to therapy with DGJ alone, as well as the additional effect in terms of enhancing enzyme activity when applying Ambroxol in accordance with the present invention.

**Table 3: List of DGJ responsive GLA mutants.**

| **No.** | **mutation** | **Reference for positive testing of chaperone (DGJ) effect** | **Tested in-house** | **Non-responder (own data)** | **additive Ambroxol effect demonstrated** |
|---|---|---|---|---|---|
| 1 | A20P | Ishii et al., 2007 | | | |
| 2 | N34S | Benjamin et al., 2008 | | | |
| 3 | P40S | Benjamin et al., 2008 | | | |
| 4 | T41I | Benjamin et al., 2008, Shin et al., 2008 | | | |
| 5 | H46P | | X | | |
| 6 | R49C | Shin et al., 2008 | X | X | |
| 7 | R49L | | | | |
| 8 | M51K | Benjamin et al., 2008, Shin et al., 2008 | X | | (*) |
| 9 | M51I | | X | | ** |
| 10 | E59K | Ishii et al., 2007, Benjamin et al., 2008 | X | | **** |
| 11 | S65I | | X | | (*) |
| 12 | E66Q | Ishii et al., 2007, Benjamin et al., 2008 | | | |
| 13 | M72V | Ishii et al., 2007 | | | |
| 14 | A73V | | X | | (*) |
| 15 | I91T | Ishii et al., 2007, Benjamin et al., 2008 | | | |
| 16 | W95S | Benjamin et al., 2008 | | | |
| 17 | A97V | Ishii et al., 2007, Benjamin et al., 2008, Shin et al., 2007, 2008 | | | |
| 18 | R100K | Benjamin et al., 2008 | | | |
| 19 | R112C | Benjamin et al., 2008, Shin et al., 2007, 2008 | X | X | |
| 20 | R112H | Ishii et al., 2007, Benjamin et al., 2008, Shin et al., 2007, 2008 | X | | (*) |
| 21 | F113L | Ishii et al., 2007, Benjamin et al., 2008 | | | |
| 22 | L120V | | X | | |
| 23 | S126G | | X | | |
| 24 | D136E | | X | | |
| 25 | N139S | | X | | |
| 26 | A143T | Benjamin et al., 2008, Shin et al., 2007, 2008 | X | | |
| 27 | G144V | Benjamin et al., 2008 | | | |
| 28 | P146S | Ishii et al., 2007 | | | |
| 29 | S148N | Benjamin et al., 2008 | | | |
| 30 | A156V | Ishii et al., 2007 | X | | **** |
| 31 | D165H | | X | | |
| 32 | L166V | Ishii et al., 2007 | | | |
| 33 | D170V | Benjamin et al., 2008 | | | |
| 34 | C172Y | Benjamin et al., 2008 | X | X | |
| 35 | D175N | | X | | |
| 36 | G183D | Benjamin et al., 2008 | | | |
| 37 | G183V | | X | | |
| 38 | S201F | Shin et al. 2008 | | | |
| 39 | P205R | Benjamin et al., 2008 | | | |
| 40 | P205T | Benjamin et al., 2008, | | | |
| | | Shin et al., 2008 | | | |
| 41 | Y207C | Benjamin et al., 2008 | | | |
| 42 | Y207S | Benjamin et al., 2008, Shin et al., 2008 | | | |
| 43 | N215S | Ishii et al., 2007, Benjamin et al., 2008, Shin et al., 2008 | X | | |
| 44 | I219T | | X | | |
| 45 | R220Q | | X | | |
| 46 | N224S | | X | | |
| 47 | H225D | | X | | |
| 48 | H225R | | X | | ** |
| 49 | I232T | | X | | (*) |
| 50 | S235C | Benjamin et al., 2008 | | | |
| 51 | S238N | | X | | |
| 52 | I242N | | X | | |
| 53 | D244N | Benjamin et al., 2008 | | | |
| 54 | P259R | Shin et al., 2008 | | | |
| 55 | N263 S | Benjamin et al., 2008 | | | |
| 56 | D264Y | | X | | |
| 57 | L268S | | X | | |
| 58 | V269M | | X | | |
| 59 | V269A | | X | | (*) |
| 60 | S276G | Benjamin et al., 2008, Shin et al. 2008 | | | |
| 61 | Q279E | Ishii et al, 2007, Benjamin et al., 2008 | | | |
| 62 | T282I | | X | | |
| 63 | W287C | Benjamin et al., 2008 | | | |
| 64 | A288P | Benjamin et al., 2008 | | | |
| 65 | I289F | Benjamin et al., 2008 | | | |
| 66 | M290I | | X | | |
| 67 | L294S | | X | | |
| 68 | F295C | Shin et al., 2008 | | | |
| 69 | M296I | Benjamin et al., 2008 | | | |
| 70 | M296V | Ishii et al, 2007, Benjamin et al., 2008 | | | |
| 71 | S297C | | X | | |
| 72 | L300P | Benjamin et al., 2008, Shin et al., 2007, 2008 | | | |
| 73 | R301G | | X | | |
| 74 | R301Q | Ishii et al, 2007, Benjamin et al., 2008, Shin et al., 2008 | X | | **** |
| 75 | R301P | | X | | |
| 76 | L310F | | X | | |
| 77 | L311V | | X | | |
| 78 | D313Y | | X | | |
| 79 | V316E | Benjamin et al., 2008 | | | |
| 80 | V316I | | X | | |
| 81 | I319T | | X | | |
| 82 | N320I | | X | | |
| 83 | N320Y | Benjamin et al., 2008 | | | |
| 84 | Q3121H | | X | | |
| 85 | G325D | Benjamin et al., 2008 | | | |
| 86 | G325S | | X | | |
| 87 | Q327E | | X | | |
| 88 | G328A | Benjamin et al., 2008, Shin et al., 2008 | X | | * |
| 89 | R342Q | Benjamin et al., 2008 | X | X | |
| 90 | S345P | | X | | |
| 91 | R356W | Ishii et al, 2007, Benjamin et al., 2008, Shin et al., 2007 (non-responder) | X | | **** |
| 92 | E358A | Benjamin et al., 2008 | | | |
| 93 | E358K | Benjamin et al., 2008 | | | |
| 94 | G360C | | X | | |
| 95 | G361R | | X | | |
| 96 | R363C | Benjamin et al., 2008 | | | |
| 97 | R363H | Benjamin et al., 2008 | X | | *** |
| 98 | G373D | Ishii et al, 2007 | | | |
| 99 | G373S | Ishii et al, 2007 | | | |
| 100 | E398A | | X | | |
| 101 | P409A | Benjamin et al., 2008 | | | |
| 102 | T410I | | X | | * |
| 103 | L415F | | X | | |
| 104 | E418G | | X | | |

All mutants were tested with regard to DGJ-responsiveness as referenced in table 3. No reference indicates the mutation is exclusively tested in the hands of the present inventors. An additional effect of the administration of the combination according to the present invention, i.e. Ambroxol in combination with DGJ, was considered significant when *p≤0.1, **p≤0.05, ***p≤0.01, ****p≤0.005. For some mutations the additive effect of Ambroxol has only been repeated twice resulting only in a trend of increased enzyme activity indicated by the symbol (*). In table 3 an "X" in the column "non-responder, own data" is indicative for mutations where the results of the experiments conducted by the instant inventors is different to at least one of the indicated references with regard to the application of DGJ alone.

### Example 3: Dose-dependent efficacy of Ambroxol

In order to determine the dose-dependent efficacy of Ambroxol on the enzyme activity of wild type GLA, wild type GLA was exposed to various titers of Ambroxol.

The results are indicated in Fig. 2 which is a diagram indicating the relative activity of wild type GLA at various concentrations of Ambroxol with the activity of GLA without Ambroxol being set as 100 %.

As may be taken from Fig. 2, the enzyme activity of wild type GLA increases with increasing titers of Ambroxol. Furthermore, it is important to note that Ambroxol is not inhibiting wild type GLA. There seems to be a local maximum or potentially saturation at or beyond an Ambroxol titer of > 60 µM. The EC₅₀ was determined to be 21.2 µM.

In connection therewith, it is important to note that in contrast to mutant GLA the activity of wild type GLA may be increased upon addition of Ambroxol which may be taken from Fig. 1 and Fig. 2.

### Example 4: Synergistic effect of combined use of DGJ and Ambroxol on enzyme activity of GLA mutants

The enzyme activity of various mutant forms of GLA was determined upon exposure of the mutant forms of GLA to (a) none of DGJ and Ambroxol, (b) 20 µM DGJ, or (c) 20 µM DGJ and 40 µM Ambroxol.

The result is indicated in Fig. 3.
As may be taken from Fig. 3, all mutants of GLA which showed a beneficial effect on DGJ treatment also responded to Ambroxol when both DGJ and Ambroxol were added to the growth medium together.

A person skilled in the art will acknowledge that the assay described herein may be used to identify particularly well responding mutants. Because of this, the use of Ambroxol in combination with DGJ will be therapeutically effective/advantageous for patients responding to DGJ treatment. More precisely, for all patients DGJ treatment has been approved to enhance Gal A activity, additional treatment with Ambroxol can only be of an even higher benefit.

In connection therewith, it is important to note that for all mutant enzymes tested an increased activity was measured upon application of Ambroxol in addition to the pharmacological chaperone compared to the application of the pharmacological chaperone alone. This proves the existence of a clear tendency which underlines the advantage of the combination according to the present invention compared to the application of the pharmacological chaperone alone. A person skilled in the art will thus immediately acknowledge that an increase in mutant enzyme activity upon administration of the combination according to the present invention has not to be necessarily significantly elevated compared to the administration of the pharmacological chaperone alone as, on the one hand, in clinical application the enhancement of mutant enzyme activity to only a low degree may already result in preventing symptoms or disease as has been outlined above. On the other hand, a person skilled in the art will also acknowledge that even if the application of the combination of the present invention is not leading to significantly higher activities of mutant enzymes, the combination of the present invention may still be advantageous over applying the pharmacological chaperone in that the significant costs usually related to pharmacological chaperone therapy may be lowered if the additional application of Ambroxol according to the present invention can substitute for a certain amount of pharmacological chaperone applied, still achieving the same or similar clinical benefit for the patient treated.

A Western Blot analysis was performed as described in Example 1 and the result is indicated in Fig. 4. As may be taken therefrom, the stabilizing effect of DGJ is enhanced/improved by Ambroxol. Ambroxol contributes to the higher amount of a Gal A in the cell. While being stabilized the enzyme is no longer depleted from the cells by the endoplasmic reticulum degradation machinery. The higher amount of protein/enzyme accompanies/correlates with the higher activity measured.

It can be taken therefrom that the application of the combination according to the present invention compared to application of DGJ alone leads to a higher amount of Gal A in the respective cell. It will be acknowledged by a person skilled in the art that the mutation of Gal A such as the ones of Fig. 4 do not lead to reduced expression of GalA. Rather, the mutant Gal A is subject to a more rapid degradation, preferably due to misfolding of the protein. It is assumed by the present inventors that application of DGJ and/or Ambroxol has no effect on the level of protein expression of the lysosomal protein. More particularly, the combination according to the present invention seems to counteract, like a chaperone, preferably a pharmacological chaperone, the more rapid degradation, more preferably such counteracting occurs through stabilizing the mutant protein and thus leading to a less rapid degradation of the protein. It can be taken from Figure 4 and more particularly from the elevated amount of protein level which can be detected that Ambroxol applied in combination with DGJ results in a stabilization of the mutant protein and/or results in less rapid degradation

### Example 5: A combinational treatment of endogenous GLA mutants can substitute for high DGJ doses

The enzyme activity of mutant forms of GLA was determined upon exposure of the mutant forms of GLA to (a) different concentrations of DGJ and to 40 µM Ambroxol or (b) different concentrations of DGJ and no Ambroxol as described in Example 1.

The results are indicated in Fig. 5.

More particularly, Fig. 5A shows a diagram indicating a dose-response relationship of GLA activity of GLA mutant form A156V; and Fig.5B shows a diagram indicating a dose-response relationship of GLA activity of GLA mutant form R301G.

As may be taken from Figs. 5A and B, treatment with 40 µM Ambroxol in addition to treatment with DGJ alone enhances GLA activity.

It can immediately be seen, that an increase of 3.8-fold of GLA[A156V] activity is achieved by application of 8 µM DGJ and 40 µM Ambroxol, whereas treatment with as much as 20µM DGJ has to be applied in order to reach the same increase in mutant enzyme activity without the additional treatment of Ambroxol, which is emphasized by the horizontal and vertical dotted line.

In other words, approximately half of the concentration of DGJ, namely 8.9 µM, has to be applied, if 40 µM Ambroxol are applied in addition, to reach the same effect as if 20 µM DGJ is applied alone. The absolut measures of said assay are approximately 300 nmol MU/mg total protein/h.

That there is a synergistic effect which arises from the combined use of both DGJ and Ambroxol is evident from the fact that alpha-galactosidase A activity is increased up to 8-fold in A156V mutant (Fig. 5A) when both 20 µM DGJ and 40 µM Ambroxol are administered, whereas said activity is only increased to 4-fold of untreated GLA[A156V] activity in case of 20 µM DGJ administration only.

It can be immediately seen, that a 4.1-fold increase of GLA[R301G] activity is achieved by application of 20 µM DGJ and 40 µM Ambroxol, whereas treatment with 20 µM DGJ without additional treatment with Ambroxol results only in an increase of GLA activity of 2.8-fold which is emphasized by the horizontal and vertical dotted line.

In connection therewith, it is important to note that the absolute value of GLA activity was determined to be 3.7±0.4 nmol MU/mg total protein/h. A skilled clinician will categorize a patient's health status, such as the status of a human male, to be "ill" if a value of GLA activity is determined in a sample from said patient which is lower than a cut-off value of 5 nmol MU/mg total protein/h. It will be acknowledged by a skilled person that the decision whether or whether not the combination according to the present invention such as ABX/DGJ will be administered to a patient is preferably solely dependent on the genetic status of the patient, i.e. the particular mutation of the respective lysosomal protein. More particularly, method of personalized therapeutic treatment of a subject would comprise the following steps:
step a): determining whether in a sample of the subject the lysosomal protein has a reduced activity, preferably such reduced activity results from one or more mutation being contained in the lysosomal protein compared to the wild type lysosomal protein;
step b): identifying a compound having the ability to rearrange the lysosomal protein having reduced activity, and wherein the compound is suitable for or is increasing the reduced activity of the lysosomal protein; and
step c): administering to the subject the pharmaceutical preparation, wherein the first constituent is the compound suitable for or increasing the reduced activity of the lysosomal protein identified in step b) and wherein the second constituent is Ambroxol and/or a derivative of Ambroxol.

Accordingly, if a patient shows less than 5 nmol MU/mg/hr GLA activity said patient has to have a "responsive mutation" such as one disclosed in table 3 herein, whereas the health status of a patient having a value of GLA activity of more than 5 nmol MU/mg total protein but less than 10 nmol MU/mg total protein/h will be categorized to be "unclear".

In other words, the present data demonstrate that applying DGJ and Ambroxol in combination according to the present invention is able to raise the activity of mutant protein, as measured in the cell-culture system used herein, to a level which would prompt a skilled clinician to evaluate the patient's health status as "healthy", i.e. having an activity of mutant protein which usually does not result in symptoms otherwise caused by the mutant protein's reduced activity, whereas the treatment with DGJ alone results in protein activity of the mutant protein which would prompt the skilled clinician to evaluate the patient's health status as being "unclear".

It is important to note that in the herein described cell-culture system, a concentration of DGJ as low as 4.72µM if applied in combination with 40µM Ambroxol is sufficient to elevate the mutant protein activity to a level which is only reached if as much as 20µM DGJ is applied, if DGJ is applied alone. In other words, this means that approximately only 25% of the concentration of DGJ has to be applied if applied in combination with 40µM Ambroxol compared to the concentration of DGJ which has to be applied to reach the same increase of protein activity if DGJ is applied alone.

From this follows that the combination of the present invention is particularly useful in the treatment of those LSDs and more specifically those forms of Fabry disease involving mutant forms of GLA where the chaperone and more specifically DGJ alone is not sufficiently effective.

### Example 6: Similar effects for Ambroxol and Ambroxol derivative Bromhexine

In this example the effect on enzyme activity of mutant forms of GLA of Ambroxol and its derivative Bromhexine when combined with DGJ was compared.

The experiment was conducted as described in Example 1.

The results are shown in Fig. 6.

As is evident from Fig. 6 Ambroxol and Bromhexine perform similarly on the mutant GLAs. In accordance with the results obtained by treatment with Ambroxol alone, see Example 2 herein, no effect could be seen by a treatment using bromhexine alone; and treatment with the chaperone DGJ alone increased alpha-Galactosidase A activity. However, upon treatment with a combination of chaperone DGJ and Ambroxol or a combination of chaperone DGJ and Bromhexine, the enzyme activity of mutant forms of GLA could be significantly increased.

As may be taken from Fig. 6 the treatment with DGJ alone always lead to a highly significant increase in protein, i.e. enzyme activity compared to "no treatment". There was no significant differnece between protein activity measured after application of a combination of Ambroxol and DGJ compared to protein activity measured after application of a combination Bromhexine and DGJ. Protein activity measured after application of a combination of Ambroxol and DGJ compared to protein activity measured after application of DGJ alone was significant for the mutant proteins having mutations E59K, A156V (see also Fig. 3) and G328A.

### Example 7: Similar effects for Ambroxol and NB-DNJ in Pompe disease

In this example the effect on enzyme activity of mutant forms of acid α-glucosidase of Ambroxol and its derivative Ambroxol when combined with NB-DNJ were compared.

The experiment was conducted as described in Example 1.

The results are indicated in Fig. 7.

As is evident from Fig. 7 and analogous to what has been outlined above in connection with the combination according to the present invention and Fabry's disease (see Figs. 1 to 6) the application of the combination according to the present invention enhances the activity of the respective mutant protein in Pompe's disease compared to the application of the pharmacological chaperone alone. More specifically, the application of 20 µM NB-DNJ in combination with the application of 40µM Ambroxol enhanced the activity of acid α-glucosidase compared to the application of 20 µM NB-DNJ alone.

More particularly, the enzyme activity of mutations Y455F, P545L and L552P was significantly increased by NB-DNJ and the combined treatment according to the present invention using both NB-DNL and Ambroxol. Said mutations showed a tendency for higher activities when additionally treated with Ambroxol. However, only L552P showed significance after 4 experiments.

Most importantly, Y575S showed a clear tendency to be responsive only towards the combination of the present invention.

It is important to note that Ambroxol administered alone is not leading to an enhancement of the activity of mutant acid α-glucosidase.

### Example 8: Inhibitory effect of DGJ is not affected by Ambroxol

Wild type α-galactosidase A (Replagal®, agalsidase alfa) was incubated with different concentrations of DGJ and with either no or a constant concentration of 40µM Ambroxol. The result is shown in Figure 8.

More particularly, Fig. 8 shows that the inhibitory effect of DGJ on enzyme activity remains unchanged by Ambroxol.

It can be taken therefrom that Ambroxol has no effect on the inhibition of WT α-galactosidase A by DGJ. The IC₅₀ was determined to be unchanged in both applications (0.07µM). Inlay diagram (upper right) shows the effect of addition of Ambroxol only at two differnet pH values (pH 4.5 and pH 6.7). No reduction of the enzyme activity could be detected.

It may be taken therefrom that Ambroxol alone displays neither an inhibitory nor a stimulating effect on the wild type enzyme.

### Example 9: Effect of candidate compounds on the activity of α-galactosidase A mutant A156V

Different compounds which are also referred to herein as candidat compounds or derivatives of Ambroxol and Bromhexine, respectively, were tested in combination with DGJ as to their effect on α-galactosidase A mutant A156V activity.

The results are shown in Fig. 9A, Fig. 9B and Fig. 10.

More particularly, Figs. 9A and 9B are diagrams indicating the activity of acid α-galactosidase A mutant A156V; the y-axis indicated the activity of α-galactosidase A mutant A156V normalized to one hundred percent wild-type (WT) α-galactosidase A activity in the absence or presence of treatment with Ambroxol (ABX), bromhexine (BHX) or the various compounds as indicated on the x-axis in combination with 20 µM the pharmacological chaperone DGJ.

It can be taken therefrom that particuarly compounds SF-54B and SF-55C show a high increase in mutant enzyme activity when applied in combination with DGJ, whereby the concentration of SF-54B and SF-55C is 10µM and 40µM, respectively. Most important, application of compound SF-80 when applied at a concentration as low as 1µM in combination with DJG resulted in an increase in mutant enzyme activity as high as if Ambroxol was applied at a concentration of up to 10µM in combination with DGJ.

Fig. 10 is a panel of diagrams showing the activity of α-galactosidase A mutant A156V. The y-axis indicates the activity of α-galactosidase A normalized to one hundred percent of wild-type (WT) α-galactosidase A activity in the absence of treatment with pharmacological chaperone DGJ and increasing concentrations of Ambroxol (ABX), Bromehexine (BHX) or the compounds indicated on the x-axis in combination with the pharmacological chaperone DGJ. On the x-axis the concentration of the compound is indicated. The experiment was carried out at least 3 times as described in Example 1 (except for Figs. 10E and 10F, as indicated in the figure). Error bars are indicated as standard error of the mean (or standard deviation when N<3) using the Excel Software (Microsoft, Redmont, Washington, USA). p-values are *= p≤0,05; **=p≤0,01; ***=p≤0,005. The indicated stars show the significances of treatment with DGJ alone compared to the treatment with a combination of ABX, BHX or the named compound and DGJ. BHX (B) and especially SF-55C (D) showed a toxic effect on the cells in a concentration of 80µM.

It can be taken therefrom that compounds SF-54B, SF-55C, SF-150B and SF153B show a high increase in mutant enzyme activity when applied in combination with DGJ.

### Example 10: Effect of candidate compounds effect on the activity of α-galactosidase A mutants Y455F and L552P

Different compounds which are also referred to herein as candidate compounds or derivatives of Ambroxol and Bromhexine, respectively, were tested in combination with DNJ for the effect on the activity of α-galactosidase A mutants Y455F and L552P, respectively.
The results are shown in Fig. 11A and Fig. 11B.

Figs. 11A and 11B are diagrams indicating the activity of acid α-glucosidase. The design is similar to Fig 7A and 7B, the iminosugar used in these experiments is DNJ. The diagram in figure 11A shows the effect on mutant acid α-glucosidase activity after addition of 20µM DNJ alone and 20µM DNJ with 40µM Bromhexine (BHX) or a derivative compound thereof. The diagram in Fig. 1 11B shows the effect on mutant acid α-glucosidase after addition of 20µM DNJ alone and 20µM DNJ with 40µM of three Ambroxol (ABX)-like compounds. The experiments were carried out at least 3 times (unless otherwise indicated). Error bars are indicated as standard error of the mean (or standard deviation when N<3) using the Excel Software (Microsoft, Redmont, Washington, USA). p-values are *= p≤0,05; **=p≤0,01; ***=p≤0,005.

It can be taken therefrom that the increase in the activity of acid α-glucosidase of mutant Y455F is comparable when DNJ is applied alone or when Bromehexine, compounds SF-54B, SF-55C and SF-124B, respectively, are applied in combination with DNJ. Most importantly, the increase in the activity of mutant Y455F is higher when DNJ is applied in combination with SF -150B and SF-153B compared to the effect arising from DNJ only.

It can also be taken therefrom that the increase in the activity of mutant L552P is higher when compounds SF-124B, SF-150B and SF-153B, respectively are applied in combination with DNJ compared to the effect arising from DNJ only.
## Claims

1. A combination comprising a first constituent and a second constituent,
wherein the first constituent is a chaperon having the ability to rearrange a lysosomal protein, wherein the lysosomal protein has a reduced activity and wherein the chaperon is an imino sugar selected from the group consisting of 1-deoxygalactonojirimycin (DGJ), alpha-galacto-homonojirimycin, alpha-allo-homonojirimycin, beta-1-C-butyl-deoxygalactonojirimycin, beta-1-C-butyl-deoxynojirimycin, N-nonyl-deoxynojirimycin (NN-DNJ), N-octyl-2,5-anhydro-2,5-imino-D-glucitol, N-octyl-isofagomine, Isofagomine (IFG), calystegine A3, calystegine B1, calystegine B2, calystegine C1, 1,5-dideoxy-1,5-iminoxylitol (DIX), alpha-1-C-nonyl-DIX, alpha-1-C-octyl-1-DNJ, N-acetyl-glucosamine-thiazoline (NGT), 6-acetamido-6-deoxycastanospermine (ACAS), N-butyl-DNJ, Deoxynojirimycin (DNJ), 2-Acetamido-1,2-dideoxynojirimycin (AdDNJ), N-dodecyl-DNJ, 6-nonyl-isofagomine, N-methyl calystegine A₃, 4-epi-isofagomine, 1-deoxynojirimycin, alpha-homonojirimycin, castanospermine, 1-deoxymannojirimycin, Swainsonine, 2-hydroxy-isofagomine, 1-deoxyfuconojirimycin, beta-homofuconojirimycin, 2,5-imino-1,2,5-trideoxy-L-glucitol, 2,5-dideoxy-2,5-imino-D-fucitol, 2,5-imino-1,2,5-trideoxy-D-altritol, 1,2-dideoxy-2-N-acetamido-nojirimycin, 1,2-dideoxy-2-N-acetamido-galaconojirimycin, 2-N-acetylamino-isofagomine, 1,2-dideoxy-2-acetamido-nojirimycin, 2-N-acetamido-isofagomine, 1,2-dideoxy-2-acetamido-nojirimycin, 1-deoxyiduronojirimycin, 2-carboxy-3,4,5-trideoxypiperidine, 6-carboxy-isofagomine, 2,6-dideoxy-2,6-imino-sialic acid, and Castanospermine (CAS); and pharmaceutically acceptable salts thereof; and
wherein the second constituent is Ambroxol or a pharmaceutically acceptable salt thereof,
bromhexine or a pharmaceutically acceptable salt thereof,
a compound of the formula (IV) 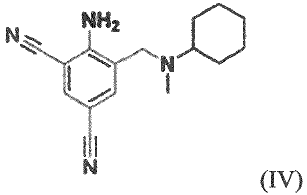 or a pharmaceutically acceptable salt thereof,
a compound of the formula (V) 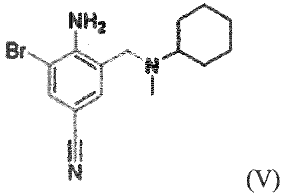 or a pharmaceutically acceptable salt thereof,
a compound of formula (VI) 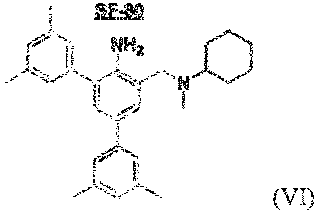 or a pharmaceutically acceptable salt thereof,
a compound of formula (VII) 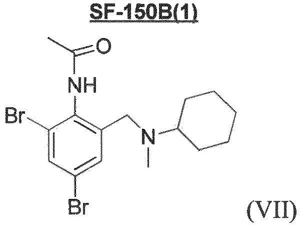 or a pharmaceutically acceptable salt thereof,
a compound of formula (VIII) 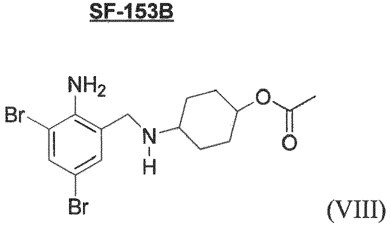 or a pharmaceutically acceptable salt thereof, and/or
a compound of formula (IX) 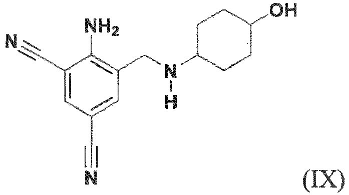 or a pharmaceutically acceptable salt thereof.

2. The combination according to claim 1, wherein the lysosomal protein is affected in a disease, and wherein the lysosomal protein is selected from the group consisting of alpha-galactosidase A and alpha-glucosidase.

3. The combination according to any one of claims 1 to 2, wherein the combination is a pharmaceutical combination.

4. The combination according to any one of claims 1 to 3, wherein the combination is suitable for or is for use in a method for the treatment of a subject comprising the administration of the combination to the subject.

5. The combination according to claim 4 or for use according to claim 4, wherein the subject is suffering from or at risk of suffering from a disease, wherein the disease is **characterized by** an enzyme having reduced activity, wherein the enzyme having reduced activity is selected from the group consisting of alpha-galactosidase A and alpha-glucosidase.

6. The combination according to claim 5 or for use according to claim 5, wherein the disease is selected from the group comprising Fabry disease, Schindler-Kanzaki disease, Pompe's disease and Parkinson disease.

7. The combination according to any one of claims 5 and 6 or for use according to any one of claims 5 and 6, wherein the enzyme is alpha-galactosidase A and the disease is selected from the group comprising Fabry disease, Schindler-Kanzaki disease and Parkinson disease.

8. The combination according to any one of claims 5 and 6 or for use according to any one of claims 5 and 6, wherein the enzyme is alpha-glucosidase and the disease is Pompe's disease.

9. Use of a combination as defined in any one of claims 1 to 8, for the manufacture of a medicament for the treatment or prevention of a disease.

10. A pharmaceutical preparation comprising a first constituent, a second constituent optionally a further constituent,
wherein the first constituent is a chaperon having the ability to rearrange a lysosomal protein, wherein the lysosomal protein has a reduced activity and wherein the chaperon is selected from the group consisting of 1-deoxygalactonojirimycin (DGJ), alpha-galacto-homonojirimycin, alpha-allo-homonojirimycin, beta-1-C-butyl-deoxygalactonojirimycin, beta-1-C-butyl-deoxynojirimycin, N-nonyl-deoxynojirimycin (NN-DNJ), N-octyl-2,5-anhydro-2,5-imino-D-glucitol, N-octyl-isofagomine, Isofagomine (IFG), calystegine A3, calystegine B1, calystegine B2, calystegine C1, 1,5-dideoxy-1,5-iminoxylitol (DIX), alpha-1-C-nonyl-DIX, alpha-1-C-octyl-1-DNJ, N-acetyl-glucosamine-thiazoline (NGT), 6-acetamido-6-deoxycastanospermine (ACAS), N-butyl-DNJ, Deoxynojirimycin (DNJ), 2-Acetamido-1,2-dideoxynojirimycin (AdDNJ), N-dodecyl-DNJ, 6-nonyl-isofagomine, N-methyl calystegine A₃, 4-epi-isofagomine, 1-deoxynojirimycin, alpha-homonojirimycin, castanospermine, 1-deoxymannojirimycin, Swainsonine, 2-hydroxy-isofagomine, 1-deoxyfuconojirimycin, beta-homofuconojirimycin, 2,5-imino-1,2,5-trideoxy-L-glucitol, 2,5-dideoxy-2,5-imino-D-fucitol, 2,5-imino-1,2,5-trideoxy-D-altritol, 1,2-dideoxy-2-N-acetamido-nojirimycin, 1,2-dideoxy-2-N-acetamido-galaconojirimycin, 2-N-acetylamino-isofagomine, 1,2-dideoxy-2-acetamido-nojirimycin, 2-N-acetamido-isofagomine, 1,2-dideoxy-2-acetamido-nojirimycin, 1-deoxyiduronojirimycin, 2-carboxy-3,4,5-trideoxypiperidine, 6-carboxy-isofagomine, 2,6-dideoxy-2,6-imino-sialic acid, and Castanospermine (CAS); and pharmaceutically acceptable salts thereof,
wherein the second constituent is Ambroxol or a pharmaceutically acceptable salt thereof, bromhexine or a pharmaceutically acceptable salt thereof, a compound of the formula (IV) 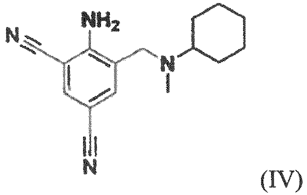 or a pharmaceutically acceptable salt thereof, a compound of the formula (V) 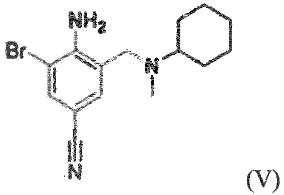 or a pharmaceutically acceptable salt thereof,
a compound of formula (VI) 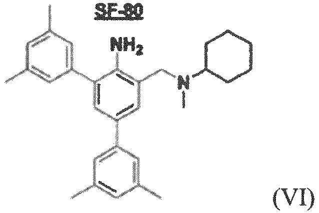 or a pharmaceutically acceptable salt thereof,
a compound of formula (VII) 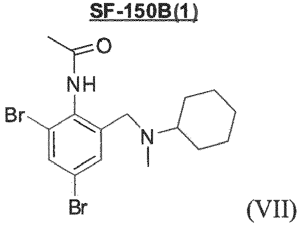 or a pharmaceutically acceptable salt thereof,
a compound of formula (VIII) 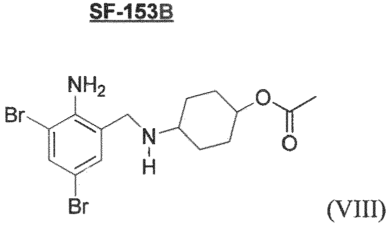 or a pharmaceutically acceptable salt thereof, and/or
a compound of formula (IX) 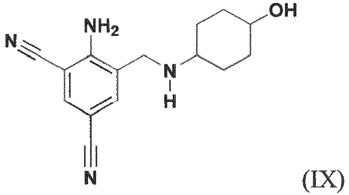 or a pharmaceutically acceptable salt thereof, and
wherein the further constituent is selected from the group comprising a pharmaceutically acceptable excipient and a pharmaceutically active agent, and
wherein preferably the pharmaceutical preparation increases the reduced activity of the lysosomal protein, and wherein the lysosomal protein has a reduced activity.

11. A method for preparing a pharmaceutical preparation according to claim 10, comprising the steps of formulating the first constituent and the second constituent into a single dosage form or into two separate dosage forms, wherein in case of two separate dosage forms a first of the two separate dosage forms contains the first constituent and a second of the two separate dosage forms contains the second constituent.

12. A chaperon having the ability to rearrange a lysosomal protein, wherein the lysosomal protein has a reduced activity and wherein the chaperone is selected from the group consisting of 1-deoxygalactonojirimycin (DGJ), alpha-galacto-homonojirimycin, alpha-allo-homonojirimycin, beta-1-C-butyl-deoxygalactonojirimycin, beta-1-C-butyl-deoxynojirimycin, N-nonyl-deoxynojirimycin (NN-DNJ), N-octyl-2,5-anhydro-2,5-imino-D-glucitol, N-octyl-isofagomine, Isofagomine (IFG), calystegine A3, calystegine B1, calystegine B2, calystegine C1, 1,5-dideoxy-1,5-iminoxylitol (DIX), alpha-1-C-nonyl-DIX, alpha-1-C-octyl-1-DNJ, N-acetyl-glucosamine-thiazoline (NGT), 6-acetamido-6-deoxycastanospermine (ACAS), N-butyl-DNJ, Deoxynojirimycin (DNJ), 2-Acetamido-1,2-dideoxynojirimycin (AdDNJ), N-dodecyl-DNJ, 6-nonyl-isofagomine, N-methyl calystegine A₃, 4-epi-isofagomine, 1-deoxynojirimycin, alpha-homonojirimycin, castanospermine, 1-deoxymannojirimycin, Swainsonine, 2-hydroxy-isofagomine, 1-deoxyfuconojirimycin, beta-homofuconojirimycin, 2,5-imino-1,2,5-trideoxy-L-glucitol, 2,5-dideoxy-2,5-imino-D-fucitol, 2,5-imino-1,2,5-trideoxy-D-altritol, 1,2-dideoxy-2-N-acetamido-nojirimycin, 1,2-dideoxy-2-N-acetamido-galaconojirimycin, 2-N-acetylamino-isofagomine, 1,2-dideoxy-2-acetamido-nojirimycin, 2-N-acetamido-isofagomine, 1,2-dideoxy-2-acetamido-nojirimycin, 1-deoxyiduronojirimycin, 2-carboxy-3,4,5-trideoxypiperidine, 6-carboxy-isofagomine, 2,6-dideoxy-2,6-imino-sialic acid, and Castanospermine (CAS); and pharmaceutically acceptable salts thereof, for use in a method for the treatment of a disease,
wherein the method comprises administering to a subject the chaperon and, prior to, concomitantly with or after Ambroxol or a pharmaceutically acceptable salt thereof, bromhexine or a pharmaceutically acceptable salt thereof, a compound of the formula (IV) 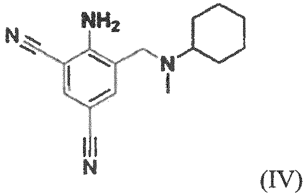 or a pharmaceutically acceptable salt thereof,
a compound of the formula (V) 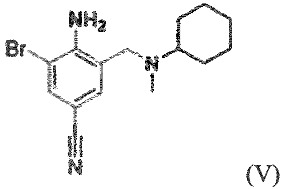 or a pharmaceutically acceptable salt thereof,
a compound of formula (VI) 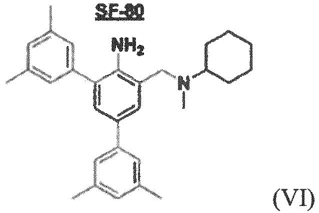 or a pharmaceutically acceptable salt thereof,
a compound of formula (VII) 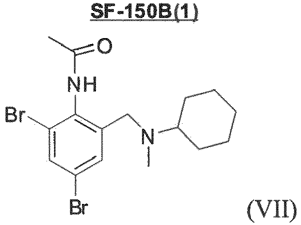 or a pharmaceutically acceptable salt thereof,
a compound of formula (VIII) 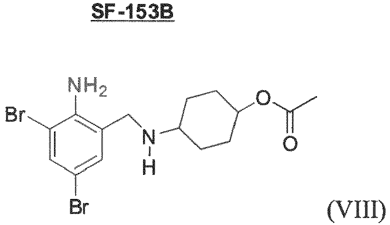 or a pharmaceutically acceptable salt thereof, and/or
a compound of formula (IX) 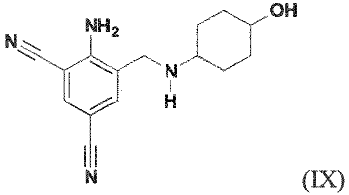 or a pharmaceutically acceptable salt thereof.

13. Ambroxol or a pharmaceutically acceptable salt thereof, bromhexine or a pharmaceutically acceptable salt thereof, a compound of the formula (IV) 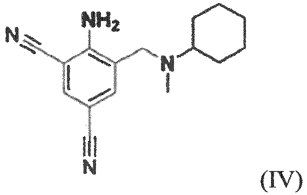 or a pharmaceutically acceptable salt thereof,
a compound of the formula (V) 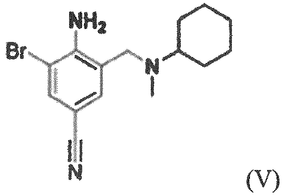 or a pharmaceutically acceptable salt thereof,
a compound of formula (VI) 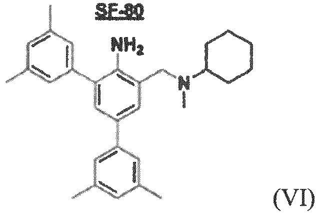 or a pharmaceutically acceptable salt thereof,
a compound of formula (VII) 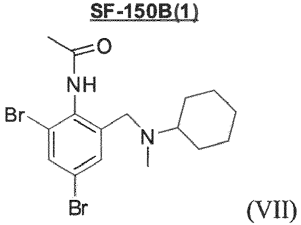 or a pharmaceutically acceptable salt thereof,
a compound of formula (VIII) 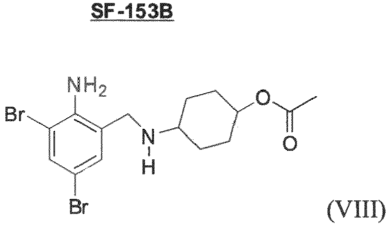 or a pharmaceutically acceptable salt thereof, and/or
a compound of formula (IX) 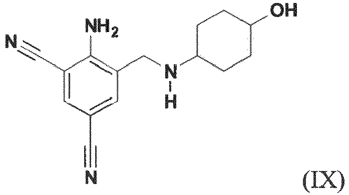 or a pharmaceutically acceptable salt thereof for use in a method for the treatment of a disease,
wherein the method comprises administering to a subject Ambroxol or a pharmaceutically acceptable salt thereof, bromhexine or a pharmaceutically acceptable salt thereof, a compound of the formula (IV) 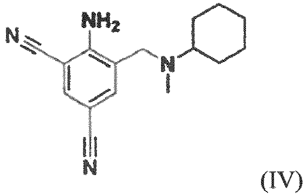 or a pharmaceutically acceptable salt thereof,
a compound of the formula (V) 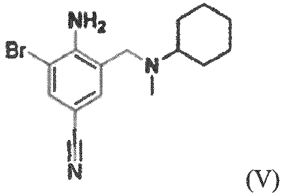 or a pharmaceutically acceptable salt thereof,
a compound of formula (VI) 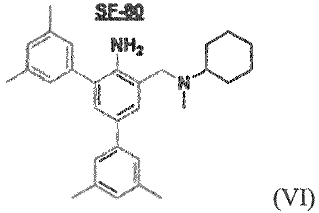 or a pharmaceutically acceptable salt thereof,
a compound of formula (VII) 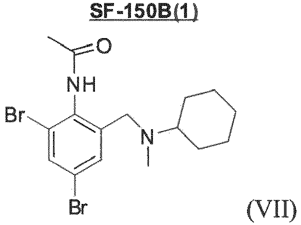 or a pharmaceutically acceptable salt thereof,
a compound of formula (VIII) 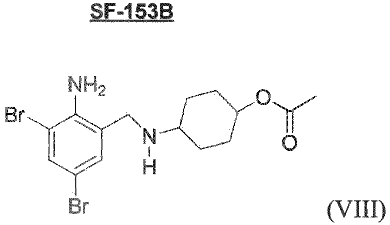 or a pharmaceutically acceptable salt thereof, and/or
a compound of formula (IX) 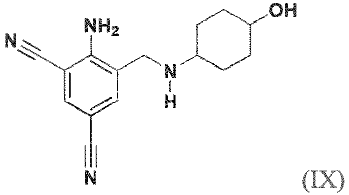 or a pharmaceutically acceptable salt thereof and, prior to, concomitantly with or after a chaperon having the ability to rearrange a lysosomal protein, wherein the lysosomal protein has a reduced activity and wherein the chaperon is selected from the group consisting of 1-deoxygalactonojirimycin (DGJ), alpha-galacto-homonojirimycin, alpha-allo-homonojirimycin, beta-1-C-butyl-deoxygalactonojirimycin, beta-1-C-butyl-deoxynojirimycin, N-nonyl-deoxynojirimycin (NN-DNJ), N-octyl-2,5-anhydro-2,5-imino-D-glucitol, N-octyl-isofagomine, Isofagomine (IFG), calystegine A3, calystegine B1, calystegine B2, calystegine C1, 1,5-dideoxy-1,5-iminoxylitol (DIX), alpha-1-C-nonyl-DIX, alpha-1-C-octyl-1-DNJ, N-acetyl-glucosamine-thiazoline (NGT), 6-acetamido-6-deoxycastanospermine (ACAS), N-butyl-DNJ, Deoxynojirimycin (DNJ), 2-Acetamido-1,2-dideoxynojirimycin (AdDNJ), N-dodecyl-DNJ, 6-nonyl-isofagomine, N-methyl calystegine A₃, 4-epi-isofagomine, 1-deoxynojirimycin, alpha-homonojirimycin, castanospermine, 1-deoxymannojirimycin, Swainsonine, 2-hydroxy-isofagomine, 1-deoxyfuconojirimycin, beta-homofuconojirimycin, 2,5-imino-1,2,5-trideoxy-L-glucitol, 2,5-dideoxy-2,5-imino-D-fucitol, 2,5-imino-1,2,5-trideoxy-D-altritol, 1,2-dideoxy-2-N-acetamido-nojirimycin, 1,2-dideoxy-2-N-acetamido-galaconojirimycin, 2-N-acetylamino-isofagomine, 1,2-dideoxy-2-acetamido-nojirimycin, 2-N-acetamido-isofagomine, 1,2-dideoxy-2-acetamido-nojirimycin, 1-deoxyiduronojirimycin, 2-carboxy-3,4,5-trideoxypiperidine, 6-carboxy-isofagomine, 2,6-dideoxy-2,6-imino-sialic acid, and Castanospermine (CAS); and pharmaceutically acceptable salts thereof.

14. A combination according to any one of claims 1 to 8, for use in a method of personalized therapeutic treatment of a subject, wherein the method comprises the following steps:
step a): determining whether in a sample of the subject the lysosomal protein has a reduced activity, preferably such reduced activity results from one or more mutation of the lysosomal protein compared to the wild type lysosomal protein;
step b): identifying a compound having the ability to rearrange the lysosomal protein having reduced activity, and wherein the compound is suitable for or is increasing the reduced activity of the lysosomal protein; and
step c): administering to the subject the first constituent prior to, concomitantly with or after the second constituent.

## Patentansprüche

1. Eine Kombination umfassend einen ersten und einen zweiten Bestandteil,
wobei der erste Bestandteil ein Chaperon mit der Fähigkeit ist, ein lysosomales Protein umzulagern, wobei das lysosomale Protein eine verringerte Aktivität aufweist und das Chaperon ein Iminozucker ist, der ausgewählt ist auf der Gruppe bestehend aus 1-Desoxygalactonojirimycin (DGJ), alpha-Galacto-Homonojirimycin, alpha-allo-Homonojirimycin, beta-1-C-Butyl-Desoxygalactonojirimycin, beta-1-C-Butyl-Desoxynojirimycin, N-Nonyl-Desoxynojirimycin (NN-DNJ), N-Octyl-2,5-anhydro-2,5-imino-D-Glucitol, N-Octyl-Isofagomin, Isofagomin (IFG), Calystegin A3, Calystegin B1, Calystegin B2, Calystegin C1, 1,5-Didesoxy-1,5-iminoxylitol (DIX), alpha-1-C-Nonyl-DIX, alpha-1-C-Octyl-1-DNJ, N-Acetyl-Glucosamin-Thiazolin (NGT), 6-Acetamido-6-desoxycastanospermin (ACAS), N-Butyl-DNJ, Desoxynojirimycin (DNJ), 2-Acetamido-1,2-didesoxynojirimycin (AdDNJ), N-Dodecyl-DNJ, 6-Nonyl-Isofagomin, N-Methyl-Calystegin A₃, 4-epi-Isofagomin, 1-Desoxynojirimycin, alpha-Homonojirimycin, Castanospermin, 1-Desoxymannojirimycin, Swainsonin, 2-Hydroxy-Isofagomin, 1-Desoxyfuconojirimycin, beta-Homofuconojirimycin, 2,5-Imino-1,2,5-tridesoxy-L-Glucitol, 2,5-Didesoxy-2,5-imino-D-Fucitol, 2,5-Imino-1,2,5-Tridesoxy-D-Altritol, 1,2-Didesoxy-2-N-acetamido-Nojirimycin, 1,2-Didesoxy-2-N-acetamido-Galaconojirimycin, 2-N-Acetylamino-Isofagomin, 1,2-Didesoxy-2-acetamido-Nojirimycin, 2-N-Acetamido-Isofagomin, 1,2-Didesoxy-2-acetamido-Nojirimycin, 1-Desoxyiduronojirimycin, 2-Carboxy-3,4,5-tridesoxypiperidin, 6-Carboxy-Isofagomin, 2,6-Didesoxy-2,6-imino-Sialsäure, und Castanospermin (CAS); und pharmazeutisch akzeptablen Salzen davon; und
wobei der zweite Bestandteile Ambroxol oder ein pharmazeutisch akzeptables Salz davon,
Bromhexin oder ein pharmazeutisch akzeptables Salz davon,
eine Verbindung gemäß Formel (IV) 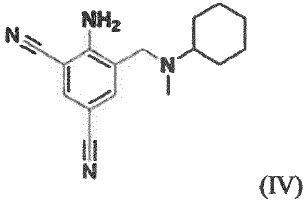 oder ein pharmazeutisch akzeptables Salz davon,
eine Verbindung gemäß Formel (V) 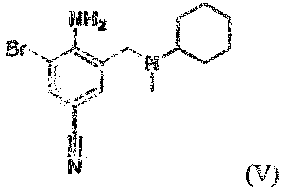 oder ein pharmazeutisch akzeptables Salz davon,
eine Verbindung gemäß Formel (VI) 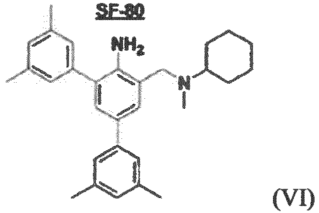 oder ein pharmazeutisch akzeptables Salz davon,
eine Verbindung gemäß Formel (VII) 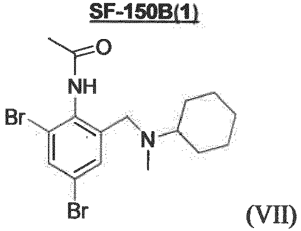 oder ein pharmazeutisch akzeptables Salz davon,
eine Verbindung gemäß Formel (VIII) 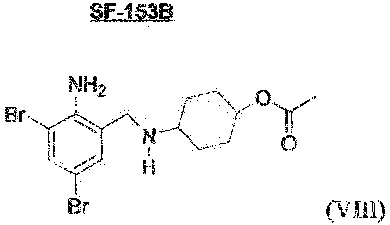 oder ein pharmazeutisch akzeptables Salz davon, und/oder
eine Verbindung gemäß Formel (IX) 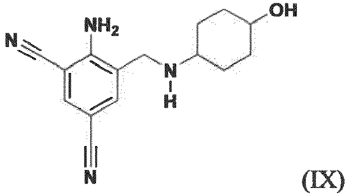 oder ein pharmazeutisch akzeptables Salz davon ist

2. Kombination nach Anspruch 1, wobei das lysosomale Protein bei einer Krankheit betroffen ist und wobei das lysosomale Protein ausgewählt ist aus der Gruppe bestehend aus alpha-Galactosidase A und alpha-Glucosidase.

3. Kombination nach einem der Ansprüche 1 bis 2, wobei die Kombination eine pharmazeutische Kombination ist.

4. Kombination nach einem der Ansprüche 1 bis 3, wobei die Kombination geeignet ist für ein Verfahren für die Behandlung eines Lebewesens oder die Kombination für die Verwendung in einem Verfahren für die Behandlung eines Lebewesens ist, wobei das Verfahren die Verabreichung der Kombination an das Lebewesen umfasst.

5. Kombination nach Anspruch 4 oder zur Verwendung nach Anspruch 4, wobei das Lebewesen an einer Krankheit leidet oder für das Lebewesen das Risiko besteht, an einer Krankheit zu leiden, wobei die Krankheit **gekennzeichnet ist durch** ein Enzym mit verringerter Aktivität, wobei das Enzym mit verringerter Aktivität ausgewählt ist aus der Gruppe bestehend aus alpha-Galactosidase A und alpha-Glucosidase.

6. Kombination nach Anspruch 5 oder zur Verwendung nach Anspruch 5, wobei die Krankheit ausgewählt ist aus der Gruppe umfassend Fabry'sche Krankheit, Schindler-Kanzaki-Krankheit, Pompe'sche Krankheit und Parkinson'sche Krankheit.

7. Kombination nach einem der Ansprüche 5 und 6 oder zur Verwendung nach einem der Ansprüche 5 und 6, wobei das Enzym alpha-Galactosidase A ist und die Krankheit ausgewählt ist aus der Gruppe umfassend Fabry'sche Krankheit, Schindler-Kanzaki Krankheit und Parkinson'sche Krankheit.

8. Kombination nach einem der Ansprüche 5 und 6 oder zur Verwendung nach einem der Ansprüche 5 und 6, wobei das Enzym alpha-Glucosidase und die Krankheit Pompe'sche Krankheit ist.

9. Verwendung einer Kombination wie in einem der Ansprüche 1 bis 8 definiert, für die Herstellung eines Medikamentes für die Behandlung oder Prävention einer Krankheit.

10. Pharmazeutische Zubereitung umfassend einen ersten Bestandteil, einen zweiten Bestandteil und optional einen weiteren Bestandteil,
wobei der erste Bestandteil ein Chaperon mit der Fähigkeit ist, ein lysosomales Protein umzulagern, wobei das lysosomale Protein eine verringerte Aktivität aufweist und das Chaperon ausgewählt ist aus der Gruppe bestehend aus 1-Desoxygalactonojirimycin (DGJ), alpha-Galacto-Homonojirimycin, alpha-allo-Homonojirimycin, beta-l-C-Butyl-Desoxygalactonojirimycin, beta-1-C-Butyl-Desoxynojirimycin, N-Nonyl-Desoxynojirimycin (NN-DNJ), N-Octyl-2,5-anhydro-2,5-imino-D-Glucitol, N-Octyl-Isofagomin, Isofagomin (IFG), Calystegin A3, Calystegin B1, Calystegin B2, Calystegin C1, 1,5-Didesoxy-1,5-iminoxylitol (DIX), alpha-1-C-Nonyl-DIX, alpha-1-C-Octyl-1-DNJ, N-Acetyl-Glucosamin-Thiazolin (NGT), 6-Acetamido-6-desoxycastanospermin (ACAS), N-Butyl-DNJ, Desoxynojirimycin (DNJ), 2-Acetamido-1,2-didesoxynojirimycin (AdDNJ), N-Dodecyl-DNJ, 6-Nonyl-Isofagomin, N-Methyl-Calystegin A₃, 4-epi-Isofagomin, 1-Desoxynojirimycin, alpha-Homonojirimycin, Castanospermin, 1-Desoxymannojirimycin, Swainsonin, 2-Hydroxy-Isofagomin, 1-Desoxyfuconojirimycin, beta-Homofuconojirimycin, 2,5-Imino-1,2,5-tridesoxy-L-Glucitol, 2,5-Didesoxy-2,5-imino-D-Fucitol, 2,5-Imino-1,2,5-tridesoxy-D-Altritol, 1,2-Didesoxy-2-N-acetamido-Nojirimycin, 1,2-Didesoxy-2-N-acetamido-Galaconojirimycin, 2-N-Acetylamino-Isofagomin, 1,2-Didesoxy-2-acetamido-Nojirimycin, 2-N-Acetamido-Isofagomin, 1,2-Didesoxy-2-acetamido-Nojirimycin, 1-Desoxyiduronojirimycin, 2-Carboxy-3,4,5-tridesoxypiperidin, 6-Carboxy-Isofagomin, 2,6-Didesoxy-2,6-imino-Sialsäure, und Castanospermin (CAS); und pharmazeutisch akzeptablen Salzen davon; und
wobei der zweite Bestandteile Ambroxol oder ein pharmazeutisch akzeptables Salz davon,
Bromhexin oder ein pharmazeutisch akzeptables Salz davon,
eine Verbindung gemäß Formel (IV) 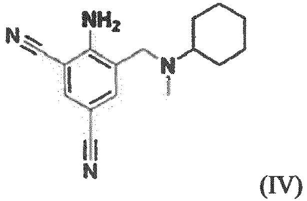 oder ein pharmazeutisch akzeptables Salz davon,
eine Verbindung gemäß Formel (V) 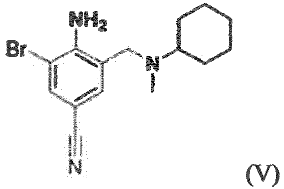 oder ein pharmazeutisch akzeptables Salz davon,
eine Verbindung gemäß Formel (VI) 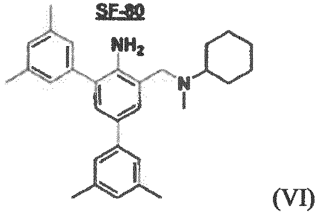 oder ein pharmazeutisch akzeptables Salz davon,
eine Verbindung gemäß Formel (VII) 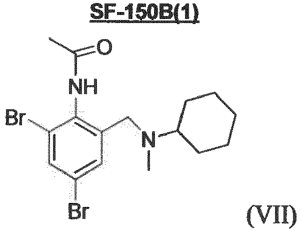 oder ein pharmazeutisch akzeptables Salz davon,
eine Verbindung gemäß Formel (VIII) 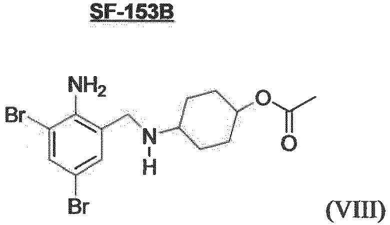 oder ein pharmazeutisch akzeptables Salz davon, und/oder
eine Verbindung gemäß Formel (IX) 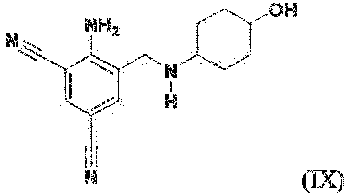 oder ein pharmazeutisch akzeptables Salz davon ist, und wobei der weitere Bestandteil ausgewählt ist aus der Gruppe umfassend ein pharmazeutisch akzeptables Bindemittel und ein pharmazeutisch akzeptables Agens, und
wobei bevorzugterweise die pharmazeutische Zubereitung die verringerte Aktivität des lysosomalen Proteins erhöht, und wobei das lysosomale Protein eine verringerte Aktivität aufweist.

11. Verfahren zum Herstellen einer pharmazeutischen Zubereitung nach Anspruch 10, umfassend die Schritte Formulieren des ersten Bestandteils und des zweiten Bestandteils in eine einzige Dosierungsform oder in zwei getrennte Dosierungsformen, wobei im Falle der zwei getrennten Dosierungsformen eine erste der zwei Dosierungsformen den ersten Bestandteil enthält, und eine zweite der zwei getrennten Dosierungsformen den zweiten Bestandteil enthält.

12. Chaperon mit der Fähigkeit, ein lysosomales Protein umzulagern, wobei das lysosomale Protein eine verringerte Aktivität aufweist und das Charperon ausgewählt ist aus der Gruppe bestehend aus 1-Desoxygalactonojirimycin (DGJ), alpha-Galacto-Homonojirimycin, alpha-allo-Homonojirimycin, beta-1-C-Butyl-Desoxygalactonojirimycin, beta-1-C-Butyl-Desoxynojirimycin, N-Nonyl-Desoxynojirimycin (NN-DNJ), N-Octyl-2,5-anhydro-2,5-imino-D-Glucitol, N-Octyl-Isofagomin, Isofagomin (IFG), Calystegin A3, Calystegin B1, Calystegin B2, Calystegin C1, 1,5-Didesoxy-1,5-iminoxylitol (DIX), alpha-1-C-Nonyl-DIX, alpha-1-C-Octyl-1-DNJ, N-Acetyl-Glucosamin-Thiazolin (NGT), 6-Acetamido-6-desoxycastanospermin (ACAS), N-Butyl-DNJ, Desoxynojirimycin (DNJ), 2-Acetamido-1,2-didesoxynojirimycin (AdDNJ), N-Dodecyl-DNJ, 6-Nonyl-Isofagomin, N-Methyl-Calystegin A₃, 4-epi-Isofagomin, 1-Desoxynojirimycin, alpha-Homonojirimycin, Castanospermin, 1-Desoxymannojirimycin, Swainsonin, 2-Hydroxy-Isofagomin, 1-Desoxyfuconojirimycin, beta-Homofuconojirimycin, 2,5-Imino-1,2,5-tridesoxy-L-Glucitol, 2,5-Didesoxy-2,5-imino-D-Fucitol, 2,5-Imino-1,2,5-tridesoxy-D-Altritol, 1,2-Didesoxy-2-N-acetamido-Nojirimycin, 1,2-Didesoxy-2-N-acetamido-Galaconojirimycin, 2-N-Acetylamino-Isofagomin, 1,2-Didesoxy-2-acetamido-Nojirimycin, 2-N-Acetamido-Isofagomin, 1,2-Didesoxy-2-Acetamido-Nojirimycin, 1-Desoxyiduronojirimycin, 2-Carboxy-3,4,5-tridesoxypiperidin, 6-Carboxy-Isofagomin, 2,6-Didesoxy-2,6-imino-Sialsäure, und Castanospermin (CAS); und pharmazeutisch akzeptablen Salzen davon,
zur Verwendung bei der Behandlung einer Krankheit,
wobei das Verfahren umfasst Verabreichen des Chaperons an ein Lebewesen und davor, zeitgleich oder danach von Ambroxol oder einem pharmazeutisch akzeptablen Salz davon,
Bromhexin oder einem pharmazeutisch akzeptablen Salz davon,
einer Verbindung gemäß Formel (IV) 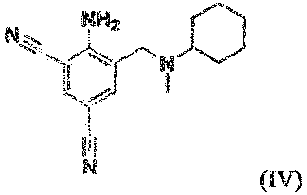 oder einem pharmazeutisch akzeptablen Salz davon,
einer Verbindung gemäß Formel (V) 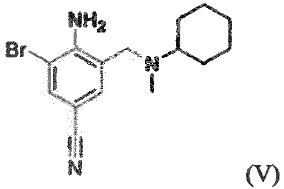 oder einem pharmazeutisch akzeptablen Salz davon,
einer Verbindung gemäß Formel (VI) 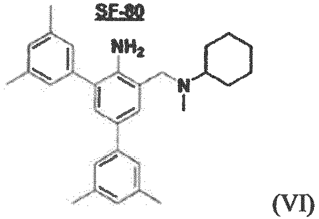 oder einem pharmazeutisch akzeptablen Salz davon,
einer Verbindung gemäß Formel (VII) 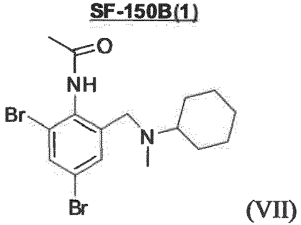 oder einem pharmazeutisch akzeptablen Salz davon,
einer Verbindung gemäß Formel (VIII) 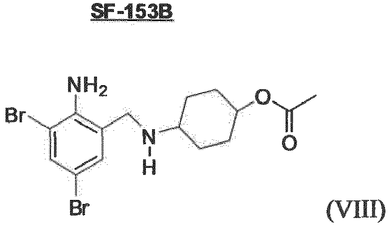 oder einem pharmazeutisch akzeptablen Salz davon, und/oder
einer Verbindung gemäß Formel (IX) 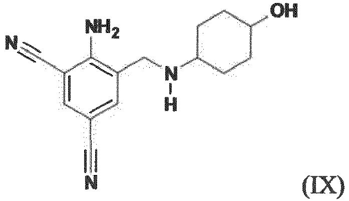 oder einem pharmazeutisch akzeptablen Salz davon.

13. Ambroxol oder ein pharmazeutisch akzeptables Salz davon, Bromhexin oder ein pharmazeutisch akzeptables Salz davon,
eine Verbindung gemäß Formel (IV) 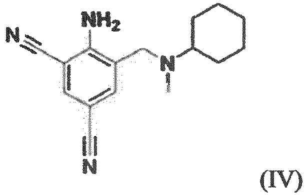 oder ein pharmazeutisch akzeptables Salz davon,
eine Verbindung gemäß Formel (V) 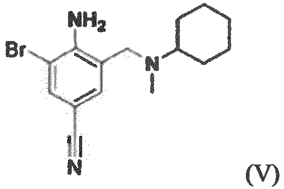 oder ein pharmazeutisch akzeptables Salz davon,
eine Verbindung gemäß Formel (VI) 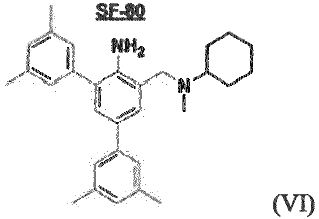 oder ein pharmazeutisch akzeptables Salz davon,
eine Verbindung gemäß Formel (VII) 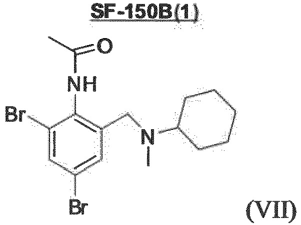 oder ein pharmazeutisch akzeptables Salz davon,
eine Verbindung gemäß Formel (VIII) 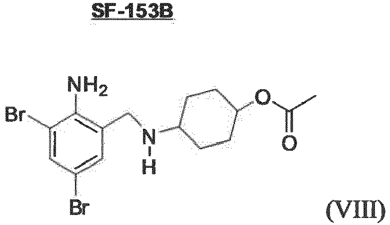 oder ein pharmazeutisch akzeptables Salz davon, und/oder
eine Verbindung gemäß Formel (IX) 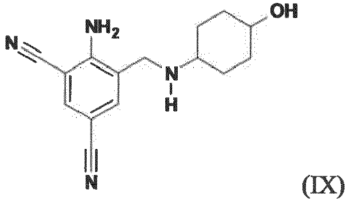 oder ein pharmazeutisch akzeptables Salz davon
zur Verwendung in einem Verfahren für die Behandlung einer Krankheit,
wobei das Verfahren umfasst Verabreichen an ein Lebewesen von Ambroxol oder einem pharmazeutisch akzeptablen Salz davon, Bromhexin oder einem pharmazeutisch akzeptablen Salz davon,
einer Verbindung gemäß Formel (IV) 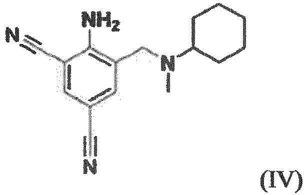 oder einem pharmazeutisch akzeptablen Salz davon,
einer Verbindung gemäß Formel (V) 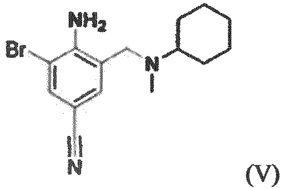 oder einem pharmazeutisch akzeptablen Salz davon,
einer Verbindung gemäß Formel (VI) 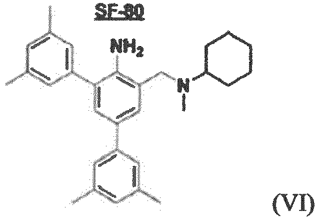 oder einem pharmazeutisch akzeptablen Salz davon,
einer Verbindung gemäß Formel (VII) 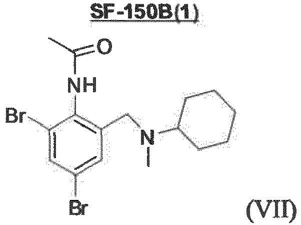 oder einem pharmazeutisch akzeptablen Salz davon,
einer Verbindung gemäß Formel (VIII) 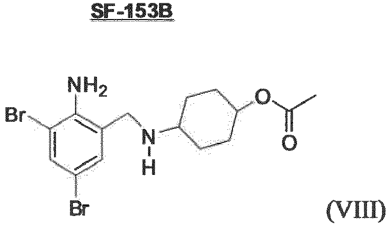 oder einem pharmazeutisch akzeptablen Salz davon, und/oder
einer Verbindung gemäß Formel (IX) 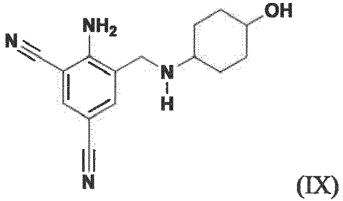 oder einem pharmazeutisch akzeptablen Salz davon und davor, zeitgleich oder danach eines Chaperons mit der Fähigkeit, ein lysosomales Protein umzulagern, wobei das lysosomale Protein eine verringerte Aktivität aufweist und das Chaperon ausgewählt ist aus der Gruppe bestehend aus 1-Desoxygalactonojirimycin (DGJ), alpha-Galacto-Homonojirimycin, alpha-allo-Homonojirimycin, beta-1-C-Butyl-Desoxygalactonojirimycin, beta-1-C-Butyl-Desoxynojirimycin, N-Nonyl-Desoxynojirimycin (NN-DNJ), N-Octyl-2,5-anhydro-2,5-imino-D-Glucitol, N-Octyl-Isofagomin, Isofagomin (IFG), Calystegin A3, Calystegin B1, Calystegin B2, Calystegin C1, 1,5-Didesoxy-1,5-iminoxylitol (DIX), alpha-1-C-Nonyl-DIX, alpha-1-C-Octyl-1-DNJ, N-Acetyl-Glucosamin-Thiazolin (NGT), 6-Acetamido-6-desoxycastanospermin (ACAS), N-Butyl-DNJ, Desoxynojirimycin (DNJ), 2-Acetamido-1,2-didesoxynojirimycin (AdDNJ), N-Dodecyl-DNJ, 6-Nonyl-Isofagomin, N-Methyl-Calystegin A₃, 4-epi-Isofagomin, 1-Desoxynojirimycin, alpha-Homonojirimycin, Castanospermin, 1-Desoxymannojirimycin, Swainsonin, 2-Hydroxy-Isofagomin, 1-Desoxyfuconojirimycin, beta-Homofuconojirimycin, 2,5-Imino-1,2,5-tridesoxy-L-Glucitol, 2,5-Didesoxy-2,5-imino-D-Fucitol, 2,5-Imino-1,2,5-tridesoxy-D-Altritol, 1,2-Didesoxy-2-N-acetamido-Nojirimycin, 1,2-Didesoxy-2-N-acetamido-Galaconojirimycin, 2-N-Acetylamino-Isofagomin, 1,2-Didesoxy-2-acetamido-Nojirimycin, 2-N-Acetamido-Isofagomin, 1,2-Didesoxy-2-acetamido-Nojirimycin, 1-Desoxyiduronojirimycin, 2-Carboxy-3,4,5-tridesoxypiperidin, 6-Carboxy-Isofagomin, 2,6-Didesoxy-2,6-imino-Sialsäure, und Castanospermin (CAS); und pharmazeutisch akzeptablen Salzen davon.

14. Kombination nach einem der Ansprüche 1 bis 8 zur Verwendung in einem Verfahren zur personalisierten Behandlung eines Lebewesens, wobei das Verfahren die folgenden Schritte umfasst:
Schritt a): Bestimmen, ob in einer Probe von dem Lebewesen das lysosomale Protein eine verringerte Aktivität aufweist, wobei bevorzugterweise diese verringerte Aktivität das Ergebnis einer oder mehrerer Mutationen des lysosomalen Proteins verglichen mit dem lysosomalen Protein vom Wildtyp ist;
Schritt b) Identifizieren einer Verbindung mit der Fähigkeit, das lysosomale Protein mit verringerter Aktivität umzulagern, und wobei die Verbindung dazu geeignet ist, die verringerte Aktivität des lysosomalen Proteins zu erhöhen oder diese erhöht; und
Schritt c): Verabreichen des ersten Bestandteils vor, gleichzeitig mit oder nach dem zweiten Bestandteil an das Lebewesen.

## Revendications

1. Combinaison comprenant un premier constituant et un second constituant,
dans laquelle le premier constituant est un chaperon ayant la capacité de réarranger une protéine lysosomale, dans laquelle la protéine lysosomale présente une activité réduite et dans laquelle le chaperon est un imino-sucre sélectionné dans le groupe consistant en la 1-désoxygalactonojirimycine (DGJ), l'alpha-galacto-homonojirimycine, l'alpha-allo-homonojirimycine, la bêta-1-C-butyl-désoxygalactonojirimycine, la bêta-1-C-butyl-désoxynojirimycine, la N-nonyl-désoxynojirimycine (NN-DNJ), la N-octyl-2,5-anhydro-2,5-imino-D-glucitol, la N-octyl-isofagomine, l'isofagomine (IFG), la calystégine A3, la calystégine B1, la calystégine B2, la calystégine C1, le 1,5-didésoxy-1,5-iminoxylitol (DIX), l'alpha-1-C-nonyl-DIX, l'alpha-1-C-octyl-1-DNJ, la N-acétyl-glucosamine-thiazoline (NGT), la 6-acétamido-6-désoxycastanospermine (ACAS),
la N-butyl-DNJ, la désoxynojirimycine (DNJ), la 2-acétamido-1,2-didésoxynojirimycine (AdDNJ), la N-dodécyl-DNJ, la 6-nonyl-isofagomine, la N-méthyl-calystégine A₃, la 4-épi-isofagomine, la 1-désoxynojirimycine, l'alpha-homonojirimycine, la castanospermine, la 1-désoxymannojirimycine, la swainsonine, la 2-hydroxy-isofagomine, la 1-désoxyfuconojirimycine, la bêta-homofuconojirimycine, la 2,5-imino-1,2,5-tridésoxy-L-glucitol, la 2,5-didésoxy-2,5-imino-D-fucitol, la 2,5-imino-1,2,5-tridésoxy-D-altritol, la 1,2-didésoxy-2-N-acétamido-nojirimycine, la 1,2-didésoxy-2-N-acétamido-galaconojirimycine, la 2-N-acétylamino-isofagomine, la 1,2-didésoxy-2-acétamido-nojirimycine, la 2-N-acétamido-isofagomine, la 1,2-didésoxy-2-acétamido-nojirimycine, la 1-désoxyiduronojirimycine, la 2-carboxy-3,4,5-tridésoxypipéridine, la 6-carboxy-isofagomine, l'acide 2,6-didésoxy-2,6-imino-sialique, et la castanospermine (CAS) ; et les sels pharmaceutiquement acceptables de ceux-ci ; et
dans laquelle le second constituant est l'ambroxol ou un sel pharmaceutiquement acceptable de celui-ci,
la bromhexine ou un sel pharmaceutiquement acceptable de celle-ci,
un composé de formule (IV) 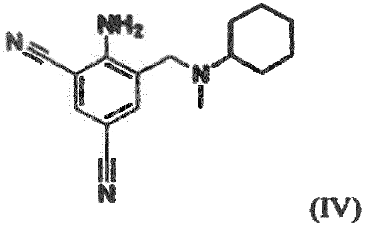 ou un sel pharmaceutiquement acceptable de celui-ci,
un composé de formule (V) 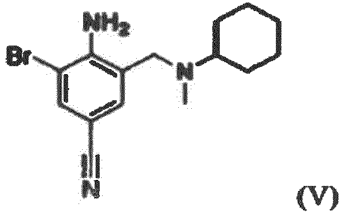 ou un sel pharmaceutiquement acceptable de celui-ci,
un composé de formule (VI) 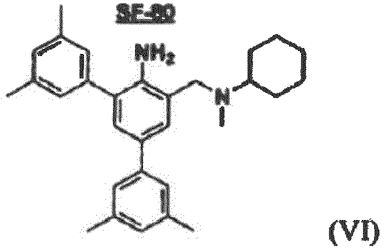 ou un sel pharmaceutiquement acceptable de celui-ci,
un composé de formule (VII) 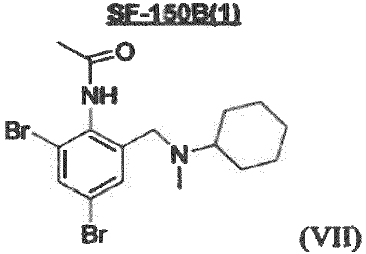 ou un sel pharmaceutiquement acceptable de celui-ci,
un composé de formule (VIII) 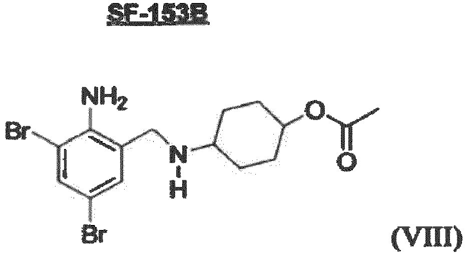 ou un sel pharmaceutiquement acceptable de celui-ci, et/ou
un composé de formule (IX) 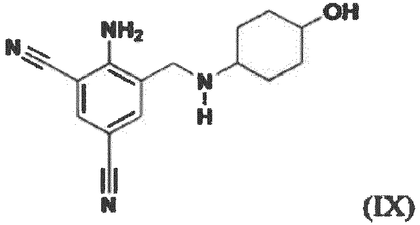 ou un sel pharmaceutiquement acceptable de celui-ci.

2. Combinaison selon la revendication 1, dans laquelle la protéine lysosomale est affectée dans une maladie, et dans laquelle la protéine lysosomale est sélectionnée dans le groupe consistant en l'alpha-galactosidase A et l'alpha-glucosidase.

3. Combinaison selon l'une quelconque des revendications 1 à 2, dans laquelle la combinaison est une combinaison pharmaceutique.

4. Combinaison selon l'une quelconque des revendications 1 à 3, dans laquelle la combinaison est appropriée pour ou est pour une utilisation dans un procédé pour le traitement d'un sujet comprenant l'administration de la combinaison au sujet.

5. Combinaison selon la revendication 4 ou pour une utilisation selon la revendication 4, dans laquelle le sujet souffre ou risque de souffrir d'une maladie, dans laquelle la maladie est **caractérisée par** une enzyme ayant une activité réduite, dans laquelle l'enzyme ayant une activité réduite est sélectionnée dans le groupe consistant en l'alpha-galactosidase A et l'alpha-glucosidase.

6. Combinaison selon la revendication 5 ou pour une utilisation selon la revendication 5, dans laquelle la maladie est sélectionnée dans le groupe comprenant la maladie de Fabry, la maladie de Schindler-Kanzaki, la maladie de Pompe et la maladie de Parkinson.

7. Combinaison selon l'une quelconque des revendications 5 et 6 ou pour une utilisation selon l'une quelconque des revendications 5 et 6, dans laquelle l'enzyme est l'alpha-galactosidase A et la maladie est sélectionnée dans le groupe comprenant la maladie de Fabry, la maladie de Schindler-Kanzaki et la maladie de Parkinson.

8. Combinaison selon l'une quelconque des revendications 5 et 6 ou pour une utilisation selon l'une quelconque des revendications 5 et 6, dans laquelle l'enzyme est l'alphaglucosidase et la maladie est la maladie de Pompe.

9. Utilisation d'une combinaison telle que définie dans l'une quelconque des revendications 1 à 8, pour la préparation d'un médicament pour le traitement ou la prévention d'une maladie.

10. Préparation pharmaceutique comprenant un premier constituant, un second constituant facultativement un constituant supplémentaire,
dans laquelle le premier constituant est un chaperon ayant la capacité de réarranger une protéine lysosomale, dans laquelle la protéine lysosomale présente une activité réduite et dans laquelle le chaperon est sélectionné dans le groupe consistant en la 1-désoxygalactonojirimycine (DGJ), l'alpha-galacto-homonojirimycine, l'alpha-allo-homonojirimycine, la bêta-1-C-butyl-désoxygalactonojirimycine, la bêta-1-C-butyl-désoxynojirimycine, la N-nonyl-désoxynojirimycine (NN-DNJ), la N-octyl-2,5-anhydro-2,5-imino-D-glucitol, la N-octyl-isofagomine, l'isofagomine (IFG), la calystégine A3, la calystégine B1, la calystégine B2, la calystégine C1, le 1,5-didésoxy-1,5-iminoxylitol (DIX), l'alpha-1-C-nonyl-DIX, l'alpha-1-C-octyl-1-DNJ, la N-acétyl-glucosamine-thiazoline (NGT), la 6-acétamido-6-désoxycastanospermine (ACAS),
la N-butyl-DNJ, la désoxynojirimycine (DNJ), la 2-acétamido-1,2-didésoxynojirimycine (AdDNJ), la N-dodécyl-DNJ, la 6-nonyl-isofagomine, la N-méthyl-calystégine A₃, la 4-épi-isofagomine, la 1-désoxynojirimycine, l'alpha-homonojirimycine, la castanospermine, la 1-désoxymannojirimycine, la swainsonine, la 2-hydroxy-isofagomine, la 1-désoxyfuconojirimycine, la bêta-homofuconojirimycine, la 2,5-imino-1,2,5-tridésoxy-L-glucitol, la 2,5-didésoxy-2,5-imino-D-fucitol, la 2,5-imino-1,2,5-tridésoxy-D-altritol, la 1,2-didésoxy-2-N-acétamido-nojirimycine, la 1,2-didésoxy-2-N-acétamido-galaconojirimycine, la 2-N-acétylamino-isofagomine, la 1,2-didésoxy-2-acétamido-nojirimycine, la 2-N-acétamido-isofagomine, la 1,2-didésoxy-2-acétamido-nojirimycine, la 1-désoxyiduronojirimycine, la 2-carboxy-3,4,5-tridésoxypipéridine, la 6-carboxy-isofagomine, l'acide 2,6-didésoxy-2,6-imino-sialique, et la castanospermine (CAS) ; et les sels pharmaceutiquement acceptables de ceux-ci ; dans laquelle le second constituant est l'ambroxol ou un sel pharmaceutiquement acceptable de celui-ci, la bromhexine ou un sel pharmaceutiquement acceptable de celle-ci, un composé de formule (IV) 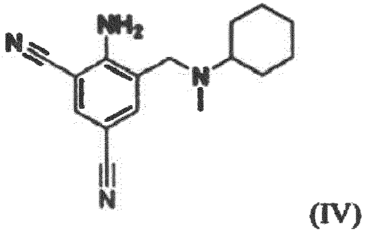 ou un sel pharmaceutiquement acceptable de celui-ci,
un composé de formule (V) 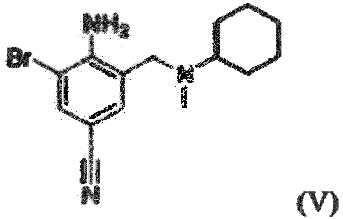 ou un sel pharmaceutiquement acceptable de celui-ci,
un composé de formule (VI) 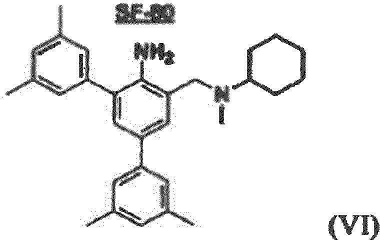 ou un sel pharmaceutiquement acceptable de celui-ci,
un composé de formule (VII) 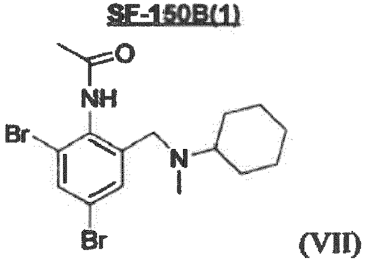 ou un sel pharmaceutiquement acceptable de celui-ci,
un composé de formule (VIII) 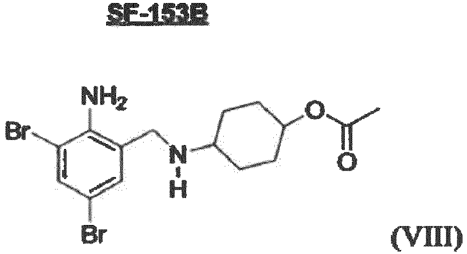 ou un sel pharmaceutiquement acceptable de celui-ci, et/ou
un composé de formule (IX) 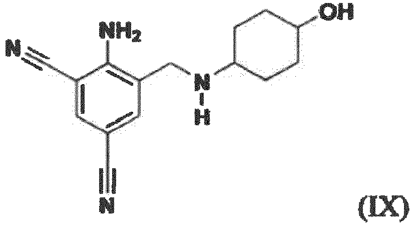 ou un sel pharmaceutiquement acceptable de celui-ci, et dans laquelle le constituant supplémentaire est sélectionné dans le groupe comprenant un excipient pharmaceutiquement acceptable et un agent pharmaceutiquement actif, et dans laquelle de préférence la préparation pharmaceutique augmente l'activité réduite de la protéine lysosomale, et dans laquelle la protéine lysosomale présente une activité réduite.

11. Procédé pour préparer une préparation pharmaceutique selon la revendication 10, comprenant les étapes de formulation du premier constituant et du second constituant dans une unique forme galénique ou dans deux formes galéniques séparées, dans lequel dans le cas de deux formes galéniques séparées une première des deux formes galéniques séparées contient le premier constituant et une seconde des deux formes galéniques séparées contient le second constituant.

12. Chaperon ayant la capacité de réarranger une protéine lysosomale, dans lequel la protéine lysosomale présente une activité réduite et dans lequel le chaperon est sélectionné dans le groupe consistant en la 1-désoxygalactonojirimycine (DGJ), l'alpha-galacto-homonojirimycine, l'alpha-allo-homonojirimycine, la bêta-1-C-butyl-désoxygalactonojirimycine, la bêta-1-C-butyl-désoxynojirimycine, la N-nonyl-désoxynojirimycine (NN-DNJ), la N-octyl-2,5-anhydro-2,5-imino-D-glucitol, la N-octyl-isofagomine, l'isofagomine (IFG), la calystégine A3, la calystégine B1, la calystégine B2, la calystégine C1, le 1,5-didésoxy-1,5-iminoxylitol (DIX), l'alpha-1-C-nonyl-DIX, l'alpha-1-C-octyl-1-DNJ, la N-acétyl-glucosamine-thiazoline (NGT), la 6-acétamido-6-désoxycastanospermine (ACAS), la N-butyl-DNJ, la désoxynojirimycine (DNJ), la 2-acétamido-1,2-didésoxynojirimycine (AdDNJ), la N-dodécyl-DNJ, la 6-nonyl-isofagomine, la N-méthyl-calystégine A₃, la 4-épi-isofagomine, la 1-désoxynojirimycine, l'alpha-homonojirimycine, la castanospermine, la 1-désoxymannojirimycine, la swainsonine, la 2-hydroxy-isofagomine, la 1-désoxyfuconojirimycine, la bêta-homofuconojirimycine, la 2,5-imino-1,2,5-tridésoxy-L-glucitol, la 2,5-didésoxy-2,5-imino-D-fucitol, la 2,5-imino-1,2,5-tridésoxy-D-altritol, la 1,2-didésoxy-2-N-acétamido-nojirimycine, la 1,2-didésoxy-2-N-acétamido-galaconojirimycine, la 2-N-acétylamino-isofagomine, la 1,2-didésoxy-2-acétamido-nojirimycine, la 2-N-acétamido-isofagomine, la 1,2-didésoxy-2-acétamido-nojirimycine, la 1-désoxyiduronojirimycine, la 2-carboxy-3,4,5-tridésoxypipéridine, la 6-carboxy-isofagomine, l'acide 2,6-didésoxy-2,6-imino-sialique, et la castanospermine (CAS) ; et les sels pharmaceutiquement acceptables de ceux-ci, pour une utilisation dans un procédé pour le traitement d'une maladie,
dans lequel le procédé comprend l'administration à un sujet du chaperon et, avant, en même temps ou après d'ambroxol ou d'un sel pharmaceutiquement acceptable de celui-ci, de bromhexine ou d'un sel pharmaceutiquement acceptable de celle-ci, d'un composé de formule (IV) 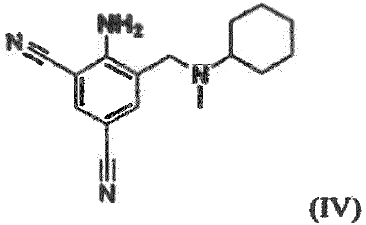 ou d'un sel pharmaceutiquement acceptable de celui-ci,
d'un composé de formule (V) 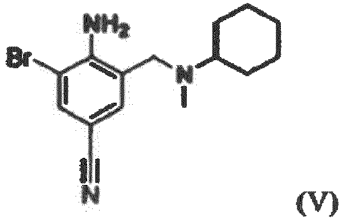 ou d'un sel pharmaceutiquement acceptable de celui-ci,
d'un composé de formule (VI) 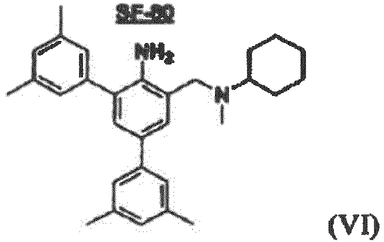 ou d'un sel pharmaceutiquement acceptable de celui-ci,
d'un composé de formule (VII) 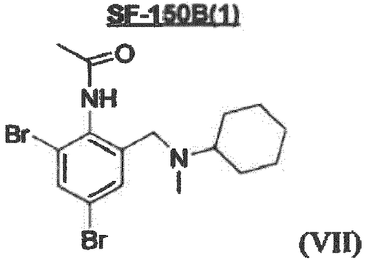 ou d'un sel pharmaceutiquement acceptable de celui-ci,
d'un composé de formule (VIII) 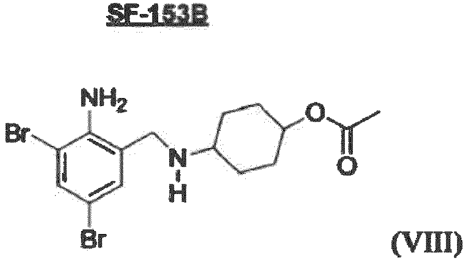 ou d'un sel pharmaceutiquement acceptable de celui-ci, et/ou
d'un composé de formule (IX) 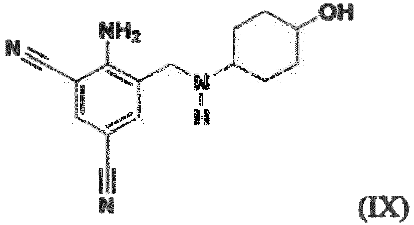 ou d'un sel pharmaceutiquement acceptable de celui-ci.

13. Ambroxol ou un sel pharmaceutiquement acceptable de celui-ci,
bromhexine ou un sel pharmaceutiquement acceptable de celle-ci,
un composé de formule (IV) 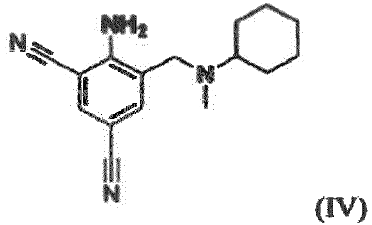 ou un sel pharmaceutiquement acceptable de celui-ci,
un composé de formule (V) 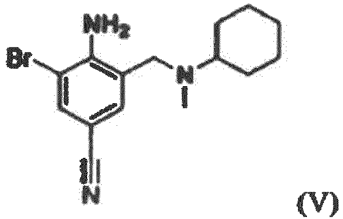 ou un sel pharmaceutiquement acceptable de celui-ci,
un composé de formule (VI) 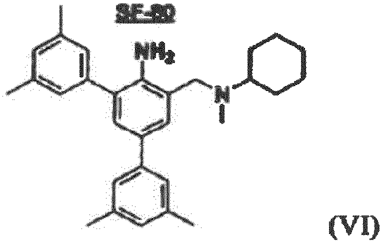 ou un sel pharmaceutiquement acceptable de celui-ci,
un composé de formule (VII) 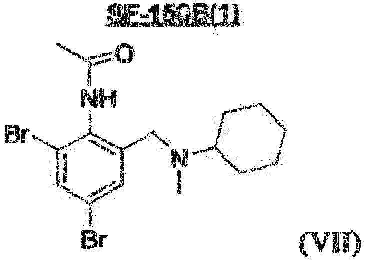 ou un sel pharmaceutiquement acceptable de celui-ci,
un composé de formule (VIII) 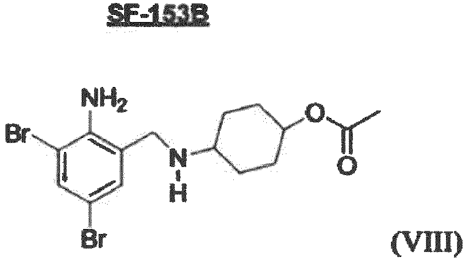 ou un sel pharmaceutiquement acceptable de celui-ci, et/ou
un composé de formule (IX) 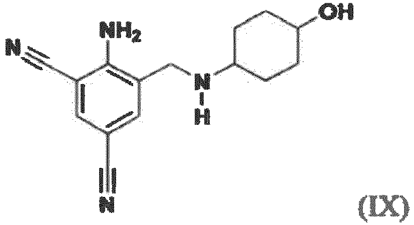 ou un sel pharmaceutiquement acceptable de celui-ci pour une utilisation dans un procédé pour le traitement d'une maladie, dans lequel le procédé comprend l'administration à un sujet d'ambroxol ou d'un sel pharmaceutiquement acceptable de celui-ci, de bromhexine ou d'un sel pharmaceutiquement acceptable de celle-ci, d'un composé de formule (IV) 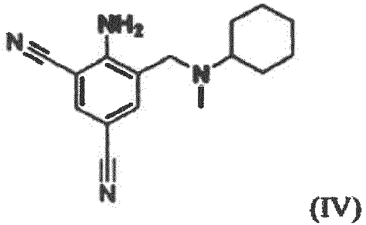 ou d'un sel pharmaceutiquement acceptable de celui-ci,
d'un composé de formule (V) 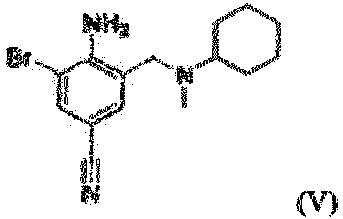 ou d'un sel pharmaceutiquement acceptable de celui-ci,
d'un composé de formule (VI) 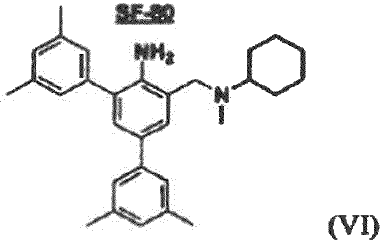 ou d'un sel pharmaceutiquement acceptable de celui-ci,
d'un composé de formule (VII) 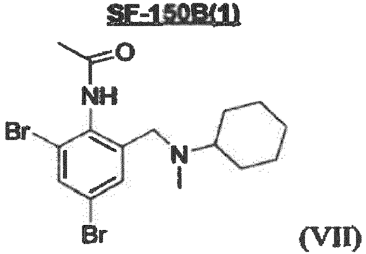 ou d'un sel pharmaceutiquement acceptable de celui-ci,
d'un composé de formule (VIII) 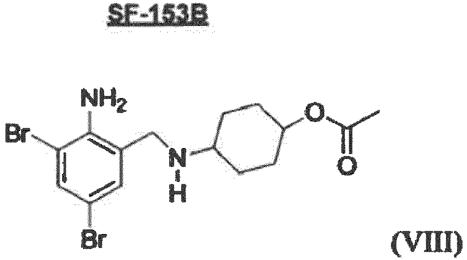 ou d'un sel pharmaceutiquement acceptable de celui-ci, et/ou
d'un composé de formule (IX) 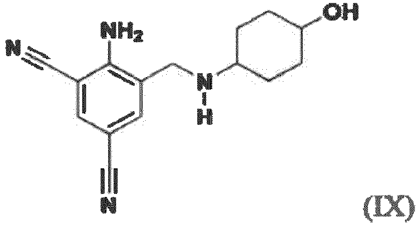 ou d'un sel pharmaceutiquement acceptable de celui-ci et, avant, en même temps ou après un chaperon ayant la capacité de réarranger une protéine lysosomale, dans lequel la protéine lysosomale présente une activité réduite et dans lequel le chaperon est sélectionné dans le groupe consistant en la 1-désoxygalactonojirimycine (DGJ), l'alpha-galacto-homonojirimycine, l'alpha-allo-homonojirimycine, la bêta-1-C-butyl-désoxygalactonojirimycine, la bêta-1-C-butyl-désoxynojirimycine, la N-nonyl-désoxynojirimycine (NN-DNJ), la N-octyl-2,5-anhydro-2,5-imino-D-glucitol, la N-octyl-isofagomine, l'isofagomine (IFG), la calystégine A3, la calystégine B1, la calystégine B2, la calystégine C1, le 1,5-didésoxy-1,5-iminoxylitol (DIX), l'alpha-1-C-nonyl-DIX, l'alpha-1-C-octyl-1-DNJ, la N-acétyl-glucosamine-thiazoline (NGT), la 6-acétamido-6-désoxycastanospermine (ACAS),
la N-butyl-DNJ, la désoxynojirimycine (DNJ), la 2-acétamido-1,2-didésoxynojirimycine (AdDNJ), la N-dodécyl-DNJ, la 6-nonyl-isofagomine, la N-méthyl-calystégine A₃, la 4-épi-isofagomine, la 1-désoxynojirimycine, l'alpha-homonojirimycine, la castanospermine, la 1-désoxymannojirimycine, la swainsonine, la 2-hydroxy-isofagomine, la 1-désoxyfuconojirimycine, la bêta-homofuconojirimycine, la 2,5-imino-1,2,5-tridésoxy-L-glucitol, la 2,5-didésoxy-2,5-imino-D-fucitol, la 2,5-imino-1,2,5-tridésoxy-D-altritol, la 1,2-didésoxy-2-N-acétamido-nojirimycine, la 1,2-didésoxy-2-N-acétamido-galaconojirimycine, la 2-N-acétylamino-isofagomine, la 1,2-didésoxy-2-acétamido-nojirimycine, la 2-N-acétamido-isofagomine, la 1,2-didésoxy-2-acétamido-nojirimycine, la 1-désoxyiduronojirimycine, la 2-carboxy-3,4,5-tridésoxypipéridine, la 6-carboxy-isofagomine, l'acide 2,6-didésoxy-2,6-imino-sialique, et la castanospermine (CAS) ; et les sels pharmaceutiquement acceptables de ceux-ci.

14. Combinaison selon l'une quelconque des revendications 1 à 8, pour une utilisation dans un procédé de traitement thérapeutique personnalisé d'un sujet, dans laquelle le procédé comprend les étapes suivantes :
étape a) : le fait de déterminer si dans un échantillon du sujet la protéine lysosomale présente une activité réduite, de préférence cette activité réduite résulte d'une ou de plusieurs mutations de la protéine lysosomale par rapport à la protéine lysosomale de type sauvage ;
étape b) : identification d'un composé ayant la capacité de réarranger la protéine lysosomale présentant une activité réduite, et dans laquelle le composé est approprié pour ou augmente l'activité réduite de la protéine lysosomale ; et
étape c) : l'administration au sujet du premier constituant avant, en même temps ou après le second constituant.


<110> Centogene GmbH
<120> Combination of a compound having the ability to rearrange a lysosomal enzyme and Ambroxol and/or a derivative of Ambroxol
<130> C 10046 PCT
<160> 4
<170> PatentIn version 3.3
<210> 1
   <211> 19
   <212> DNA
   <213> Artificial
<220>
   <223> synthetic
<400> 1
   acaatgcagc tgaggaacc 19
<210> 2
   <211> 39
   <212> DNA
   <213> Artificial
<220>
   <223> synthetic
<400> 2
   ggtgttcgaa ttaaagtaag tcttttaatg acatctgca 39
<210> 3
   <211> 20
   <212> DNA
   <213> Artificial
<220>
   <223> synthetic
<400> 3
   taggagctgt ccaggccatc 20
<210> 4
   <211> 20
   <212> DNA
   <213> Artificial
<220>
   <223> synthetic
<400> 4
   gagagactaa cacactccgc 20